(19) Europäisches Patentamt
European Patent Office
Office européen des brevets

(11) **EP 2 866 019 A1**

(12) **EUROPEAN PATENT APPLICATION**
published in accordance with Art. 153(4) EPC

(43) Date of publication:
**29.04.2015 Bulletin 2015/18**

(51) Int Cl.:
*G01N 3/00* (2006.01)    *E02D 3/02* (2006.01)

(21) Application number: **13809275.4**

(22) Date of filing: **25.06.2013**

(86) International application number:
**PCT/JP2013/067315**

(87) International publication number:
**WO 2014/002977 (03.01.2014 Gazette 2014/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.06.2012   JP 2012141888**

(71) Applicants:
• **National University Corporation Nagoya University**
**Nagoya-shi, Aichi 464-8601 (JP)**
• **Geoasia Research Society**
**Nagoya-shi, Aichi 468-0026 (JP)**

(72) Inventors:
• **NODA, Toshihiro**
**Nagoya-shi**
**Aichi 464-8601 (JP)**

• **NAKANO, Masaki**
**Nagoya-shi**
**Aichi 464-8601 (JP)**
• **YOSHIKAWA, Takahiro**
**Nagoya-shi**
**Aichi 464-8601 (JP)**
• **ASAOKA, Akira**
**Nagoya-shi**
**Aichi 464-8601 (JP)**

(74) Representative: **Grünecker Patent- und Rechtsanwälte**
**PartG mbB**
**Leopoldstraße 4**
**80802 München (DE)**

(54) **AIR-WATER-SOIL SKELETON COUPLED CALCULATION DEVICE, COUPLED CALCULATION METHOD, AND COUPLED CALCULATION PROGRAM**

(57)    Soil-water-air coupled analyzing computes various matrices with respect to a time rate of volume change of a soil skeleton, tangent stiffness and water permeability, air permeability, moisture characteristic and mass of soil, based on the settings of soils such as clay, intermediate soil and sand with regard to respective soil elements of a soil foundation, settings of a solid soil model and settings of analysis conditions (S140 to S220), establishes simultaneous equations using these matrices, provides a boundary condition regarding deformation, a stress rate or the like, a hydraulic condition regarding pore water and an air boundary condition regarding pore air and determines solutions of unknown "jerk field", pore water pressure field and pore air field (S230). This enables the soil foundation consisting of any types of soils in any states to be analyzed with high accuracy.

Fig. 2

**Description**

Technical Field

The invention relates to a soil-water-air coupled analyzer, a soil-water-air coupled analyzing method and a soil-water-air coupled analyzing program, and more specifically relates to a device for soil-water-air coupled analysis to perform deformation analysis of a soil skeleton, a method for soil-water-air coupled analysis to perform deformation analysis of a soil skeleton and a program for soil-water-air coupled analysis to perform deformation analysis of a soil skeleton.

Background Art

One proposed configuration of this type of coupled analyzer of a soil skeleton establishes rate-type simultaneous equations of motion including a jerk term of the soil skeleton with respect to velocity of the soil skeleton and pore water pressure, based on data regarding a condition of the soil skeleton and data regarding an external force, integrates the rate-type simultaneous equations of motion as needed by providing a geometric boundary condition such as a displacement, a mechanical boundary condition such as a stress and a hydraulic boundary condition such as a pore water pressure, and determines time history responses of a velocity field and a pore water pressure field, so as to perform deformation analysis of the soil skeleton (see, for example, Patent Literature 1). This coupled analyzer of the soil skeleton performs deformation analysis of the soil skeleton by dynamic analysis under large influence of acceleration, while performing deformation analysis of the soil skeleton by static analysis under small influence of acceleration. This allows for deformation analysis of the soil skeleton, whether static analysis or dynamic analysis. The "jerk term" herein means a second-order derivative term of a velocity vector as an explanatory variable with respect to time.

Citation List

Patent Literature

Patent Literature 1: WO 2007/046178 A1

SUMMARY OF INVENTIOM

Technical Problem

The above coupled analyzer of the soil skeleton, however, deals with saturated soil consisting of only soil particles and water and does not allow for sufficient analysis of the soil having water unsaturated in voids of the soil particles, i.e., unsaturated soil having the air as well as water in the voids of the soil particles, while allowing for deformation analysis of the soil skeleton, whether static analysis or dynamic analysis, with regard to the soil foundation of any intermediate soil from sand to clay having water saturated in the voids of the soil particles. Embankment and slope are often in unsaturated states. The coupled analyzer of the soil skeleton described above is accordingly not able to analyze deformation and failure of embankment or slope due to rainfall seepage, and deformation and failure on embankment or slope under the combined condition of torrential rain with earthquake

The soil-water-air coupled analyzer, the soil-water-air coupled analyzing method and the soil-water-air coupled analyzing program of the invention mainly aim to allow for deformation analysis of the soil skeleton, whether static analysis or dynamic analysis, with regard to unsaturated soil having the air in addition to water in the voids of the soil particles. More specifically, the soil-water-air coupled analyzer, the soil-water-air coupled analyzing method and the soil-water-air coupled analyzing program of the invention mainly aim to allow for static or dynamic deformation analysis of the soil skeleton, irrespective of whether soil is saturated soil or unsaturated soil and are applicable to continuous analysis from unsaturated soil to saturated soil, as well as analysis of deformation and failure of embankment or slope due to rainfall seepage and deformation and failure on embankment or slope under the combined condition of torrential rain with earthquake

Solution to Problem

The soil-water-air coupled analyzer, the soil-water-air coupled analyzing method and the soil-water-air coupled analyzing program of the invention are implemented by the following aspects, in order to achieve the object described above.

According to one aspect, there is provided a soil-water-air coupled analyzer, comprising: a data input unit configured to input data regarding a condition of a soil skeleton and data regarding an external force; an analyzer configured to establish rate-type simultaneous equations of motion including a jerk term of the soil skeleton with respect to a velocity of the soil skeleton, a pore water pressure and a pore air pressure, based on the input data regarding the condition of the soil skeleton and the input data regarding the external force, to integrate the rate-type simultaneous equations of motion as needed by providing a geometric boundary condition with regard to a displacement, a displacement rate or an acceleration, a mechanical boundary condition with regard to a stress or a stress rate, a hydraulic boundary condition with regard to a pore water pressure or a total water head and a flow rate of pore water and an air boundary condition with regard to a pore air pressure or a flow rate of pore air and to determine time history responses of a velocity field, a pore water pressure field and a pore air pressure field, so as to perform deformation analysis of the soil skeleton; and a result output unit configured to output a result of the deformation analysis.

The soil-water-air coupled analyzer of this aspect integrates the rate-type simultaneous equations of motion including the jerk term of the soil skeleton with respect to the velocity of the soil skeleton, the pore water pressure and the pore air pressure as needed by providing the geometric boundary condition with regard to the displacement, the displacement rate or the acceleration, the mechanical boundary condition with regard to the stress or the stress rate, the hydraulic boundary condition with regard to the pore water pressure or the total water head and the flow rate of pore water and the air boundary condition with regard to the pore air pressure or the flow rate of pore water, and determines the time history responses of the velocity field, the pore water pressure field and the pore air pressure field. With regard to unsaturated soil having the air in addition to water in voids of the soil particles, this allows for deformation analysis of the soil skeleton by dynamic analysis under large influence of acceleration, while allowing for deformation analysis of the soil skeleton by static analysis under small influence of acceleration. Accordingly, this allows for deformation analysis of the soil skeleton, whether static analysis or dynamic analysis, with regard to soil having water unsaturated in voids of the soil particles, i.e., unsaturated soil having the air in addition to water in voids of the soil particles, as well as saturated soil having water saturated in voids of the soil particles. The "jerk term" herein means a second-order derivative term of a velocity vector as an explanatory variable with respect to time.

In the soil-water-air coupled analyzer of the above aspect, the analyzer may calculate a first term regarding a time rate of volume change of the soil skeleton, a second term regarding water permeability in soil, a third term regarding air permeability in soil, a fourth term regarding a moisture characteristic of soil, a fifth term regarding time rates of volume change of the pore water and the pore air, a sixth term regarding a contribution of the pore water pressure to a load change rate, a seventh term regarding a contribution of the pore air pressure to the load change rate, an eighth term regarding conversion of the flow rate of the pore water, a ninth term regarding conversion of the flow rate of the pore air, a tenth term regarding a mass and an eleventh term regarding a tangent stiffness of the soil skeleton, based on the input data regarding the condition of the soil skeleton and the input data regarding the external force, and may establish the rate-type simultaneous equations of motion using the calculated terms. This allows for more accurate deformation analysis of the soil skeleton with regard to the unsaturated soil.

In the soil-water-air coupled analyzer of the above aspect, the rate-type simultaneous equations of motion may be equations established from an equation of motion of a three-phase mixture consisting of the air, the water and the soil skeleton, an equation for conservation of mass between water and the soil skeleton and an equation for conservation of mass between the air and the soil skeleton. In this aspect, the equation of motion of the three-phase mixture consisting of the air, the water and the soil skeleton may be expressed by Equation (A1) ; the equation for conservation of mass between water and the soil skeleton may be expressed by Equation (B1) ; and the equation for conservation of mass between the air and the soil skeleton may be expressed by Equation (C1).

[Math. 1]

$$\rho \overset{\backslash\backslash}{\boldsymbol{v}}_s + \left[ \left( \rho^w s^w + \rho^a s^a \right)(\mathrm{tr}\, \boldsymbol{D}_s) + n\left\{ \frac{s^w \rho^w}{\mathrm{K}_w} - C\left( \rho^w - \rho^a \right) \right\} \overset{\backslash}{p}^w + n\left\{ \frac{s^a}{\overline{\mathrm{R}}\Theta} + C\left( \rho^w - \rho^a \right) \right\} \overset{\backslash}{p}^a \right]\left( \overset{\backslash}{\boldsymbol{v}}_s - \boldsymbol{b} \right) = \mathrm{div}\, \overset{\backslash}{\boldsymbol{S}}_t \qquad (A1)$$

$$s^w \,\mathrm{div}\, \boldsymbol{v}_s + \frac{1}{\rho^w}\mathrm{div}\left\{ \rho^w \frac{k^w}{\gamma_w}\left( -\mathrm{grad}\, p^w + \rho^w \boldsymbol{b} - \rho^w \overset{\backslash}{\boldsymbol{v}}_s \right) \right\} + \frac{ns^w}{\mathrm{K}_w}\overset{\backslash}{p}^w + nC\left( \overset{\backslash}{p}^a - \overset{\backslash}{p}^w \right) = 0 \qquad (B1)$$

$$s^a \,\mathrm{div}\, \boldsymbol{v}_s + \frac{1}{\rho^a}\mathrm{div}\left\{ \rho^a \frac{k^a}{\gamma_w}\left( -\mathrm{grad}\, p^a + \rho^a \boldsymbol{b} - \rho^a \overset{\backslash}{\boldsymbol{v}}_s \right) \right\} + \frac{ns^a}{\rho^a \overline{\mathrm{R}}\Theta}\overset{\backslash}{p}^a - nC\left( \overset{\backslash}{p}^a - \overset{\backslash}{p}^w \right) = 0 \qquad (C1)$$

$\boldsymbol{v}_s$ : velocity vector of soil skeleton

$\boldsymbol{D}_s$ : stretching tensor of soil skeleton

$\rho$ : density of entire mixture

$\rho^w$ : density of water

$\rho^a$ : density of air

$s^w$ : degree of saturation

$s^a$ : air void ratio

$n$ : porosity

$p^w$ : pore water pressure

$p^a$ : pore air pressure

$C$ : specific moisture capacity

$K_w$ : volume elasticity modulus of water

$\overline{R}$ : gas constant of air

$\Theta$ : absolute temperature of air

$\boldsymbol{b}$ : body force vector per unit mass

$k^w$ : coefficient of water permeability

$k^a$ : coefficient of air permeability

$\gamma_w$ : unit volume weight of water at 4°C (standard density) $\dot{\boldsymbol{S}}_t = \dot{\boldsymbol{T}} + (\text{tr } \boldsymbol{D}_s)\boldsymbol{T} - \boldsymbol{T}\boldsymbol{L}_s^T$

$\boldsymbol{T}$ : total stress tensor

$\boldsymbol{L}_s$ : velocity gradient tensor of soil skeleton

In Equations, the superscript "\" (hereinafter referred to as "\ (superscript)" means a material time derivative viewed from the soil skeleton. For example, "v" with two "\ (superscript) " represents a jerk vector of the soil skeleton. In the description below, "\ (superscript) "meaning-material time derivative viewed from the soil skeleton is expressed as superscript "·" (hereinafter referred to as "· (superscript)", and s (subscript) representing the soil skeleton is omitted. For example, "v" with two "\ (superscript) " is expressed as "v" with two " · (superscript) ". For the further simplicity, the pore water is assumed as an incompressible fluid (Kw is infinite). As a result, the fundamental equations, i.e., Equations (A1), (B1) and (C1) are rewritten as Equations (A1)', (B1)' and (C1)' given below.

[Math. 2]

$$\rho\ddot{\boldsymbol{v}}+\left[\left(\rho^w s^w+\rho^a s^a\right)(\text{tr }\boldsymbol{D})-nC\left(\rho^w-\rho^a\right)\dot{p}^w+n\left\{\frac{s^a}{\overline{R}\Theta}+C\left(\rho^w-\rho^a\right)\right\}\dot{p}^a\right](\dot{\boldsymbol{v}}-\boldsymbol{b})=\text{div }\dot{\boldsymbol{S}}_t \qquad (A1)'$$

$$s^w\text{ div }\boldsymbol{v}+\text{div}\left\{\frac{k^w}{\gamma_w}\left(-\text{ grad }p^w+\rho^w\boldsymbol{b}-\rho^w\dot{\boldsymbol{v}}\right)\right\}+nC\left(\dot{p}^a-\dot{p}^w\right)=0 \qquad (B1)'$$

$$s^a\text{ div }\boldsymbol{v}+\frac{1}{\rho^a}\text{div}\left\{\rho^a\frac{k^a}{\gamma_w}\left(-\text{ grad }p^a+\rho^a\boldsymbol{b}-\rho^a\dot{\boldsymbol{v}}\right)\right\}+\frac{ns^a}{\rho^a\overline{R}\Theta}\dot{p}^a-nC\left(\dot{p}^a-\dot{p}^w\right)=0 \qquad (C1)'$$

The weak form of the above Equation (A1)', i.e., the weak form using a virtual displacement rate satisfying Equations (a11) and (a12) given below, is given by Equation (a13). Using the equation of average soil skeleton stress (equation of X= $s^w$ in the Bishop's effective stress equation) as the effective stress equation and the Green Naghdi's effective stress rate as the effective stress rate having objectivity expresses the effective stress rate tensor by Equation (a14) . In summary, the weak form of the equation of motion of the three-phase mixture consisting of the air, the water and the soil skeleton is given by Equation (a15).

[Math. 3]

$$\delta\boldsymbol{L}=\frac{\partial(\delta\boldsymbol{v})}{\partial\boldsymbol{x}} \qquad (a11)$$

$$\delta\boldsymbol{D}=\frac{1}{2}\left\{\left(\frac{\partial(\delta\boldsymbol{v})}{\partial\boldsymbol{x}}\right)+\left(\frac{\partial(\delta\boldsymbol{v})}{\partial\boldsymbol{x}}\right)^T\right\} \qquad (a12)$$

$$\int_v \left\{ \rho \ddot{v} + \left[ \left( \rho^w s^w + \rho^a s^a \right)(\mathrm{tr}\,\boldsymbol{D}) - nC\left( \rho^w - \rho^a \right)\dot{p}^w + n\left\{ \frac{s^a}{R\Theta} + C\left( \rho^w - \rho^a \right) \right\}\dot{p}^a \right](\dot{v} - b) \right\} \cdot \delta v \, dv + \int_v \dot{\boldsymbol{S}}_t \cdot \delta L \, dv = \int_a \dot{s}_t \cdot \delta v \, da \qquad \text{(a13)}$$

$\dot{s}_t = \dot{\boldsymbol{S}}_t n$ (*n* is outward unit normal covector to local surface element *da*)

[Math. 4]

$$\dot{\boldsymbol{T}} = \dot{\boldsymbol{T}}' - \left( \dot{s}^w p^w + s^w \dot{p}^w + \dot{s}^a p^a + s^a \dot{p}^a \right)\boldsymbol{I} = \overset{\circ}{\boldsymbol{T}}' + \boldsymbol{\Omega}\boldsymbol{T}' - \boldsymbol{T}'\boldsymbol{\Omega} - \left\{ C\left( \dot{p}^a - \dot{p}^w \right)p^w + s^w \dot{p}^w - C\left( \dot{p}^a - \dot{p}^w \right)p^a + s^a \dot{p}^a \right\}\boldsymbol{I}$$
$$= \overset{\circ}{\boldsymbol{T}}' + \boldsymbol{\Omega}\boldsymbol{T}' - \boldsymbol{T}'\boldsymbol{\Omega} - \left\{ s^w + C\left( p^a - p^w \right) \right\}\dot{p}^w \boldsymbol{I} - \left\{ s^a - C\left( p^a - p^w \right) \right\}\dot{p}^a \boldsymbol{I} \qquad \text{(a14)}$$

$\boldsymbol{I}$ : unit tensor

$\Omega$ : material spin tensor ($\Omega = \overset{\backslash}{\boldsymbol{R}}_s \, \boldsymbol{R}_s{}^\mathrm{T}$)

$\boldsymbol{R}_s$ : rotation tensor of soil skeleton

$\dot{\boldsymbol{T}}'$ : effective stress rate tensor

$\overset{\circ}{\boldsymbol{T}}'$ : effective stress rate tensor having objectivity

[Math. 5]

$$\int_v \rho \ddot{v} \cdot \delta v \, dv + \int_v \left\{ \overset{\circ}{\boldsymbol{T}}' \cdot \delta \boldsymbol{D} + (\mathrm{tr}\,\boldsymbol{D})\boldsymbol{T} \cdot \delta L - \boldsymbol{T}\boldsymbol{L}^\mathrm{T} \cdot \delta L \right\} dv + \int_v \left( \rho^w s^w + \rho^a s^a \right)(\mathrm{tr}\,\boldsymbol{D})(\dot{v} - b) \cdot \delta v \, dv$$
$$- \int_v nC\left( \rho^w - \rho^a \right)\dot{p}^w(\dot{v} - b) \cdot \delta v \, dv + \int_v n \cdot \left( \frac{s^a}{R\Theta} + C\left( \rho^w - \rho^a \right) \right)\dot{p}^a(\dot{v} - b) \cdot \delta v \, dv \qquad \text{(a15)}$$
$$- \int_v \left\{ s^w + C\left( p^a - p^w \right) \right\}\dot{p}^w(\mathrm{tr}\,\delta \boldsymbol{D}) \, dv - \int_v \left\{ s^a - C\left( p^a - p^w \right) \right\}\dot{p}^a(\mathrm{tr}\,\delta \boldsymbol{D}) \, dv = \int_a \dot{s}_t \cdot \delta v \, da - \int_v \left( \boldsymbol{\Omega}\boldsymbol{T}' - \boldsymbol{T}'\boldsymbol{\Omega} \right) \cdot \delta \boldsymbol{D} \, dv$$

This weak form is expressed by Equation (A1)'' given below by employing the finite element method as the spatial discretization technique and the Tamura's model or the Christian's model of allocating the pore water pressure and the pore air pressure to the element center. Specific forms of the respective matrices are described in Description of Embodiments. The M matrix, the K matrix, the $L_W{}^\mathrm{T}$ matrix and the $L_A{}^\mathrm{T}$ matrix respectively correspond to Equation (48), Equation (50), Equation (75) and Equation (77). The first term on the right side corresponds to Equation (29).

[Math. 6]

$$\mathrm{M}\{\ddot{v}^N\} + \mathrm{K}\{v^N\} - \mathrm{L}_W{}^\mathrm{T}\{\dot{p}^w\} - \mathrm{L}_A{}^\mathrm{T}\{\dot{p}^a\} = \{\dot{f}\} \qquad \text{(A1)''}$$

The soil-water coupled equation, i.e., Equation (B1)' and the soil-air coupled equation, i.e. , Equation (C1)' are then spatially discretized. Like the equation of motion of the three-phase mixture consisting of the air, the water and the soil skeleton, discretization based on the Tamura's model or the Christian's model gives Equations (B)'' and (C1)''. Specific forms of the respective matrices are described in Description of Embodiments below. The $L_{W1}$ matrix, the $L_{W2}$ matrix, the $L_{A1}$ matrix, the $L_{A2}$ matrix, the $L_{BW}$ matrix, the $L_{BA}$ matrix, the $S_{WA}$ matrix, the $S_{AC}$ matrix, the $H_W$ matrix and the $H_A$ matrix respectively correspond to Equation (81), Equation (83), Equation (82), Equation (84), Equation (20), Equation (25), Equation (79), Equation (80), Equation (14) and Equation (15) . The first term on the right side of Equation (B1)'' corresponds to Equation (44), and the first term on the right side of Equation (C1)'' corresponds to Equation (46).

[Math. 7]

$$\left( \mathrm{L}_{W1} + \mathrm{L}_{BW} \right)\{\dot{v}^N\} - \mathrm{L}_{W2}\{v^N\} + \mathrm{S}_{WA}\{\dot{p}^w\} - \mathrm{S}_{WA}\{\dot{p}^a\} + \mathrm{H}_W\{p^w\} = \{\dot{f}_w\} \qquad \text{(B1)''}$$

$$\left( \mathrm{L}_{A1} + \mathrm{L}_{BA} \right)\{\dot{v}^N\} - \mathrm{L}_{A2}\{v^N\} - \mathrm{S}_{WA}\{\dot{p}^w\} + \left( \mathrm{S}_{WA} - \mathrm{S}_{AC} \right)\{\dot{p}^a\} + \mathrm{H}_A\{p^a\} = \{\dot{f}_a\} \qquad \text{(C1)''}$$

Rewriting Equations (A1)'', (B)'' and (C1)'' in the form of ordinary differential equations gives Equation (E1) given below. In Equation (E1), the $L_{W1}$, $L_{W2}$, $L_{A1}$ and $L_{A2}$ matrices correspond to the first term regarding the time rate of volume change of the soil skeleton; the $H_W$ matrix corresponds to the second term regarding water permeability in soil; the $H_A$ matrix corresponds to the third term regarding air permeability in soil; the $S_{WA}$ matrix corresponds to the fourth term regarding the moisture characteristic of soil; the $S_{AC}$ matrix corresponds to the fifth term regarding the time rate of volume change of (the pore water and) the pore air; the $L_W^T$ matrix corresponds to the sixth term regarding the contribution of the pore water pressure to the load change rate; the $L_A^T$ matrix corresponds to the seventh term regarding the contribution of the pore air pressure to the load change rate; the $L_{BW}$ matrix corresponds to the eighth term regarding conversion of the flow rate of the pore water; the $L_{BA}$ matrix corresponds to the ninth term regarding conversion of the flow rate of the pore air; the M matrix corresponds to the tenth term regarding the mass; and the K matrix corresponds to the eleventh term regarding the tangent stiffness of the soil skeleton.

[Math. 8]

$$
\begin{bmatrix} M & 0 & 0 \\ 0 & 0 & 0 \\ 0 & 0 & 0 \end{bmatrix} \frac{d^2}{dt^2} \begin{Bmatrix} \{v^N\} \\ \{p^w\} \\ \{p^a\} \end{Bmatrix} + \begin{bmatrix} 0 & -L_W^T & -L_A^T \\ L_{W1}+L_{BW} & S_{WA} & -S_{WA} \\ L_{A1}+L_{BA} & -S_{WA} & S_{WA}-S_{AC} \end{bmatrix} \frac{d}{dt} \begin{Bmatrix} \{v^N\} \\ \{p^w\} \\ \{p^a\} \end{Bmatrix}
$$
$$
+ \begin{bmatrix} K & 0 & 0 \\ -L_{W2} & H_W & 0 \\ -L_{A2} & 0 & H_A \end{bmatrix} \begin{Bmatrix} \{v^N\} \\ \{p^w\} \\ \{p^a\} \end{Bmatrix} = \begin{Bmatrix} \{\dot{f}\} \\ \{\dot{f}_w\} \\ \{\dot{f}_a\} \end{Bmatrix} \qquad (E1)
$$

The time integration method such as the Wilson's θ method or the Newmark's β method may be used to establish simultaneous linear equations. The following describes a process conforming to the Wilson's θ method.

[Math. 9]

$$
\left\{\ddot{v}^N(\theta\Delta t)^2\right\}\Big|_{t+\theta\Delta t} = \left\{\ddot{v}^N(\theta\Delta t)^2\right\}\Big|_t + \frac{1}{2}\left\{\dddot{v}^N(\theta\Delta t)^3\right\}\Big|_t + \frac{1}{2}\left\{\dddot{v}^N(\theta\Delta t)^3\right\}\Big|_{t+\theta\Delta t}
$$

$$
\left\{\dot{v}^N(\theta\Delta t)\right\}\Big|_{t+\theta\Delta t} = \left\{\dot{v}^N(\theta\Delta t)\right\}\Big|_t + \left\{\ddot{v}^N(\theta\Delta t)^2\right\}\Big|_t + \frac{1}{3}\left\{\dddot{v}^N(\theta\Delta t)^3\right\}\Big|_t + \frac{1}{6}\left\{\dddot{v}^N(\theta\Delta t)^3\right\}\Big|_{t+\theta\Delta t}
$$

$\{\dddot{v}^N(\theta\Delta t)^3\}|_{t+\theta\Delta t}$ : column vector by multiplying column vector expression of jerk vectors of all node points at time $t = t + \theta\Delta t$ by $(\theta\Delta t)^3$

$\{\ddot{v}^N(\theta\Delta t)^2\}|_{t+\theta\Delta t}$ : column vector by multiplying column vector expression of acceleration vectors of all node points at time $t = t + \theta\Delta t$ by $(\theta\Delta t)^2$

$\{\dot{v}^N(\theta\Delta t)\}|_{t+\theta\Delta t}$ : column vector by multiplying column vector expression of velocity vectors of all node points at time $t = t + \theta\Delta t$ by $(\theta\Delta t)$

$\{\dddot{v}^N(\theta\Delta t)^3\}|_t$ : column vector by multiplying column vector expression of jerk vectors of all node points at time $t = t$ by $(\theta\Delta t)^3$

$\{\ddot{v}^N(\theta\Delta t)^2\}|_t$ : column vector by multiplying column vector expression of acceleration vectors of all node points at time $t = t$ by $(\theta\Delta t)^2$

$\{\dot{v}^N(\theta\Delta t)\}|_t$ : column vector by multiplying column vector expression of velocity vectors of all node points at time $t = t$ by $(\theta\Delta t)$

With regard to the pore water pressure and the pore air pressure, the time integration method may be employed with the trapezoidal rule. The following describes the time integration method with the trapezoidal rule.

[Math. 10]

$$
\left\{p^\alpha\right\}\Big|_{t+\theta\Delta t} = \left\{p^\alpha\right\}\Big|_t + \frac{1}{2}\left(\left\{\dot{p}^\alpha\right\}\Big|_{t+\theta\Delta t} + \left\{\dot{p}^\alpha\right\}\Big|_t\right)(\theta\Delta t) \qquad (\alpha=w,\,a)
$$

$\{p^\alpha\}|_{t+\theta\Delta t}$ : column vector expression of $p^\alpha$ of all elements at time $t = t + \theta\Delta t$

$\{\dot{p}^\alpha\}|_{t+\theta\Delta t}$ : column vector expression of rates of $p^\alpha$ of all elements at time $t = t + \theta\Delta t$

$\{p^\alpha\}|_t$ : column vector expression of $p^\alpha$ of all elements at time $t = t$

$\{\dot{p}^\alpha\}|_t$ : column vector expression of rates of $p^\alpha$ of all elements at time $t = t$

Accordingly, simultaneous linear equations expressed by Equation (F1) given below are established from Equation (E1) given above as the ordinary differential equation.

[Math. 11]

$$
\begin{bmatrix}
\dfrac{1}{(\theta\Delta t)^2}\mathrm{M} + \dfrac{1}{6}\mathrm{K} & -2\mathrm{L_W}^{\mathrm{T}} & -2\mathrm{L_A}^{\mathrm{T}} \\[2mm]
-\mathrm{L}_{\theta 1}{}^{\mathrm{W}} + \dfrac{1}{2(\theta\Delta t)}\mathrm{L_{BW}} & \mathrm{H_W}(\theta\Delta t) + 2\mathrm{S_{WA}} & -2\mathrm{S_{WA}} \\[2mm]
-\mathrm{L}_{\theta 1}{}^{\mathrm{A}} + \dfrac{1}{2(\theta\Delta t)}\mathrm{L_{BA}} & -2\mathrm{S_{WA}} & \mathrm{H_A}(\theta\Delta t) + 2\mathrm{S_{WA}} - 2\mathrm{S_{AC}}
\end{bmatrix}
\begin{Bmatrix}
\{\dddot{v}^N(\theta\Delta t)^3\}\big|_{t+\theta\Delta t} \\[2mm]
\{p^{\mathrm{w}}\}\big|_{t+\theta\Delta t} \\[2mm]
\{p^{\mathrm{a}}\}\big|_{t+\theta\Delta t}
\end{Bmatrix}
$$

$$
= \left\{
\begin{aligned}
&\{f(\theta\Delta t)\}\big|_{t+\theta\Delta t} - \mathrm{K}\left[\{v^N(\theta\Delta t)\}\big|_t + \{\dot{v}^N(\theta\Delta t)^2\}\big|_t + \tfrac{1}{3}\{\ddot{v}^N(\theta\Delta t)^3\}\big|_t\right] \\[2mm]
&\quad -2\mathrm{L_W}^{\mathrm{T}}\left[\{p^{\mathrm{w}}\}\big|_t + \tfrac{1}{2}\{\dot{p}^{\mathrm{w}}(\theta\Delta t)\}\big|_t\right] - 2\mathrm{L_A}^{\mathrm{T}}\left[\{p^{\mathrm{a}}\}\big|_t + \tfrac{1}{2}\{\dot{p}^{\mathrm{a}}(\theta\Delta t)\}\big|_t\right] \\[3mm]
&\{f_{\mathrm{w}}(\theta\Delta t)\}\big|_{t+\theta\Delta t} + \mathrm{L_{W2}}\{v^N(\theta\Delta t)\}\big|_t + \mathrm{L}_{\theta 2}{}^{\mathrm{W}}\{\dot{v}^N(\theta\Delta t)^2\}\big|_t + \mathrm{L}_{\theta 3}{}^{\mathrm{W}}\{\ddot{v}^N(\theta\Delta t)^3\}\big|_t \\[2mm]
&\quad + 2\mathrm{S_{WA}}\left[\{p^{\mathrm{w}}\}\big|_t + \tfrac{1}{2}\{\dot{p}^{\mathrm{w}}(\theta\Delta t)\}\big|_t\right] - 2\mathrm{S_{WA}}\left[\{p^{\mathrm{a}}\}\big|_t + \tfrac{1}{2}\{\dot{p}^{\mathrm{a}}(\theta\Delta t)\}\big|_t\right] \\[2mm]
&\quad - \dfrac{1}{2(\theta\Delta t)}\mathrm{L_{BW}}\left[2\{\dot{v}^N(\theta\Delta t)^2\}\big|_t + \{\ddot{v}^N(\theta\Delta t)^3\}\big|_t\right] \\[3mm]
&\{f_{\mathrm{a}}(\theta\Delta t)\}\big|_{t+\theta\Delta t} + \mathrm{L_{A2}}\{v^N(\theta\Delta t)\}\big|_t + \mathrm{L}_{\theta 2}{}^{\mathrm{A}}\{\dot{v}^N(\theta\Delta t)^2\}\big|_t + \mathrm{L}_{\theta 3}{}^{\mathrm{A}}\{\ddot{v}^N(\theta\Delta t)^3\}\big|_t \\[2mm]
&\quad + (2\mathrm{S_{WA}} - 2\mathrm{S_{AC}})\left[\{p^{\mathrm{a}}\}\big|_t + \tfrac{1}{2}\{\dot{p}^{\mathrm{a}}(\theta\Delta t)\}\big|_t\right] - 2\mathrm{S_{WA}}\left[\{p^{\mathrm{w}}\}\big|_t + \tfrac{1}{2}\{\dot{p}^{\mathrm{w}}(\theta\Delta t)\}\big|_t\right] \\[2mm]
&\quad - \dfrac{1}{2(\theta\Delta t)}\mathrm{L_{BA}}\left[2\{\dot{v}^N(\theta\Delta t)^2\}\big|_t + \{\ddot{v}^N(\theta\Delta t)^3\}\big|_t\right]
\end{aligned}
\right\}
\tag{F1}
$$

In the soil-water-air coupled analyzer of the above aspect, the rate-type simultaneous equations of motion including the jerk term of the soil skeleton may be formulated with respect to the velocity of the soil skeleton, the pore water pressure, the pore air pressure and a degree of saturation, and the deformation analysis of the soil skeleton may integrate the rate-type simultaneous equations of motion as needed by providing the geometric boundary condition, the mechanical boundary condition, the hydraulic boundary condition and the air boundary condition and may determine time history responses of the velocity field, the pore water pressure field, the pore air pressure field and a saturation degree field. Consideration of the compressibility of pore water and more detailed consideration of the degree of saturation allow for more detailed deformation analysis of the soil skeleton.

In the soil-water-air coupled analyzer of the above aspect with consideration of the compressibility of pore water, the analyzer may calculate a first term regarding a time rate of volume change of the soil skeleton, a second term regarding water permeability in soil, a third term regarding air permeability in soil, a fourth term regarding a moisture characteristic of soil, a fifth term regarding a time rate of volume change of the pore water, a sixth term regarding a time rate of volume change of the pore air, a seventh term regarding a time rate of change of saturation degree, an eighth term regarding a contribution of the pore water pressure to a load change rate, a ninth term regarding a contribution of the pore air pressure to the load change rate; a tenth term regarding a contribution of the degree of saturation to the load change rate, an eleventh term regarding conversion of the flow rate of the pore water, a twelfth term regarding conversion of the flow rate of the pore air, a thirteenth term regarding a mass and a fourteenth term regarding a tangent stiffness of the soil skeleton, based on the input data regarding the condition of the soil skeleton and the input data regarding the external force, and may establish the rate-type simultaneous equations of motion using the calculated terms. This allows for more accurate deformation analysis of the soil skeleton with regard to the unsaturated soil.

In the soil-water-air coupled analyzer of the above aspect with consideration of the compressibility of pore water, the rate-type simultaneous equations of motion may be equations established from an equation of motion of a three-phase mixture consisting of the air, the water and the soil skeleton, an equation for conservation of mass between water and the soil skeleton, an equation for conservation of mass between the air and the soil skeleton and an equation regarding moisture characteristic of soil. In this aspect, the equation of motion of the three-phase mixture consisting of the air, the water and the soil skeleton may be expressed by Equation (A2); the equation for conservation of mass between water and the soil skeleton may be expressed by Equation (B2); the equation for conservation of mass between the air and the soil skeleton may be expressed by Equation (C2) ; and the equation regarding the moisture characteristic of soil may be expressed by Equation (D2).

[Math. 12]

$$\rho \overset{\backslash\backslash}{\boldsymbol{v}}_s + \left[ \left( \rho^w s^w + \rho^a s^a \right)(\text{tr } \boldsymbol{D}_s) + \frac{ns^w \rho^w}{K_w} \overset{\backslash}{p}{}^w + \frac{ns^a}{\overline{R}\Theta} \overset{\backslash}{p}{}^a + n\left( \rho^w - \rho^a \right)\overset{\backslash}{s}{}^w \right]\left( \overset{\backslash}{\boldsymbol{v}}_s - \boldsymbol{b} \right) = \text{div } \overset{\backslash}{\boldsymbol{S}}_t \qquad (A2)$$

$$s^w \text{ div } \boldsymbol{v}_s + \frac{1}{\rho^w} \text{div}\left\{ \rho^w \frac{k^w}{\gamma_w}\left( -\text{ grad } p^w + \rho^w \boldsymbol{b} - \rho^w \overset{\backslash}{\boldsymbol{v}}_s \right) \right\} + n\overset{\backslash}{s}{}^w + \frac{ns^w}{K_w}\overset{\backslash}{p}{}^w = 0 \qquad (B2)$$

$$s^a \text{ div } \boldsymbol{v}_s + \frac{1}{\rho^a} \text{div}\left\{ \rho^a \frac{k^a}{\gamma_w}\left( -\text{ grad } p^a + \rho^a \boldsymbol{b} - \rho^a \overset{\backslash}{\boldsymbol{v}}_s \right) \right\} + n\overset{\backslash}{s}{}^a + \frac{ns^a}{\rho^a \overline{R}\Theta}\overset{\backslash}{p}{}^a = 0 \qquad (C2)$$

$$SD(p^w, p^a, s^w, e, \overset{\backslash}{p}{}^w, \overset{\backslash}{p}{}^a, \overset{\backslash}{s}{}^w, \overset{\backslash}{e}, \cdots) = 0 \quad \text{(The argument of the function depends on the model used.)}$$

$$(D2)$$

$\boldsymbol{v}_s$ : velocity vector of soil skeleton

$\boldsymbol{D}_s$ : stretching tensor of soil skeleton

$\rho$ : density of entire mixture

$\rho^w$ : density of water

$\rho^a$ : density of air

$s^w$ : degree of saturation

$s^a$ : air void ratio

$n$ : porosity

$e$ : void ratio

$p^w$ : pore water pressure

$p^a$ : pore air pressure

$C$ : specific moisture capacity

$K_w$ : volume elasticity modulus of water

$\overline{R}$ : gas constant of air

$\Theta$ : absolute temperature of air

$\boldsymbol{b}$ : body force vector per unit mass

$k^w$ : coefficient of water permeability

$k^a$ : coefficient of air permeability

$\gamma_w$ : unit volume weight of water at 4°C (standard unit volume weight)

$SD$ : one example of function expressing equation regarding moisture characteristic of soil

$$\overset{\backslash}{\boldsymbol{S}}_t = \overset{\backslash}{\boldsymbol{T}} + (\text{tr } \boldsymbol{D}_s)\boldsymbol{T} - \boldsymbol{T}\boldsymbol{L}_s^{\mathsf{T}}$$

$\boldsymbol{T}$ : total stress tensor

$\boldsymbol{L}_s$ : velocity gradient tensor of soil skeleton

As described above, in Equations, the superscript "backlash" means a material time derivative viewed from the soil skeleton. For example, "v" with two "backlashes (superscript) represents a jerk vector of the soil skeleton. As shown by Equation (D2), the equation regarding the moisture characteristic of soil depends on the model. In the description below, for the simplicity of explanation, the equation regarding the moisture characteristic of soil is expressed by the relationship between the suction and the material time derivative of suction viewed from the soil skeleton and the degree of saturation and the material time derivative of the degree of saturation viewed from the soil skeleton. The suction means the difference between the pore air pressure and the pore water pressure. As a result, the fundamental equations, i.e., Equations (A2), (B2), (C2) and (D2) are rewritten as Equations (A2)', (B2)', (C2)' and (D2)' given below.

[Math. 13]

$$\rho \ddot{\boldsymbol{v}} + \left[ \left( \rho^{\mathrm{w}} s^{\mathrm{w}} + \rho^{\mathrm{a}} s^{\mathrm{a}} \right)(\mathrm{tr}\,\boldsymbol{D}) + \frac{ns^{\mathrm{w}} \rho^{\mathrm{w}}}{\mathrm{K_w}} \dot{p}^{\mathrm{w}} + \frac{ns^{\mathrm{a}}}{\overline{\mathrm{R}}\Theta} \dot{p}^{\mathrm{a}} + n\left( \rho^{\mathrm{w}} - \rho^{\mathrm{a}} \right)\dot{s}^{\mathrm{w}} \right]\left( \dot{\boldsymbol{v}} - \boldsymbol{b} \right) = \mathrm{div}\,\dot{\boldsymbol{S}}_{\mathrm{t}} \qquad \text{(A2)'}$$

$$s^{\mathrm{w}}\,\mathrm{div}\,\boldsymbol{v} + \frac{1}{\rho^{\mathrm{w}}}\,\mathrm{div}\left\{ \rho^{\mathrm{w}} \frac{k^{\mathrm{w}}}{\gamma_{\mathrm{w}}} \left( -\,\mathrm{grad}\,p^{\mathrm{w}} + \rho^{\mathrm{w}}\boldsymbol{b} - \rho^{\mathrm{w}}\dot{\boldsymbol{v}} \right) \right\} + n\dot{s}^{\mathrm{w}} + \frac{ns^{\mathrm{w}}}{\mathrm{K_w}} \dot{p}^{\mathrm{w}} = 0 \qquad \text{(B2)'}$$

$$s^{\mathrm{a}}\,\mathrm{div}\,\boldsymbol{v} + \frac{1}{\rho^{\mathrm{a}}}\,\mathrm{div}\left\{ \rho^{\mathrm{a}} \frac{k^{\mathrm{a}}}{\gamma_{\mathrm{w}}} \left( -\,\mathrm{grad}\,p^{\mathrm{a}} + \rho^{\mathrm{a}}\boldsymbol{b} - \rho^{\mathrm{a}}\dot{\boldsymbol{v}} \right) \right\} + n\dot{s}^{\mathrm{a}} + \frac{ns^{\mathrm{a}}}{\rho^{\mathrm{a}}\overline{\mathrm{R}}\Theta} \dot{p}^{\mathrm{a}} = 0 \qquad \text{(C2)'}$$

$$SD_1\left( p^{\mathrm{s}}, \dot{p}^{\mathrm{s}}, s^{\mathrm{w}}, \dot{s}^{\mathrm{w}} \right) = 0 \qquad \text{(D2)'}$$

$p^{\mathrm{s}}$ : suction (difference between pore air pressure $p^{\mathrm{a}}$ and pore water pressure $p^{\mathrm{w}}$)

$SD_1$ : one example of function expressing equation regarding moisture

The weak form of the above Equation (A2)', i.e., the weak form using a virtual displacement rate satisfying Equations (a21) and (a22) given below, is given by Equation (a23). Using the equation of average soil skeleton stress (equation of X= $s^{\mathrm{w}}$ in the Bishop's effective stress equation) as the effective stress equation and the Green Naghdi's effective stress rate as the effective stress rate having objectivity expresses the effective stress rate tensor by Equation (a24) . In summary, the weak form of the equation of motion of the three-phase mixture consisting of the air, the water and the soil skeleton is given by Equation (a25).

[Math. 14]

$$\delta\boldsymbol{L} = \frac{\partial(\delta\boldsymbol{v})}{\partial\boldsymbol{x}} \qquad \text{(a21)}$$

$$\delta\boldsymbol{D} = \frac{1}{2}\left\{ \left( \frac{\partial(\delta\boldsymbol{v})}{\partial\boldsymbol{x}} \right) + \left( \frac{\partial(\delta\boldsymbol{v})}{\partial\boldsymbol{x}} \right)^{\mathrm{T}} \right\} \qquad \text{(a22)}$$

$$\int_v \left\{ \rho\ddot{\boldsymbol{v}} + \left[ \left( \rho^{\mathrm{w}} s^{\mathrm{w}} + \rho^{\mathrm{a}} s^{\mathrm{a}} \right)(\mathrm{tr}\,\boldsymbol{D}) + \frac{ns^{\mathrm{w}} \rho^{\mathrm{w}}}{\mathrm{K_w}} \dot{p}^{\mathrm{w}} + \frac{ns^{\mathrm{a}}}{\overline{\mathrm{R}}\Theta} \dot{p}^{\mathrm{a}} + n\left( \rho^{\mathrm{w}} - \rho^{\mathrm{a}} \right)\dot{s}^{\mathrm{w}} \right]\left( \dot{\boldsymbol{v}} - \boldsymbol{b} \right) \right\} \cdot \delta\boldsymbol{v}\mathrm{d}v + \int_v \dot{\boldsymbol{S}}_{\mathrm{t}} \cdot \delta\boldsymbol{L}\mathrm{d}v = \int_a \dot{\boldsymbol{s}}_{\mathrm{t}} \cdot \delta\boldsymbol{v}\mathrm{d}a \quad \text{(a23)}$$

$\dot{\boldsymbol{s}}_{\mathrm{t}} = \dot{\boldsymbol{S}}_{\mathrm{t}}\boldsymbol{n}$ ($\boldsymbol{n}$ is outward unit normal covector to local surface element $da$)

[Math. 15]

$$\dot{\boldsymbol{T}} = \dot{\boldsymbol{T}}' - \left( \dot{s}^{\mathrm{w}} p^{\mathrm{w}} + s^{\mathrm{w}} \dot{p}^{\mathrm{w}} + \dot{s}^{\mathrm{a}} p^{\mathrm{a}} + s^{\mathrm{a}} \dot{p}^{\mathrm{a}} \right)\boldsymbol{I} = \overset{\circ}{\boldsymbol{T}}' + \boldsymbol{\Omega}\boldsymbol{T}' - \boldsymbol{T}'\boldsymbol{\Omega} - \left\{ s^{\mathrm{w}} \dot{p}^{\mathrm{w}} + s^{\mathrm{a}} \dot{p}^{\mathrm{a}} + \left( p^{\mathrm{w}} - p^{\mathrm{a}} \right)\dot{s}^{\mathrm{w}} \right\}\boldsymbol{I}$$

$$= \overset{\circ}{\boldsymbol{T}}' + \boldsymbol{\Omega}\boldsymbol{T}' - \boldsymbol{T}'\boldsymbol{\Omega} - s^{\mathrm{w}}\dot{p}^{\mathrm{w}}\boldsymbol{I} - s^{\mathrm{a}}\dot{p}^{\mathrm{a}}\boldsymbol{I} + \left( p^{\mathrm{a}} - p^{\mathrm{w}} \right)\dot{s}^{\mathrm{w}}\boldsymbol{I} \qquad \text{(a24)}$$

$\boldsymbol{I}$ : unit tensor

$\boldsymbol{\Omega}$ : material spin tensor ($\boldsymbol{\Omega} = \dot{\boldsymbol{R}}_{\mathrm{s}}\,\boldsymbol{R}_{\mathrm{s}}^{\mathrm{T}}$)

$\boldsymbol{R}_{\mathrm{s}}$ : rotation tensor of soil skeleton

$\dot{\boldsymbol{T}}'$ : effective stress rate tensor

$\overset{\circ}{\boldsymbol{T}}'$ : effective stress rate tensor having objectivity

[Math. 16]

$$\int_{\nu}\rho\ddot{v}\cdot\delta v\,\mathrm{d}v+\int_{\nu}\left\{\overset{\circ}{T}\cdot\delta D+(\mathrm{tr}\,D)T\cdot\delta L-TL^{\mathrm{T}}\cdot\delta L\right\}\mathrm{d}v+\int_{\nu}\left(\rho^{\mathrm{w}}s^{\mathrm{w}}+\rho^{\mathrm{a}}s^{\mathrm{a}}\right)(\mathrm{tr}\,D)(\dot{v}-b)\cdot\delta v\,\mathrm{d}v$$

$$-\int_{\nu}s^{\mathrm{w}}\dot{p}^{\mathrm{w}}(\mathrm{tr}\,\delta D)\mathrm{d}v+\int_{\nu}\frac{ns^{\mathrm{w}}\rho^{\mathrm{w}}}{\mathrm{K}_{\mathrm{w}}}\dot{p}^{\mathrm{w}}(\dot{v}-b)\cdot\delta v\,\mathrm{d}v-\int_{\nu}s^{\mathrm{a}}\dot{p}^{\mathrm{a}}(\mathrm{tr}\,\delta D)\mathrm{d}v+\int_{\nu}\frac{ns^{\mathrm{a}}}{\mathrm{R}\Theta}\dot{p}^{\mathrm{a}}(\dot{v}-b)\cdot\delta v\,\mathrm{d}v \qquad (a25)$$

$$+\int_{\nu}n(\rho^{\mathrm{w}}-\rho^{\mathrm{a}})\dot{s}^{\mathrm{w}}(\dot{v}-b)\cdot\delta v\,\mathrm{d}v+\int_{\nu}(p^{\mathrm{a}}-p^{\mathrm{w}})\dot{s}^{\mathrm{w}}(\mathrm{tr}\,\delta D)\mathrm{d}v=\int_{a}\dot{s}_{\mathrm{t}}\cdot\delta v\,\mathrm{d}a-\int_{\nu}(\Omega T'-T'\Omega)\cdot\delta D\,\mathrm{d}v$$

This weak form is expressed by Equation (A2)'' given below by employing the finite element method as the spatial discretization technique and the model like the Tamura's model or the Christian's model of allocating the pore water pressure, the pore air pressure and the degree of saturation to the element center. Specific forms of the respective matrices are described in Description of Embodiments. The M matrix, the K matrix, the $L_W^T$ matrix, the $L_A^T$ matrix and the $L_S^T$ matrix respectively correspond to Equation (249), Equation (251), Equation (276), Equation (278) and Equation (280). The first term on the right side corresponds to Equation (229).

[Math. 17]

$$\mathrm{M}\{\ddot{v}^N\}+\mathrm{K}\{v^N\}-\mathrm{L_W}^{\mathrm{T}}\{\dot{p}^{\mathrm{w}}\}-\mathrm{L_A}^{\mathrm{T}}\{\dot{p}^{\mathrm{a}}\}-\mathrm{L_S}^{\mathrm{T}}\{\dot{s}^{\mathrm{w}}\}=\{\dot{f}\} \qquad (A2)''$$

The soil-water coupled equation, i.e., Equation (B2)', the soil-air coupled equation, i.e., Equation (C2)' and the equation regarding the moisture characteristic of soil, i.e., Equation (D2)' are then spatially discretized. Like the equation of motion of the three-phase mixture consisting of the air, the water and the soil skeleton, discretization based on the model like the Tamura's model or the Christian's model gives Equations (B2)'', (C2)'' and (D2)''. Specific forms of the respective matrices are described in Description of Embodiments below. The $L_{W1}$ matrix, the $L_{W2}$ matrix, the $L_{A1}$ matrix, the $L_{A2}$ matrix, the $L_{BW}$ matrix, the $L_{BA}$ matrix, the $S_{WC}$ matrix, the $S_{AC}$ matrix, the $S_{SW}$ matrix, the $H_W$ matrix and the $H_A$ matrix, the $S_C$ matrix and the $S_W$ matrix respectively correspond to Equation (287), Equation (289), Equation (288), Equation (290), Equation (220), Equation (225), Equation (282), Equation (283), Equation (284), Equation (214), Equation (215), Equation (285) and Equation (286). The first term on the right side of Equation (B2)'' corresponds to Equation (244), and the first term on the right side of Equation (C2)'' corresponds to Equation (247) .

[Math. 18]

$$\left(\mathrm{L_{W1}}+\mathrm{L_{BW}}\right)\{\dot{v}^N\}-\mathrm{L_{W2}}\{v^N\}-\mathrm{S_{WC}}\{\dot{p}^{\mathrm{w}}\}-\mathrm{S_{SW}}\{\dot{s}^{\mathrm{w}}\}+\mathrm{H_W}\{p^{\mathrm{w}}\}=\{\dot{f}_{\mathrm{w}}\} \qquad (B2)''$$

$$\left(\mathrm{L_{A1}}+\mathrm{L_{BA}}\right)\{\dot{v}^N\}-\mathrm{L_{A2}}\{v^N\}-\mathrm{S_{AC}}\{\dot{p}^{\mathrm{a}}\}+\mathrm{S_{SW}}\{\dot{s}^{\mathrm{w}}\}+\mathrm{H_A}\{p^{\mathrm{a}}\}=\{\dot{f}_{\mathrm{a}}\} \qquad (C2)''$$

$$\mathrm{S_C}\{\dot{p}^{\mathrm{w}}\}-\mathrm{S_C}\{\dot{p}^{\mathrm{a}}\}+\mathrm{S_W}\{\dot{s}^{\mathrm{w}}\}=\{0\} \qquad (D2)''$$

Rewriting Equations (A2)'', (B2)'', (C2)'' and (D2)'' in the form of ordinary differential equations gives Equation (E2) given below. In Equation (E2), the $L_{W1}$, $L_{W2}$, $L_{A1}$ and $L_{A2}$ matrices correspond to the first term regarding the time rate of volume change of the soil skeleton; the $H_W$ matrix corresponds to the second term regarding water permeability in soil; the $H_A$ matrix corresponds to the third term regarding air permeability in soil; the $S_C$ and $S_W$ matrices correspond to the fourth term regarding the moisture characteristic of soil; the $S_{WC}$ matrix corresponds to the fifth term regarding the time rate of volume change of the pore water; the $S_{AC}$ matrix corresponds to the sixth term regarding the time rate of volume change of the pore air; the $S_{SW}$ matrix corresponds to the seventh term regarding the time rate of change of saturation degree; the $L_W^T$ matrix corresponds to the eighth term regarding the contribution of the pore water pressure to the load change rate; the $L_A^T$ matrix corresponds to the ninth term regarding the contribution of the pore air pressure to the load change rate; the $L_S^T$ matrix corresponds to the tenth term regarding the contribution of the degree of saturation to the load change rate; the $L_{BW}$ matrix corresponds to the eleventh term regarding conversion of the flow rate of the pore water; the $L_{BA}$ matrix corresponds to the twelfth term regarding conversion of the flow rate of the pore air; the M matrix corresponds to the thirteenth term regarding the mass; and the K matrix corresponds to the fourteenth term regarding the tangent stiffness of the soil skeleton.

[Math. 19]

$$\begin{bmatrix} M & 0 & 0 & 0 \\ 0 & 0 & 0 & 0 \\ 0 & 0 & 0 & 0 \\ 0 & 0 & 0 & 0 \end{bmatrix} \frac{d^2}{dt^2} \begin{Bmatrix} \{v^N\} \\ \{p^w\} \\ \{p^a\} \\ \{s^w\} \end{Bmatrix} + \begin{bmatrix} 0 & -L_W^T & -L_A^T & -L_S^T \\ L_{W1}+L_{BW} & -S_{WC} & 0 & -S_{SW} \\ L_{A1}+L_{BA} & 0 & -S_{AC} & S_{SW} \\ 0 & S_C & -S_C & S_W \end{bmatrix} \frac{d}{dt} \begin{Bmatrix} \{v^N\} \\ \{p^w\} \\ \{p^a\} \\ \{s^w\} \end{Bmatrix}$$

$$+ \begin{bmatrix} K & 0 & 0 & 0 \\ -L_{W2} & H_W & 0 & 0 \\ -L_{A2} & 0 & H_A & 0 \\ 0 & 0 & 0 & 0 \end{bmatrix} \begin{Bmatrix} \{v^N\} \\ \{p^w\} \\ \{p^a\} \\ \{s^w\} \end{Bmatrix} = \begin{Bmatrix} \{\dot{f}\} \\ \{\dot{f}_w\} \\ \{\dot{f}_a\} \\ \{0\} \end{Bmatrix} \tag{E2}$$

The time integration method such as the Wilson's θ method or the Newmark's P method may be used to establish simultaneous linear equations. The following describes a process conforming to the Wilson's θ method.

[Math. 20]

$$\left. \{\dot{v}^N(\theta\Delta t)^2\}\right|_{t+\theta\Delta t} = \left. \{\dot{v}^N(\theta\Delta t)^2\}\right|_{t} + \frac{1}{2}\left. \{\ddot{v}^N(\theta\Delta t)^3\}\right|_{t} + \frac{1}{2}\left. \{\dddot{v}^N(\theta\Delta t)^3\}\right|_{t+\theta\Delta t}$$

$$\left. \{v^N(\theta\Delta t)\}\right|_{t+\theta\Delta t} = \left. \{v^N(\theta\Delta t)\}\right|_{t} + \left. \{\dot{v}^N(\theta\Delta t)^2\}\right|_{t} + \frac{1}{3}\left. \{\ddot{v}^N(\theta\Delta t)^3\}\right|_{t} + \frac{1}{6}\left. \{\dddot{v}^N(\theta\Delta t)^3\}\right|_{t+\theta\Delta t}$$

$\{\dddot{v}^N(\theta\Delta t)^3\}|_{t+\theta\Delta t}$ : column vector by multiplying column vector expression of jerk vectors of all node points at time $t = t + \theta\Delta t$ by $(\theta\Delta t)^3$

$\{\ddot{v}^N(\theta\Delta t)^2\}|_{t+\theta\Delta t}$ : column vector by multiplying column vector expression of acceleration vectors of all node points at time $t = t + \theta\Delta t$ by $(\theta\Delta t)^2$

$\{v^N(\theta\Delta t)\}|_{t+\theta\Delta t}$ : column vector by multiplying column vector expression of velocity vectors of all node points at time $t = t + \theta\Delta t$ by $(\theta\Delta t)$

$\dddot{v}^N(\theta\Delta t)^3\}|_t$ : {column vector by multiplying column vector expression of jerk vectors of all node points at time $t = t$ by $(\theta\Delta t)^3$

$\{\ddot{v}^N(\theta\Delta t)^2\}|_t$ : column vector by multiplying column vector expression of acceleration vectors of all node points at time $t = t$ by $(\theta\Delta t)^2$

$\{v^N(\theta\Delta t)\}|_t$ : column vector by multiplying column vector expression of velocity vectors of all node points at time $t = t$ by $(\theta\Delta t)$

With regard to the pore water pressure, the pore air pressure and the degree of saturation, the time integration method may be employed, for example, with the trapezoidal rule. The following describes the time integration method with the trapezoidal rule.

[Math. 21]

$$\left. \{\alpha\}\right|_{t+\theta\Delta t} = \left. \{\alpha\}\right|_{t} + \frac{1}{2}\left( \left. \{\dot{\alpha}\}\right|_{t+\theta\Delta t} + \left. \{\dot{\alpha}\}\right|_{t} \right)(\theta\Delta t) \qquad (\alpha = p^w, p^a, s^w)$$

$\{\alpha\}|_{t+\theta\Delta t}$ : column vector expression of $\alpha$ of all elements at time $t = t + \theta\Delta t$
$\{\dot{\alpha}\}|_{t+\theta\Delta t}$ : column vector expression of rates of $\alpha$ of all elements at time $t = t + \theta\Delta t$
$\{\alpha\}|_{t}$ : column vector expression of $\alpha$ of all elements at time $t = t$
$\{\dot{\alpha}\}|_{t}$ : column vector expression of rates of $\alpha$ of all elements at time $t = t$

Accordingly, simultaneous linear equations expressed by Equation (F2) given below are established from Equation (E2) given above as the ordinary differential equation.

[Math. 22]

$$
\begin{bmatrix}
\dfrac{1}{(\theta\Delta t)^2}M+\dfrac{1}{6}K & -2L_W{}^T & -2L_A{}^T & -2L_S{}^T \\[2ex]
-L_{\theta1}{}^W+\dfrac{1}{2(\theta\Delta t)}L_{BW} & H_W(\theta\Delta t)-2S_{WC} & 0 & -2S_{SW} \\[2ex]
-L_{\theta1}{}^A+\dfrac{1}{2(\theta\Delta t)}L_{BA} & 0 & H_A(\theta\Delta t)-2S_{AC} & 2S_{SW} \\[2ex]
0 & 2S_C & -2S_C & 2S_W
\end{bmatrix}
\left\{
\begin{array}{c}
\left.\{\ddot{v}^N(\theta\Delta t)^3\}\right|_{t+\theta\Delta t} \\[1ex]
\left.\{p^w\}\right|_{t+\theta\Delta t} \\[1ex]
\left.\{p^a\}\right|_{t+\theta\Delta t} \\[1ex]
\left.\{s^w\}\right|_{t+\theta\Delta t}
\end{array}
\right\}
$$

$$
=\left\{
\begin{array}{l}
\left.\{f(\theta\Delta t)\}\right|_{t+\theta\Delta t}-K\left[\left.\{v^N(\theta\Delta t)\}\right|_t+\left.\{\dot{v}^N(\theta\Delta t)^2\}\right|_t+\dfrac{1}{3}\left.\{\ddot{v}^N(\theta\Delta t)^3\}\right|_t\right] \\[2ex]
-2L_W{}^T\left[\left.\{p^w\}\right|_t+\dfrac{1}{2}\left.\{\dot{p}^w(\theta\Delta t)\}\right|_t\right]-2L_A{}^T\left[\left.\{p^a\}\right|_t+\dfrac{1}{2}\left.\{\dot{p}^a(\theta\Delta t)\}\right|_t\right]-2L_S{}^T\left[\left.\{s^w\}\right|_t+\dfrac{1}{2}\left.\{\dot{s}^w(\theta\Delta t)\}\right|_t\right] \\[3ex]
\left.\{f_w(\theta\Delta t)\}\right|_{t+\theta\Delta t}+L_{W2}\left.\{v^N(\theta\Delta t)\}\right|_t+L_{\theta2}{}^W\left.\{\dot{v}^N(\theta\Delta t)^2\}\right|_t+L_{\theta3}{}^W\left.\{\ddot{v}^N(\theta\Delta t)^3\}\right|_t \\[1.5ex]
-2S_{WC}\left[\left.\{p^w\}\right|_t+\dfrac{1}{2}\left.\{\dot{p}^w(\theta\Delta t)\}\right|_t\right]-2S_{SW}\left[\left.\{s^w\}\right|_t+\dfrac{1}{2}\left.\{\dot{s}^w(\theta\Delta t)\}\right|_t\right] \\[1.5ex]
-\dfrac{1}{2(\theta\Delta t)}L_{BW}\left[2\left.\{\dot{v}^N(\theta\Delta t)^2\}\right|_t+\left.\{\ddot{v}^N(\theta\Delta t)^3\}\right|_t\right] \\[3ex]
\left.\{f_a(\theta\Delta t)\}\right|_{t+\theta\Delta t}+L_{A2}\left.\{v^N(\theta\Delta t)\}\right|_t+L_{\theta2}{}^A\left.\{\dot{v}^N(\theta\Delta t)^2\}\right|_t+L_{\theta3}{}^A\left.\{\ddot{v}^N(\theta\Delta t)^3\}\right|_t \\[1.5ex]
-2S_{AC}\left[\left.\{p^a\}\right|_t+\dfrac{1}{2}\left.\{\dot{p}^a(\theta\Delta t)\}\right|_t\right]+2S_{SW}\left[\left.\{s^w\}\right|_t+\dfrac{1}{2}\left.\{\dot{s}^w(\theta\Delta t)\}\right|_t\right] \\[1.5ex]
-\dfrac{1}{2(\theta\Delta t)}L_{BA}\left[2\left.\{\dot{v}^N(\theta\Delta t)^2\}\right|_t+\left.\{\ddot{v}^N(\theta\Delta t)^3\}\right|_t\right] \\[3ex]
2S_C\left[\left.\{p^w\}\right|_t+\dfrac{1}{2}\left.\{\dot{p}^w(\theta\Delta t)\}\right|_t\right]-2S_C\left[\left.\{p^a\}\right|_t+\dfrac{1}{2}\left.\{\dot{p}^a(\theta\Delta t)\}\right|_t\right]+2S_W\left[\left.\{s^w\}\right|_t+\dfrac{1}{2}\left.\{\dot{s}^w(\theta\Delta t)\}\right|_t\right]
\end{array}
\right\}
$$

(F2)

In the soil-water-air coupled analyzer of the above aspect, the analyzer may establish the rate-type simultaneous equations of motion using a numerical analysis method such as a finite element method or a finite difference method. This facilitates the analysis.

In the soil-water-air coupled analyzer of the above aspect, the data regarding the condition of the soil skeleton may include data regarding a profile of each element of a plurality of elements constituting the soil skeleton and data regarding a relationship to an adjacent element and/or adjacent elements. In this aspect, the data regarding the profile of each element may include data regarding a soil profile. This allows for setting the profiles with respect to each of the plurality of elements constituting the soil skeleton, and also allows for setting the relationship between each set of adjacent elements. This accordingly enables deformation analysis of the soil skeleton to be performed with high accuracy with regard to the soil foundation of any unsaturated intermediate soil from sand to clay. This also enables deformation analysis of the soil skeleton to be performed with high accuracy with regard to the soil foundation of unsaturated soil having the layered structure of clay, sand and intermediate soil.

In the soil-water-air coupled analyzer of the above aspect, the data regarding the external force may include data regarding a load and a displacement applied to a soil-water-air coupled system. The data regarding the external force may include data regarding a vibration applied to a soil-water-air coupled system. This allows for deformation analysis of the soil skeleton with regard to the load and the displacement, as well as deformation analysis of the soil skeleton with regard to the vibration.

In the soil-water-air coupled analyzer of the above aspect, the analyzer may perform the analysis with changing the data regarding the external force a plurality of times. In this aspect, when the data regarding the external force is changed, the analyzer may perform the analysis using a result of the analysis prior to the change as data regarding an initial state of the soil skeleton. This enables deformation analysis of the soil skeleton with respect to unsaturated soil to be performed repeatedly according to the state of soil and the state of loading. This allows for static deformation analysis subsequent to dynamic deformation analysis or dynamic deformation analysis subsequent to static deformation analysis with respect to unsaturated soil.

According to another aspect, there is provided a soil-water-air coupled analyzing method, comprising: establishing rate-type simultaneous equations of motion including a jerk term of the soil skeleton with respect to a velocity of a soil skeleton, a pore water pressure and a pore air pressure, based on data regarding a condition of the soil skeleton and data regarding an external force; integrating the rate-type simultaneous equations of motion as needed by providing a geometric boundary condition with regard to a displacement, a displacement rate or an acceleration, a mechanical boundary condition with regard to a stress or a stress rate, a hydraulic boundary condition with regard to a pore water pressure or a total water head and a flow rate of pore water and an air boundary condition with regard to a pore air pressure or a flow rate of pore air; and determining time history responses of a velocity field, a pore water pressure field and a pore air pressure field, so as to perform deformation analysis of the soil skeleton.

The soil-water-air coupled analyzing method of this aspect integrates the rate-type simultaneous equations of motion including the jerk term of the soil skeleton with respect to the velocity of the soil skeleton, the pore water pressure and the pore air pressure as needed by providing the geometric boundary condition with regard to the displacement, the displacement rate or the acceleration, the mechanical boundary condition with regard to the stress or the stress rate, the hydraulic boundary condition with regard to the pore water pressure or the total water head and the flow rate of pore water and the air boundary condition with regard to the pore air pressure or the flow rate of pore air, and determines the time history responses of the velocity field, the pore water pressure field and the pore air pressure field. With regard to unsaturated soil having the air in addition to water in voids of the soil skeleton, this allows for deformation analysis of the soil skeleton by dynamic analysis under large influence of acceleration, while allowing for deformation analysis of the soil skeleton by static analysis under small influence of acceleration. Accordingly, this allows for deformation analysis of the soil skeleton, whether static analysis or dynamic analysis, with regard to soil having water unsaturated in voids of the soil skeleton, i.e., unsaturated soil having the air in addition to water in voids of the soil skeleton, as well as saturated soil having water saturated in voids of the soil skeleton.

The soil-water-air coupled analyzing method of this aspect may further comprise: calculating a first term regarding a time rate of volume change of the soil skeleton, a second term regarding water permeability in soil, a third term regarding air permeability in soil, a fourth term regarding a moisture characteristic of soil, a fifth term regarding time rates of volume change of the pore water and the pore air, a sixth term regarding a contribution of the pore water pressure to a load change rate; a seventh term regarding a contribution of the pore air pressure to the load change rate, an eighth term regarding conversion of the flow rate of the pore water, a ninth term regarding conversion of the flow rate of the pore air, a tenth term regarding a mass and an eleventh term regarding a tangent stiffness of the soil skeleton, based on the data regarding the condition of the soil skeleton and the data regarding the external force, and establishing the rate-type simultaneous equations of motion using the calculated terms. This allows for more accurate deformation analysis of the soil skeleton with regard to the unsaturated soil.

In the soil-water-air coupled analyzing method of the above aspect, the rate-type simultaneous equations of motion may be equations established from an equation of motion of a three-phase mixture consisting of the air, the water and the soil skeleton, an equation for conservation of mass between water and the soil skeleton and an equation for conservation of mass between the air and the soil skeleton. In this aspect, the equation of motion of the three-phase mixture consisting of the air, the water and the soil skeleton may be expressed by Equation (A1) given above; the equation for conservation of mass between water and the soil skeleton may be expressed by Equation (B1) given above; and the equation for conservation of mass between the air and the soil skeleton may be expressed by Equation (C1) given above. Like the soil-water-air coupled analyzer of the above aspect, Equations (A1), (B1) and (C1) are respectively rewritten as Equations (A1)', (B1) ' and (C1) ' given above on the assumption that pore water is an incompressible fluid (Kw is infinite). Equation (A1)' is rewritten as Equation (A1)'' given above by employing the finite element method as the spatial discretization technique and the Tamura's model or the Christian's model of allocating the pore water pressure and the pore air pressure to the element center. Spatial discretization of Equations (B1)' and (C1) ' similarly based on the Tamura's model or the Christian's model gives Equations (B1)'' and (C1)'' given above. Rewriting Equations (A1)'', (B1)'' and (C1)'' in the form of ordinary differential equations gives Equation (E1) given above. Employing the method conforming to the Wilson's $\theta$ method in order to establish simultaneous linear equations, the simultaneous linear equations of Equation (F1) given above are eventually established.

In the soil-water-air coupled analyzing method of the above aspect, the rate-type simultaneous equations of motion including the jerk term of the soil skeleton may be formulated with respect to the velocity of the soil skeleton, the pore water pressure, the pore air pressure and a degree of saturation, and the deformation analysis of the soil skeleton may integrate the rate-type simultaneous equations of motion as needed by providing the geometric boundary condition, the mechanical boundary condition, the hydraulic boundary condition and the air boundary condition and may determine time history responses of the velocity field, the pore water pressure field, the pore air pressure field and a saturation degree field. Consideration of the compressibility of pore water and more detailed consideration of the degree of saturation allow for more detailed deformation analysis of the soil skeleton.

The soil-water-air coupled analyzing method of the above aspect with consideration of the compressibility of pore water may further comprise: calculating a first term regarding a time rate of volume change of the soil skeleton, a second term regarding water permeability in soil, a third term regarding air permeability in soil, a fourth term regarding a moisture characteristic of soil, a fifth term regarding a time rate of volume change of the pore water, a sixth term regarding a time rate of volume change of the pore air, a seventh term regarding a time rate of change of saturation degree, an eighth term regarding a contribution of the pore water pressure to a load change rate, a ninth term regarding a contribution of the pore air pressure to the load change rate; a tenth term regarding a contribution of the degree of saturation to the load change rate; an eleventh term regarding conversion of the flow rate of the pore water, a twelfth term regarding conversion of the flow rate of the pore air, a thirteenth term regarding a mass and a fourteenth term regarding a tangent stiffness of the soil skeleton, based on the data regarding the condition of the soil skeleton and the data regarding the external force, and establishing the rate-type simultaneous equations of motion using the calculated terms. This allows for more accurate deformation analysis of the soil skeleton with regard to the unsaturated soil.

In the soil-water-air coupled analyzing method of the above aspect with consideration of the compressibility of pore water, the rate-type simultaneous equations of motion may be equations established from an equation of motion of a three-phase mixture consisting of the air, the water and the soil skeleton, an equation for conservation of mass between water and the soil skeleton, an equation for conservation of mass between the air and the soil skeleton and an equation regarding moisture characteristic of soil. In this aspect, the equation of motion of the three-phase mixture consisting of the air, the water and the soil skeleton may be expressed by Equation (A2) given above; the equation for conservation of mass between water and the soil skeleton may be expressed by Equation (B2) given above; the equation for conservation of mass between the air and the soil skeleton may be expressed by Equation (C2) given above; and the equation regarding the moisture characteristic of soil may be expressed by Equation (D2) given above. Like the soil-water-air coupled analyzer of the above aspect, Equations (A2), (B2), (C2) and (D2) are respectively rewritten as Equations (A2)', (B2)', (C2)' and (D2)' given above on the assumption that the equation regarding the moisture characteristic of soil is expressed by the relationship between the suction and the material time derivative of suction viewed from the soil skeleton and the degree of saturation and the material time derivative of the degree of saturation viewed from the soil skeleton. Equation (A2)' is rewritten as Equation (A2)'' given above by employing the finite element method as the spatial discretization technique and the model like the Tamura's model or the Christian's model of allocating the pore water pressure, the pore air pressure and the degree of saturation to the element center. Spatial discretization of Equations (B2)', (C2)' and (D2)' similarly based on the model like the Tamura's model or the Christian' s model gives Equations (B2)'', (C2)'' and (D2)'' given above. Rewriting Equations (A2)'', (B2)'', (C2)'' and (D2)'' in the form of ordinary differential equations gives Equation (E2) given above. Employing the method conforming to the Wilson's θ method in order to establish simultaneous linear equations, the simultaneous linear equations of Equation (F2) given above are eventually established.

According to another aspect, there is provided a soil-water-air coupled analyzing program configured to perform deformation analysis of a soil skeleton, comprising: a data input module configured to input data regarding a condition of the soil skeleton and data regarding an external force; and an analyzing module configured to establish rate-type simultaneous equations including a jerk term of the soil skeleton of motion with respect to a velocity of the soil skeleton, a pore water pressure and a pore air pressure, based on the input data regarding the condition of the soil skeleton and the input data regarding the external force, to integrate the rate-type simultaneous equations of motion as needed by providing a geometric boundary condition with regard to a displacement, a displacement rate or an acceleration, a mechanical boundary condition with regard to a stress or a stress rate, a hydraulic boundary condition with regard to a pore water pressure or a total water head and a flow rate of pore water and an air boundary condition with regard to a pore air pressure or a flow rate of pore air and to determine time history responses of a velocity field, a pore water pressure field and a pore air pressure field, so as to perform deformation analysis of the soil skeleton.

The soil-water-air coupled analyzing program of this aspect is installed and activated in a computer, so that the computer serves as the soil-water-air coupled analyzer of the above aspect. More specifically, the computer serves as the soil-water-air coupled analyzer that integrates the rate-type simultaneous equations of motion including the jerk term of the soil skeleton with respect to the velocity of the soil skeleton, the pore water pressure and the pore air pressure as needed by providing the geometric boundary condition with regard to the displacement, the displacement rate or the acceleration, the mechanical boundary condition with regard to the stress or the stress rate, the hydraulic boundary condition with regard to the pore water pressure or the total water head and the flow rate of pore water and the air boundary condition with regard to the pore air pressure or the flow rate of pore water, and determines the time history responses of the velocity field, the pore water pressure field and the pore air pressure field. With regard to unsaturated soil having the air in addition to water in voids of the soil skeleton, this enables the computer to serve as the device for deformation analysis of the soil skeleton by dynamic analysis under large influence of acceleration, while allowing for deformation analysis of the soil skeleton by static analysis under small influence of acceleration. Accordingly, this enables the computer to serve as the device for deformation analysis of the soil skeleton, whether static analysis or dynamic analysis, with regard to soil having water unsaturated in voids of the soil skeleton, i.e., unsaturated soil having the air in addition to water in voids of the soil skeleton, as well as saturated soil having water saturated in voids of the soil skeleton.

In the soil-water-air coupled analyzing program of this aspect, the analyzing module may calculate a first term regarding a time rate of volume change of the soil skeleton, a second term regarding water permeability in soil, a third term regarding air permeability in soil, a fourth term regarding a moisture characteristic of soil, a fifth term regarding time rates of volume change of the pore water and the pore air, a sixth term regarding a contribution of the pore water pressure to a load change rate; a seventh term regarding a contribution of the pore air pressure to the load change rate, an eighth term regarding conversion of the flow rate of the pore water, a ninth term regarding conversion of the flow rate of the pore air, a tenth term regarding a mass and an eleventh term regarding a tangent stiffness of the soil skeleton, based on the data regarding the condition of the soil skeleton and the data regarding the external force, and may establish the rate-type simultaneous equations of motion using the calculated terms. This enables the computer to serve as the device for more accurate deformation analysis of the soil skeleton with regard to the unsaturated soil.

In the soil-water-air coupled analyzing program of the above aspect, the rate-type simultaneous equations of motion may be equations established from an equation of motion of a three-phase mixture consisting of the air, the water and the soil skeleton, an equation for conservation of mass between water and the soil skeleton and an equation for conservation of mass between the air and the soil skeleton. In this aspect, the equation of motion of the three-phase mixture consisting of the air, the water and the soil skeleton may be expressed by Equation (A1) given above; the equation for conservation of mass between water and the soil skeleton may be expressed by Equation (B1) given above; and the equation for conservation of mass between the air and the soil skeleton may be expressed by Equation (C1) given above. Like the soil-water-air coupled analyzer of the above aspect, Equations (A1), (B1) and (C1) are respectively rewritten as Equations (A1)', (B1) ' and (C1) ' given above on the assumption that pore water is an incompressible fluid (Kw is infinite). Equation (A1)' is rewritten as Equation (A1)'' given above by employing the finite element method as the spatial discretization technique and the Tamura's model or the Christian's model of allocating the pore water pressure and the pore air pressure to the element center. Spatial discretization of Equations (B1)' and (C1) ' similarly based on the Tamura's model or the Christian's model gives Equations (B1)'' and (C1)'' given above. Rewriting Equations (A1)'', (B1)'' and (C1)'' in the form of ordinary differential equations gives Equation (E1) given above. Employing the method conforming to the Wilson's θ method in order to establish simultaneous linear equations, the simultaneous linear equations of Equation (F1) given above are eventually established.

In the soil-water-air coupled analyzing program of the above aspect, the rate-type simultaneous equations of motion including the jerk term of the soil skeleton may be formulated with respect to the velocity of the soil skeleton, the pore water pressure, the pore air pressure and a degree of saturation, and the deformation analysis of the soil skeleton may integrate the rate-type simultaneous equations of motion as needed by providing the geometric boundary condition, the mechanical boundary condition, the hydraulic boundary condition and the air boundary condition and may determine time history responses of the velocity field, the pore water pressure field, the pore air pressure field and a saturation degree field. Consideration of the compressibility of pore water and more detailed consideration of the degree of saturation enables the computer to serve as the device for more detailed deformation analysis of the soil skeleton.

In the soil-water-air coupled analyzing program of the above aspect with consideration of the compressibility of pore water, the analyzing module may calculate a first term regarding a time rate of volume change of the soil skeleton, a second term regarding water permeability in soil, a third term regarding air permeability in soil, a fourth term regarding a moisture characteristic of soil, a fifth term regarding a time rate of volume change of the pore water, a sixth term regarding a time rate of volume change of the pore air, a seventh term regarding a time rate of change of saturation degree, an eighth term regarding a contribution of the pore water pressure to a load change rate, a ninth term regarding a contribution of the pore air pressure to the load change rate; a tenth term regarding a contribution of the degree of saturation to the load change rate; an eleventh term regarding conversion of the flow rate of the pore water, a twelfth term regarding conversion of the flow rate of the pore air, a thirteenth term regarding a mass and a fourteenth term regarding a tangent stiffness of the soil skeleton, based on the data regarding the condition of the soil skeleton and the data regarding the external force, and may establish the rate-type simultaneous equations of motion using the calculated terms. This enables the computer to serve as the device for more accurate deformation analysis of the soil skeleton with regard to the unsaturated soil.

In the soil-water-air coupled analyzing program of the above aspect, the rate-type simultaneous equations of motion may be equations established from an equation of motion of a three-phase mixture consisting of the air, the water and the soil skeleton, an equation for conservation of mass between water and the soil skeleton, an equation for conservation of mass between the air and the soil skeleton and an equation regarding moisture characteristic of soil. In this aspect, the equation of motion of the three-phase mixture consisting of the air, the water and the soil skeleton may be expressed by Equation (A2) given above; the equation for conservation of mass between water and the soil skeleton may be expressed by Equation (B2) given above; the equation for conservation of mass between the air and the soil skeleton may be expressed by Equation (C2) given above; and the equation regarding the moisture characteristic of soil may be expressed by Equation (D2) given above. Like the soil-water-air coupled analyzer of the above aspect, Equations (A2), (B2), (C2) and (D2) are respectively rewritten as Equations (A2)', (B2)', (C2)' and (D2)' given above by expression based on the relationship between the suction and the material time derivative of suction viewed from the soil skeleton and the degree of saturation and the material time derivative of the degree of saturation viewed from the soil skeleton. Equation (A2)' is rewritten as Equation (A2) " given above by employing the finite element method as the spatial discretization technique and the model like the Tamura's model or the Christian's model of allocating the pore water pressure, the pore air pressure and the degree of saturation to the element center. Spatial discretization of Equations (B2)', (C2)' and (D2)' similarly based on the model like the Tamura's model or the Christian's model gives Equations (B2)", (C2)" and (D2)'' given above. Rewriting Equations (A2)'', (B2)'', (C2)'' and (D2)'' in the form of ordinary differential equations gives Equation (E2) given above. Employing the method conforming to the Wilson's θ method in order to establish simultaneous linear equations, the simultaneous linear equations of Equation (F2) given above are eventually established.

BRIEF DESCRIPTION OF DRAWINGS

Fig. 1 is a configuration illustrating the general configuration of a soil-water-air coupled analyzer 20 according to a first embodiment of the invention;
Fig. 2 is a flowchart showing one exemplary process according to a soil-water-air coupled analyzing program 30 of the first embodiment;
Fig. 3 is a diagram illustrating a specific example on the assumption that a soil foundation as the analysis object has four elements expressed by a 2x2 matrix;
Fig. 4 is a diagram illustrating local nodes in each element;
Fig. 5 is a diagram illustrating one exemplary file as a specific example for inputting the settings of analysis conditions;
Fig. 6 is a diagram illustrating one exemplary file as a specific example for inputting the settings of the analysis conditions;
Fig. 7 is a diagram illustrating one exemplary file as a specific example for inputting the settings of soils;
Fig. 8 is a diagram illustrating one exemplary file for inputting the settings of a solid soil model as a specific example;
Fig. 9 is a diagram illustrating modelling the flows of pore water and pore air under a two-dimensional plane strain condition;
Fig. 10 is a diagram illustrating modelling the flows of pore water and pore air under an axisymmetric condition;
Fig. 11 is a diagram illustrating modelling the flows of pore water and pore air under a three-dimensional condition;
Fig. 12 is a diagram illustrating one example of an output file;
Fig. 13 is a flowchart showing one exemplary process of performing the soil-water-air coupled analyzing program a plurality of times with using the results of the previous analysis as new analysis conditions or initial conditions;
Figs. 14a and 14b are diagrams illustrating exemplary simulation results of an undrained and unexhausted triaxial compression test performed at an initial degree of saturation equal to a maximum degree of saturation;
Figs. 15a and 15b are diagrams illustrating exemplary simulation results of an drained and exhausted triaxial compression test performed at an initial degree of saturation equal to a maximum degree of saturation;
Figs. 16a and 16b are diagrams illustrating exemplary simulation results of an undrained and unexhausted triaxial compression test performed with regard to five different initial degrees of saturation;
Fig. 17 is a flowchart showing one exemplary process according to a soil-water-air coupled analyzing program 230 according to a second embodiment;
Figs. 18a and 18b are diagrams illustrating exemplary simulation results of an undrained and unexhausted triaxial compression test performed with regard to five different initial degrees of saturation using the method of the second embodiment;
Fig. 19 is a diagram illustrating a distribution of saturation degree by loading of embankment in the unsaturated state on the clay foundation in the substantially saturated state; and
Fig. 20 is a diagram illustrating a process of generating a phreatic surface in the embankment with a rise of water level.

DESCRIPTION OF EMBODIMENTS

The following describes some aspects of the invention with reference to embodiments.

First Embodiment

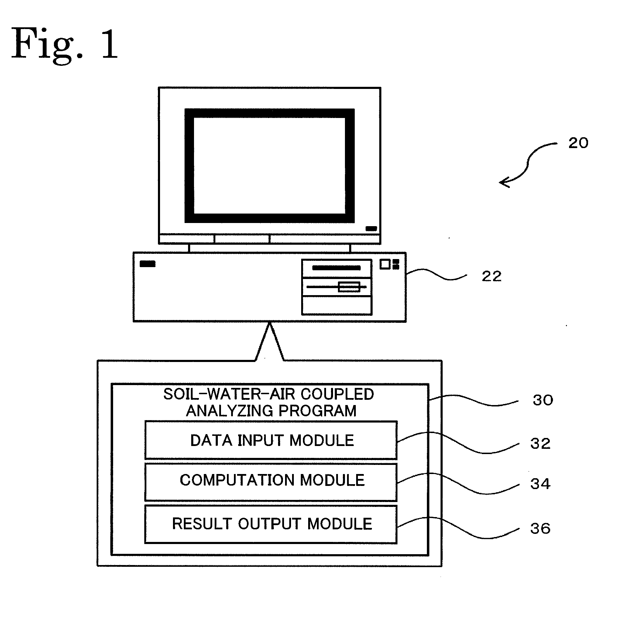

Fig. 1 is a configuration diagram illustrating the general configuration of a soil-water-air coupled analyzer 20 according to a first embodiment of the invention. As illustrated, the soil-water-air coupled analyzer 20 of the first embodiment is configured by installing a soil-water-air coupled analyzing program 30 as application software in a general-purpose computer 22. The soil-water-air coupled analyzing program 30 includes : a data input module 32 configured to input data for setting the analysis conditions, the soil profiles and a solid soil model; a computation module 34 configured to analyze the mechanical behaviors of unsaturated soil from the input data according to an elasto-plastic constitutive equation; and a result output module 36 configured to output the result of the analysis.

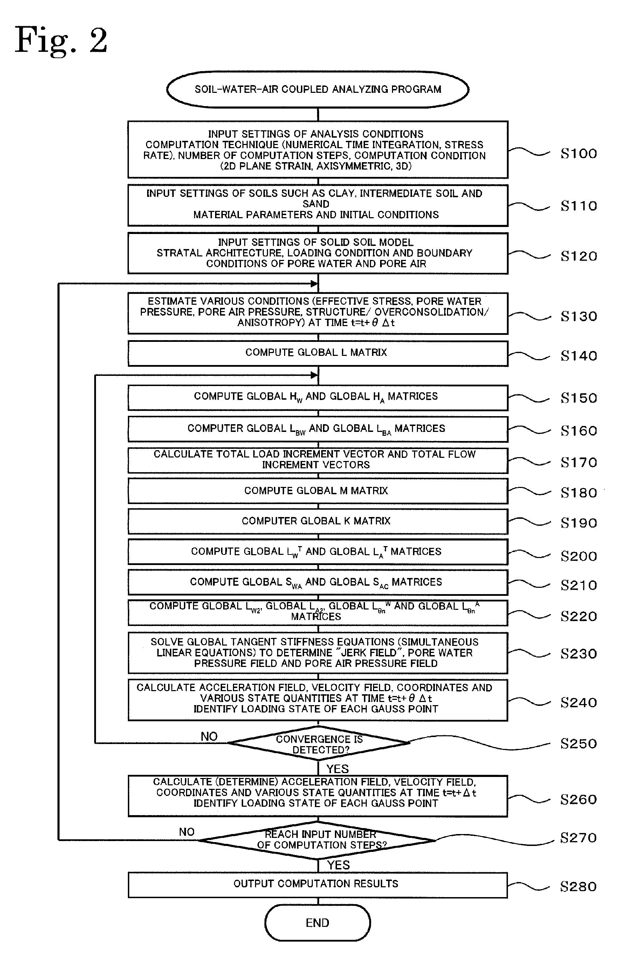

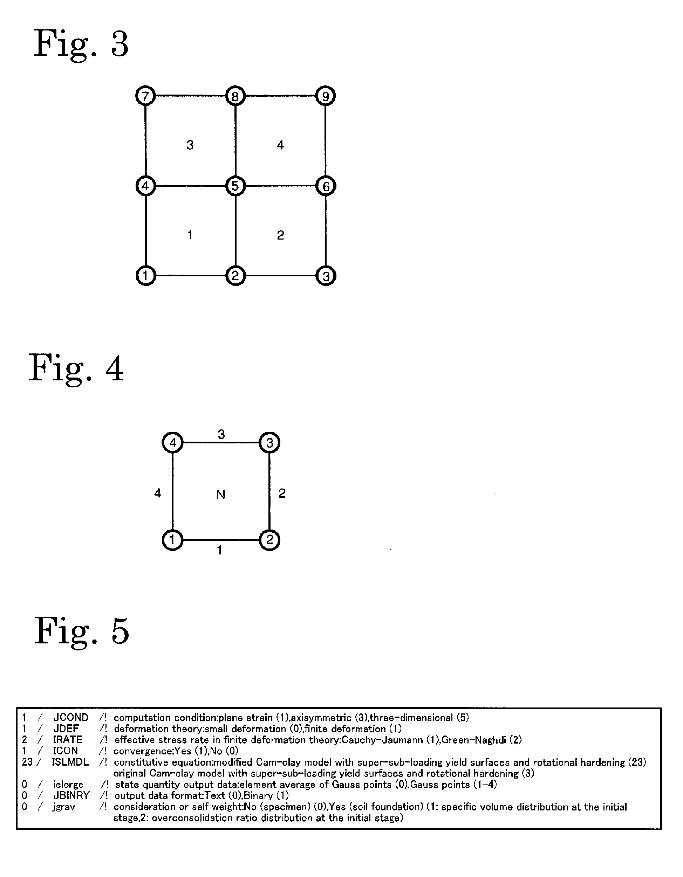

Fig. 2 is a flowchart showing one exemplary process according to the soil-water-air coupled analyzing program 30 of the first embodiment. The process of the soil-water-air coupled analyzing program 30 is described below with reference to this flowchart. For the simplicity of explanation, the process of the soil-water-air coupled analyzing program 30 is described with regard to a specific example on the assumption that a soil foundation as the analysis object has four elements expressed by a 2x2 matrix. The four elements of the specific example are shown in Fig. 3. As illustrated, element numbers 1, 2, 3 and 4 are allocated respectively to a lower left element, a lower right element, an upper left element and an upper right element. Node numbers 1, 2, 3, 4, 5, 6, 7, 8 and 9 are allocated respectively to a lower left node, a lower center node, a lower right node, a middle left node, a middle center node, a middle right node, an upper left node, an upper center node and an upper right node. Local node numbers 1, 2, 3 and 4 are allocated to respective local nodes in each element counterclockwise from a lower left local node as shown in Fig. 4. Internode numbers 1, 2, 3 and 4 are allocated to respective internodes in each element counterclockwise from a bottom internode as shown in Fig. 4.

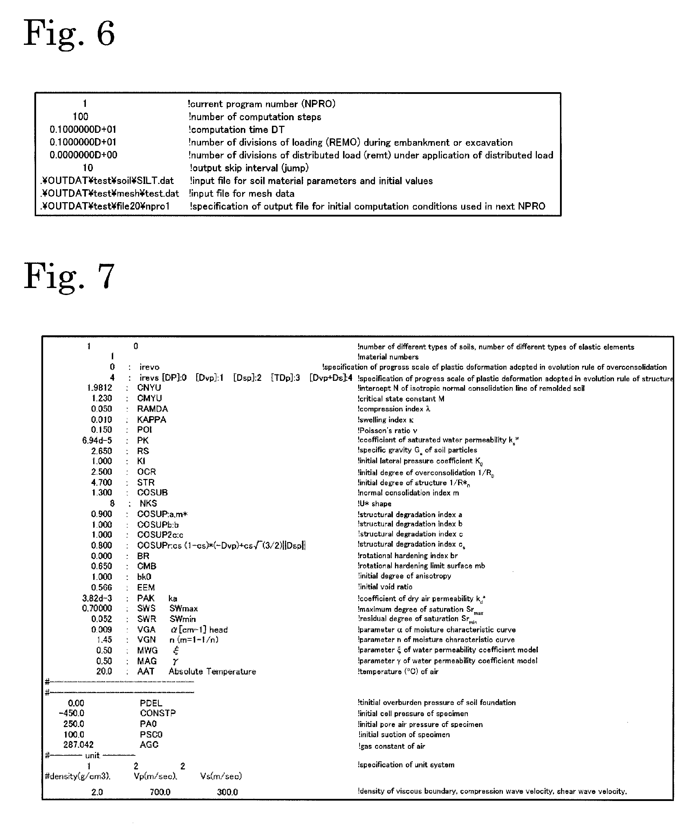

The soil-water-air coupled analyzing program 30 is triggered to first input settings of the analysis conditions (step S100). The settings of analysis conditions input herein may include, for example, selection of a technique of numerical time integration based on the difference method, an update frequency of time (number of computation steps), and selection of a computation condition, such as two-dimensional plane strain, axisymmetric or three-dimensional. Exemplary files as a specific example to input the settings of analysis conditions are shown in Figs. 5 and 6. The inputs in the file of Fig. 5 include: from the top to the bottom, the setting of "plane strain", "axisymmetric" or "three-dimensional" as the computation condition; selection of either "small deformation theory" or "finite deformation theory" as the deformation theory; the setting of either "Jaumann's rate of Cauchy stress" or "Green-Naghdi's rate" as the effective stress rate with objectivity used in the finite deformation theory; the setting of convergence or non-convergence; selection of either "original Cam-clay model with super-sub-loading yield surfaces and rotational hardening" or "modified Cam-clay model with super-sub-loading yield surfaces and rotational hardening" as the constitutive equation; the setting of either "element average of Gauss points" or "value of a specific Gauss point" as the state quantity output data; setting of either "Text" or "Binary" as the output data format; and the setting of consideration or non-consideration of self weight. The inputs in the file of Fig. 6 include from the top to the bottom, the settings of a current program number, the number of computation steps, a computation time DT per computation step (sec/step) (hereinafter may be expressed as $\Delta t$), the number of divisions of loading during embankment or excavation, the number of divisions of distributed load under application of distributed load, an input file for soil material parameters and initial values, an input file for mesh data, and specification of an output file for initial computation conditions used in execution of a next program.

The program subsequently inputs the settings of soils, such as clay, intermediate soil and sand with regard to the respective elements of the soil foundation (step S110). The settings of soil include material parameters and initial conditions. An exemplary file as a specific example of inputting the settings of soils is shown in Fig. 7. The inputs in the file of Fig. 7 include the number of different types of soils as the elasto-plastic bodies, the number of different types of elastic elements, material numbers, specification of a progress scale of plastic deformation adopted in an evolution rule of overconsolidation, specification of a progress scale of plastic deformation adopted in an evolution rule of structure, an intercept N of an isotropic normal consolidation line of remolded soil, a critical state constant M, a compression index $\lambda$, a swelling index $\kappa$, a Poisson's ratio $\nu$, a coefficient of saturated water permeability $k_s^w$, a specific gravity $G_s$ of soil particles, an initial lateral pressure coefficient $K_0$, an initial degree of overconsolidation $1/R_0$, an initial degree of structure $1/R^*_0$, a normal consolidation index m, a U* shape, a structural degradation index a, a structural degradation index b, a structural degradation index c, a structural degradation index $C_s$, a rotational hardening index $b_r$, a rotational hardening limit surface $m_b$, an initial degree of anisotropy, an initial void ratio, a coefficient of dry air permeability $k_d^a$, parameters $s^w_{max}$, $s^w_{min}$, $\alpha_v$, $m_v$ and $n_v$ of a moisture characteristic curve, parameters $\xi$ and $\gamma$ of a water permeability coefficient/ air permeability coefficient model, temperature of the air, an initial overburden pressure of the soil foundation, cell pressure (tensile: positive), air pressure, suction, gas constant of the air, settings of a unit system (time, force and length), density of a viscous boundary, a compression wave velocity and a shear wave velocity.

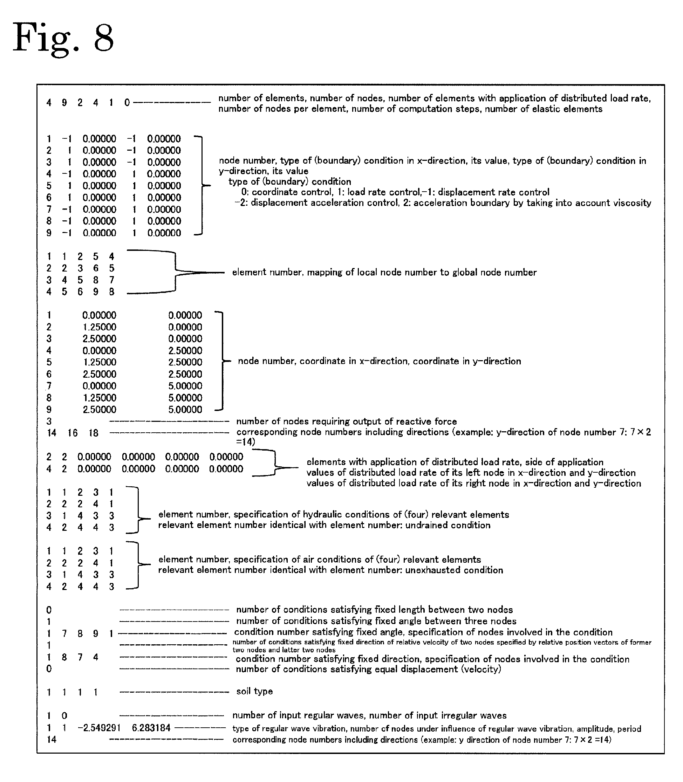

The program then inputs the settings of a solid soil model (step S120). The settings of the solid soil model include conditions with regard to elements of the soil model. Fig. 8 shows one exemplary file for inputting the settings of the solid soil model as the specific example shown in Figs. 3 and 4. In the file of Fig. 8, 1×6 data on the 1st line successively show the number of elements, the number of nodes and the number of elements with application of distributed load rate in the solid soil model, the number of nodes per element, the number of computation steps and the number of elastic elements. In the file, 9×5 data on the 2nd to the 10th lines successively show, from the left in each line, a node number, a type of boundary condition in an x-direction, a value of the boundary condition in the x-direction, a type of boundary condition in a y-direction and a value of the boundary condition in the y-direction. There are the following types of boundary condition herein: coordinate control (0), load rate control (1), displacement rate control (-1), displacement acceleration control (-2) and acceleration boundary by taking into account viscosity (2). In the file of Fig.

8, 4×5 data on the 11th to the 14th lines show an element number at the left most end in each line and global node numbers mapped to local node numbers with regard to the corresponding element (i.e., setting of a mapping of the local node number to the global node number). In the file, 9×3 data on the 15th to the 23rd lines successively show, from the left in each line, a node number, a coordinate in the x-direction and a coordinate in the y-direction. Data on the 24th line show the number of nodes requiring output of reactive force, and data on the 25th line show their node numbers requiring output of reactive force. There are two output directions of the reactive force, i.e., the x-direction and the y-direction with regard to each node number. For example, with regard to the node number 7, output of the reactive force in the x-direction is expressed as 7×2-1= 13, and output of the reactive force in the y-direction is expressed as 7×2= 14. In the file 2×6 data on the 26th and the 27th lines successively show, from the left in each line, an element number with application of distributed load rate, a side of application (internode of application), a value in the x-direction of the distributed load rate of its left node, a value in the y-direction of the distributed load rate of the left node, a value in the x-direction of the distributed load rate of its right node and a value in the y-direction of the distributed load rate of the right node. In the file, 4×6 data on the 28th to the 31st lines successively show, from the left in each line, an element number, an element number adjacent to the internode 1 of the corresponding element, an element number adjacent to the internode 2 of the corresponding element, an element number adjacent to the internode 3 of the corresponding element and an element number adjacent to the internode 4 of the corresponding element (i.e., setting of a mapping of the internode number to the global element number) . When a certain element has undrain to a specific adjacent element, the element number of the certain element itself should be input instead of the element number of the specific adjacent element. In the file, 4×6 data on the 32nd to the 35th lines successively show, from the left in each line, an element number, an element number adjacent to the internode 1 of the corresponding element, an element number adjacent to the internode 2 of the corresponding element, an element number adjacent to the internode 3 of the corresponding element and an element number adjacent to the internode 4 of the corresponding element (i.e., setting of a mapping of the internode number to the global element number). When a certain element has unexhaust to a specific adjacent element, the element number of the certain element itself should be input instead of the element number of the specific adjacent element. In the file, data on the 36th line shows the number of conditions satisfying a fixed length between two nodes. When there is any condition satisfying the fixed length between two nodes, its condition number and specification of nodes involved in the condition are shown on a next line inserted immediately below the data on the 36th line. Data on the 37th line shows the number of conditions satisfying a fixed angle between three nodes. Data on the 38th line successively show, from the left, a condition number satisfying the fixed angle between three nodes and specification of nodes involved in the condition. Data on the 39th line show the number of conditions satisfying a fixed direction of a relative velocity of two nodes specified by relative position vectors of former two nodes and latter two nodes. Data on the 40th line successively show, from the left, a condition number satisfying the fixed direction of the relative velocity of two nodes and specification of nodes involved in the condition. Data on the 41st line shows the number of conditions satisfying an equal displacement (velocity). When there is any condition satisfying the equal displacement (velocity), its condition number and specification relating to the condition are shown on a next line inserted immediately below the data on the 41st line. Data on the 42nd line shows a soil type. Data on the 43rd line successively show, from the left, the number of regular waves input into the model and the number of irregular waves input into the model. Data on the 44th line successively show, from the left, a type of regular wave vibration, the number of nodes under influence of the regular wave vibration input into the model and the amplitude and the period of the regular wave vibration. Data on the 45th line shows a node number under influence of the regular wave vibration. There are two directions of the regular wave vibration, i.e. , the x-direction and the y-direction with regard to each node number. For example, with regard to the node number 7, the regular wave vibration in the x-direction is expressed as 7x2-1= 13, and the regular wave vibration in the y-direction is expressed as 7x2= 14. When there is any irregular wave input into the model, specification of the irregular wave is shown on a next line inserted immediately below the data on the 45th line.

After the data input, the program performs computations using the input data (steps S130 to S270). The computation process of the first embodiment employ the Wilson's θ method for time integration, but another technique such as the Newmark's P method may be applied alternatively. The computation process of the first embodiment also employs the Tamura's model or the Christian's model of the finite element method of allocating the pore water pressure and the pore air pressure to the element center for spatial discretization, but another technique such as the Sandhu's model of the finite element method of allocating the pore water pressure to the node or the mesh-free method may be applied alternatively. The following describes computations according to the Wilson's θ method and the Tamura's model of the finite element method. The program first estimates a "total position vector", a "total increment vector concerning velocity" and a "total increment vector concerning acceleration" as well as various conditions (for example, effective stress, pore water pressure, pore air pressure, structure/ overconsolidation/ anisotropy and degree of saturation) according to Equations (1) to (5) given below (step S130). These predictive calculations are performed for the purpose of convergence in a shorter time period and are not essential. In the case of omission of such predictive calculations, the values eventually determined at a time t=t or the initial values at an initial time are set to the values at the time t = t+θΔt. Each set of vector quantities of all nodes as in the left side or in each term of the right side of Equation (1) is expressed by a column vector having components as shown in Equation (6). Each set of scalar quantities of all elements as in the left side or in each term of the right side of Equation (4) is expressed by a column vector having components as shown in Equation (7).

[Math. 23]

$$\left\{x^N\right\}\Big|_{t+\theta\Delta t} = \left\{x^N\right\}\Big|_t + \left\{v^N(\theta\Delta t)\right\}\Big|_t + \frac{1}{2}\left\{\dot{v}^N(\theta\Delta t)^2\right\}\Big|_t + \frac{1}{6}\left\{\ddot{v}^N(\theta\Delta t)^3\right\}\Big|_t \qquad (1)$$

$$\left\{v^N(\theta\Delta t)\right\}\Big|_{t+\theta\Delta t} = \left\{v^N(\theta\Delta t)\right\}\Big|_t + \left\{\dot{v}^N(\theta\Delta t)^2\right\}\Big|_t + \frac{1}{2}\left\{\ddot{v}^N(\theta\Delta t)^3\right\}\Big|_t \qquad (2)$$

$$\left\{\dot{v}^N(\theta\Delta t)^2\right\}\Big|_{t+\theta\Delta t} = \left\{\dot{v}^N(\theta\Delta t)^2\right\}\Big|_t + \left\{\ddot{v}^N(\theta\Delta t)^3\right\}\Big|_t \qquad (3)$$

$$\left\{p^w\right\}\Big|_{t+\theta\Delta t} = \left\{p^w\right\}\Big|_t + \left\{\dot{p}^w(\theta\Delta t)\right\}\Big|_t \qquad (4)$$

$$A\Big|_{t+\theta\Delta t} = A\Big|_t + \dot{A}\Big|_t(\theta\Delta t) \qquad (5)$$

$\{x^N\}|_t$, $\{x^N\}|_{t+\theta\Delta t}$ : column vector (total position vector) expressing set of position vectors of all nodes at time $t = t$ or at time $t = t + \theta\Delta t$

$\{v^N(\theta\Delta t)\}|_t$, $\{v^N(\theta\Delta t)\}|_{t+\theta\Delta t}$ : column vector (total increment vector concerning velocity) given as production of $\theta\Delta t$ and column vector $\{v^N\}$ (total velocity vector) expressing set of velocity vectors of all nodes at time $t = t$ or at time $t = t + \theta\Delta t$

$\{\dot{v}^N(\theta\Delta t)^2\}|_t$, $\{\dot{v}^N(\theta\Delta t)^2\}|_{t+\theta\Delta t}$ : column vector (total increment vector concerning acceleration) given as production of $(\theta\Delta t)^2$ and column vector $\{\dot{v}^N\}$ (total acceleration vector) expressing set of acceleration vectors of all nodes at time $t = t$ or at time $t = t + \theta\Delta t$

$\{\ddot{v}^N(\theta\Delta t)^3\}|_t$ : column vector (total increment vector concerning jerk) given as production of $(\theta\Delta t)^3$ and column vector $\{\ddot{v}^N\}$ (total jerk vector) expressing set of jerk vectors of all nodes at time $t = t$

$A$: state quantity, for example, effective stress tensor $T'$, rotational hardening variable tensor $\beta$ (tensor quantity), surface force vector $t$ (vector quantity), pore water pressure $p^w$, pore air pressure $p^a$, degree of saturation $s^w$, degree of structure $R^*$, degree of overconsolidation R (scalar quantity)

$A|_t$, $A|_{t+\theta\Delta t}$ : state quantity $A$ at time $t = t$ or at time $t = t + \theta\Delta t$

$A|_t$ : rate of state quantity $A$ at time $t = t$

[Math. 24]

$$\{a^N\} = \left\{ \begin{array}{c} a^1_{\ 1} \\ a^1_{\ 2} \\ a^1_{\ r} \\ \vdots \\ a^j_{\ 1} \\ a^j_{\ 2} \\ a^j_{\ r} \\ \vdots \\ a^{(NP)}_{\ 1} \\ a^{(NP)}_{\ 2} \\ a^{(NP)}_{\ r} \end{array} \right\} \qquad (6)$$

$a^j_{\ r}$ : r-direction component of $a^N$ at $j$-th node

$j$ : node number ($j$= 1, 2, ..., $NP$, $NP$: total number of nodes)

$r$: number of elements per node ($r$= 2 for 2D plane strain condition and axisymmetric condition, $r$= 3 for 3D condition)

[Math. 25]

$$\{\alpha\} = \begin{Bmatrix} \alpha^1 \\ \alpha^2 \\ \vdots \\ \alpha^i \\ \vdots \\ \alpha^{(NE-1)} \\ \alpha^{(NE)} \end{Bmatrix} \tag{7}$$

$\alpha^i$ : value of $\alpha$ at $i$-th element

$i$ : element number ($i$= 1, 2, ..., $NE$, $NE$: total number of elements)

The program subsequently computes a global L matrix (global volume change rate matrix) according to Equation (8) given below as a matrix for converting the displacement rate of each node to the time rate of volume change of the soil skeleton (step S140). The respective elements of the global L matrix are shown by Equation (9). The global L matrix is a matrix of NE rows and (NP×r) columns (r: the number of components per node). According to the mapping of the global node number to the local node number specified in setting the solid soil model, a value "0" is set to any column component having an m-th node number different from the global node number in an element L matrix for an element i shown in Equation (9). A Bv matrix constituting the element L matrix is expressed by Equation (10) for the two-dimensional plane strain condition, by Equation (11) for the axisymmetric condition and by Equation (12) for the three-dimensional condition.

[Math. 26]

$$L = \begin{bmatrix} L^{e1} \\ L^{e2} \\ \vdots \\ L^{ei} \\ \vdots \\ L^{e(NE-1)} \\ L^{e(NE)} \end{bmatrix} : \text{global L matrix (global volume change rate matrix)} \tag{8}$$

$$L^{ei} = \begin{bmatrix} L^{i1}_{1} & L^{i1}_{2} & L^{i1}_{r} & \cdots & L^{im}_{1} & L^{im}_{2} & L^{im}_{r} & \cdots & L^{ip}_{1} & L^{ip}_{2} & L^{ip}_{r} \end{bmatrix} = \int_{ve^i} [B_v] dv \tag{9}$$

: element L matrix for element $i$ (element volume change rate matrix)

$m$ : local node number in element ($m$= 1, 2, ..., $p$)

$p$ : number of nodes per element ($p$= 4 for 2D plane strain condition and axisymmetric condition, $p$= 8 for 3D condition)

$ve^i$ : volume area occupied by element $i$

[Math. 27]

$$[B_v] = \begin{bmatrix} N^1_{,1} & N^1_{,2} & \cdots & N^m_{,1} & N^m_{,2} & \cdots & N^p_{,1} & N^p_{,2} \end{bmatrix} \qquad (p=4) \tag{10}$$

$N^m$ : shape function regarding $m$-th node of element $i$

$$[B_v] = \begin{bmatrix} N^1_{,1} + \dfrac{N^1}{x^G_1} & N^1_{,2} & \cdots & N^m_{,1} + \dfrac{N^m}{x^G_1} & N^m_{,2} & \cdots & N^p_{,1} + \dfrac{N^p}{x^G_1} & N^p_{,2} \end{bmatrix} \qquad (p=4) \tag{11}$$

$x^{G}_1$ : distance in radial direction from rotational axis to Gauss point

$$[B_v] = \begin{bmatrix} N^1_{,1} & N^1_{,2} & N^1_{,3} & \cdots & N^m_{,1} & N^m_{,2} & N^m_{,3} & \cdots & N^p_{,1} & N^p_{,2} & N^p_{,3} \end{bmatrix} \qquad (p{=}8) \qquad (12)$$

The integrals like Equation (9) are performed according to the Gauss numerical integration method after conversion of a target analysis area in a global coordinate system into a local coordinate system as shown in Equation (13). Equation (13) herein shows volume integration for the three-dimensional condition. The integration is performed in the light of a unit thickness in a direction perpendicular to the target analysis plane for the two-dimensional plane strain condition, while being performed in the light of the rotational symmetry about the axis for the axisymmetric condition.
[Math. 28]

$$\iiint f(x, y, z)\mathrm{d}x\mathrm{d}y\mathrm{d}z$$
$$= \sum_{i=1}^{NG}\sum_{j=1}^{NG}\sum_{k=1}^{NG} w_i w_j w_k f(x(\xi_i,\eta_j,\zeta_k), y(\xi_i,\eta_j,\zeta_k), z(\xi_i,\eta_j,\zeta_k))J'(\xi_i,\eta_j,\zeta_k) \qquad (13)$$

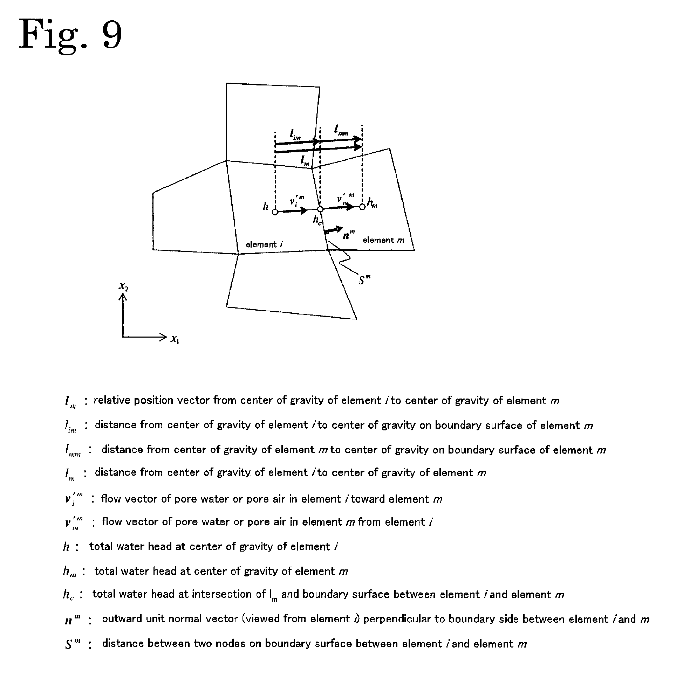

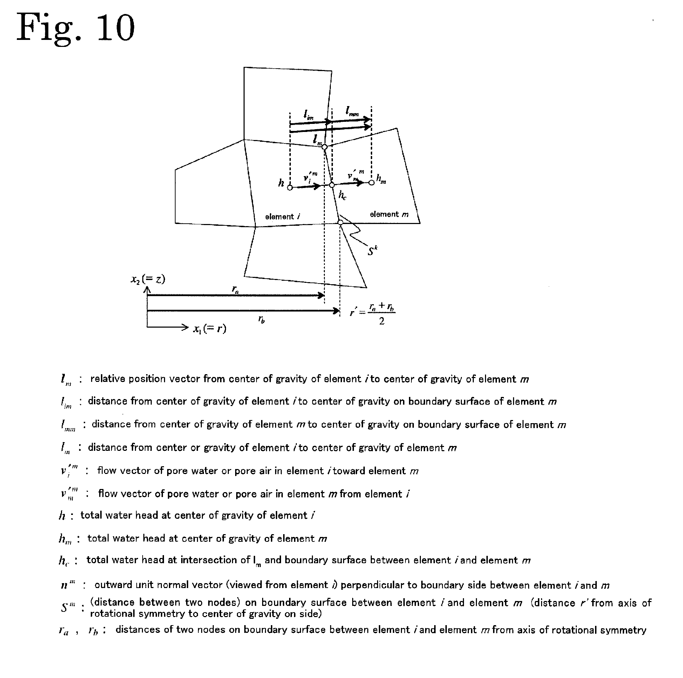

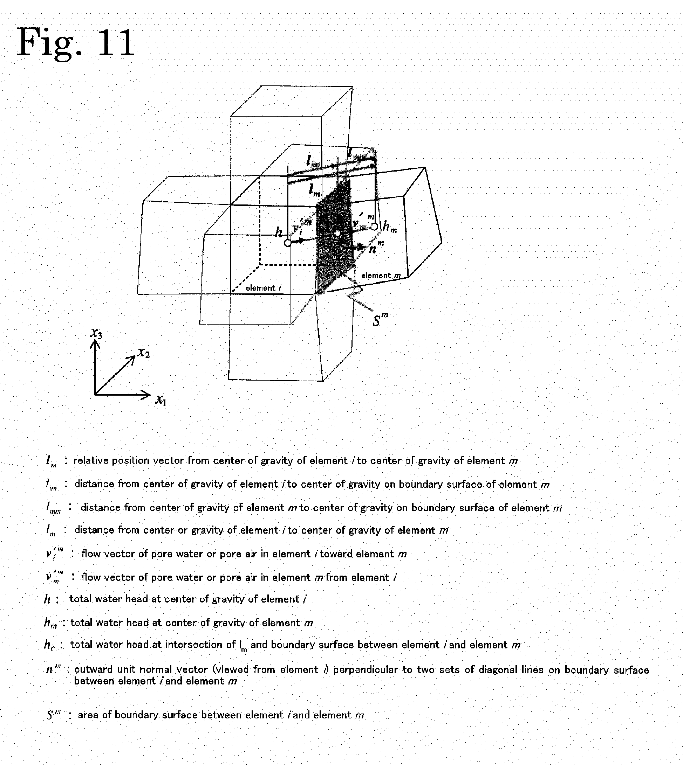

$f = f(x,y,z)$ : integrand in integral area in o-xyz coordinate system (for 3D condition)
$J'$ : Jacobian determinant for conversion from o-xyz coordinate system into o-$\xi\eta\zeta$ local coordinate system having domain of [-1,1]
$NG$ : number of Gauss points
$\xi_i(i = 1,\cdots,NG)$, $\eta_j(j = 1,\cdots,NG)$, $\zeta_k(k{=}1,\cdots,NG)$ : coordinates of Gauss points on $\xi,\eta,\zeta$ axes
$w_i(i = 1,...,NG)$, $w_j(j = 1,\cdots, NG)$, $w_k(k = 1,\cdots,NG)$ : weights of respective Gauss points
The program subsequently computes a global $H_W$ matrix (global water permeability matrix) representing water permeability in the soil according to Equation (14) and a global $H_A$ matrix (global air permeability matrix) representing air permeability in the soil according to Equation (15) (step S150). The hydraulic boundary condition, such as drain or undrain and the air boundary condition, such as exhaust or unexhaust are respectively obtained by changing the component of an element $H_W$ matrix shown in Equation (16) and by changing the component of an element $H_A$ matrix shown in Equation (17). For example, in the case of the undrained condition, a value "0" is set to a component in an element $H_W$ matrix corresponding to an internode under this condition. In the case of the unexhausted condition, a value "0" is set to a component in an element $H_A$ matrix corresponding to an internode under this condition. Both the global $H_W$ matrix and global $H_A$ matrix are matrices of NE rows and NE columns. According to the mapping of the internode number of the element to the global element number specified in setting the solid soil model, a value "0" is set to any component in the global $H_W$ matrix of Equation (14) and to any component in the global $H_A$ matrix of Equation (15) having an m-th internode number in an element $H_W$ matrix and in an element $H_A$ matrix for an element i different from the global element number. The last component in the element $H_W$ matrix for the element i of Equation (16) or the last component in the element $H_A$ matrix for the element i of Equation (17) is substituted into an i-th column component of the element i (i.e., i-th row). The respective components on the right side of Equation (16) are expressed by Equation (18). The respective components on the right side of Equation (17) are expressed by Equation (19) . With regard to the meanings of the respective symbols, refer to Fig. 9 for the two-dimensional plane strain condition, refer to Fig. 10 for the axisymmetric condition and refer to Fig. 11 for the three-dimensional condition.
[Math. 29]

$$H_W = \begin{bmatrix} H_W{}^{e1} \\ H_W{}^{e2} \\ \vdots \\ H_W{}^{ei} \\ \vdots \\ H_W{}^{e(NE-1)} \\ H_W{}^{e(NE)} \end{bmatrix} \qquad (14)$$

: global $H_W$ matrix (global water permeability matrix)

EP 2 866 019 A1

$$H_A = \begin{bmatrix} H_A^{e1} \\ H_A^{e2} \\ \vdots \\ H_A^{ei} \\ \vdots \\ H_A^{e(NE-1)} \\ H_A^{e(NE)} \end{bmatrix}$$

(15)

: global $H_A$ matrix (global air permeability matrix)

$$H_W^{ei} = \begin{bmatrix} \alpha_W^{ei}{}_1 & \cdots & \alpha_W^{ei}{}_m & \cdots & \alpha_W^{ei}{}_s & -\sum_{m=1}^{s} \alpha_W^{ei}{}_m \end{bmatrix}$$

(16)

: element $H_W$ matrix for element $i$ (element water permeability matrix)

$$H_A^{ei} = \begin{bmatrix} \alpha_A^{ei}{}_1 & \cdots & \alpha_A^{ei}{}_m & \cdots & \alpha_A^{ei}{}_s & -\sum_{m=1}^{s} \alpha_A^{ei}{}_m \end{bmatrix}$$

(17)

: element $H_A$ matrix for element $i$ (element air permeability matrix)

$m$ : element plane number in element (for 3D condition) or element side number in element (for 2D plane strain condition or axisymmetric condition)( $m$ =1,2, $\cdots$,$s$ )
s : number of planes per element (s= 6 for 3D condition) or number of sides per element (s= 4 for 2D plane strain condition and axisymmetric condition)
[Math. 30]

$$\alpha_W^{ei}{}_m = \frac{1}{\gamma_w} \cdot \frac{\dfrac{k^w{}_m \cdot k^w{}_i}{l_{mm} \cdot l_{im}}}{\dfrac{k^w{}_m}{l_{mm}} + \dfrac{k^w{}_i}{l_{im}}} \cdot \frac{l_m}{l_m} \cdot n^m S^m \qquad \text{(no summation)}$$

(18)

$$\alpha_A^{ei}{}_m = \frac{1}{\gamma_w} \cdot \frac{\dfrac{\rho^a{}_m \cdot k^a{}_m \cdot k^a{}_i}{l_{mm} \cdot l_{im}}}{\dfrac{\rho^a{}_m \cdot k^a{}_m}{l_{mm}} + \dfrac{\rho^a{}_i \cdot k^a{}_i}{l_{im}}} \cdot \frac{l_m}{l_m} \cdot n^m S^m \qquad \text{(no summation)}$$

(19)

$m$ : element number of element adjacent to element i
$k^w{}_m$: coefficient of water permeability of element $m$ at time $t = t + \theta\Delta t$
$k^w i$ : coefficient of water permeability of element $i$ at time $t = t + \theta\Delta t$
$k^a{}_m$ : coefficient of air permeability of element $m$ at time $t = r + \theta\Delta t$
$k^a{}_i$ : coefficient of air permeability of element i at time $t = t + \theta\Delta t$
$\rho^a{}_m$ : density of air of element $m$ at time $t = t + \theta\Delta t$
$\rho^a{}_i$ : density of air of element $i$ at time $t = t + \theta\Delta t$
$\gamma_w$ : unit volume weight of water at 4°C (standard unit volume weight)
$n^m$ : <for 2D plane strain condition and axisymmetric condition>
outward (viewed from element $i$) unit normal vector perpendicular to boundary side between element $i$ and element $m$
<for 3D condition>
outward (viewed from element $i$) unit normal vector perpendicular to two diagonal lines on
boundary surface between element $i$ and element $m$

22

$S^m$ : <for 2D plane strain condition>

distance between two nodes on boundary surface between element i and element $m$

<for axisymmetric condition>

(distance between two nodes) on boundary surface between element $i$ and element $m$ ×

(distance $r'$ from axis of rotational symmetry to center of gravity on side)

<for 3D condition>

area of boundary surface between element $i$ and element $m$

$l_{im}$ : distance from center of gravity of element $i$ to center of gravity on boundary surface of element $m$

$l_{mm}$ : distance from center of gravity of element $m$ to center of gravity on boundary surface of element $m$

$l_m$ : distance from center of gravity of element $i$ to center of gravity of element $m$

$\vec{l}_m$ : relative position vector from center of gravity of element i to center of gravity of element $m$

The program subsequently computes a global $L_{BW}$ matrix (global water flow rate conversion matrix) according to Equation (20) and a global $L_{BA}$ matrix (global air flow rate conversion matrix) according to Equation (25) (step S160). The respective elements of the global $L_{BW}$ matrix are expressed by Equation (21), and the respective elements of the global $L_{BA}$ matrix are expressed by Equation (26). Both the global $L_{BW}$ matrix and the global $L_{BA}$ matrix are matrices of NE rows and (NPxr) columns. According to the mapping of the global node number to the local number specified in setting the solid soil mode, a value "0" is set to any column component having an n-th node number different from the global node number in an element $L_{BW}$ matrix for an element i for Equation (21) or in an element $L_{BA}$ matrix for the element i of Equation (26). An $L_B$ matrix constituting the element $L_{BW}$ matrix or the element $L_{BA}$ matrix is expressed by Equation (22). The respective components of the element $L_{BW}$ matrix are expressed by Equation (23), where a value to be added differs whether the hydraulic condition between adjacent elements is drained condition or undrained condition. A value $\beta_j^{wi}$ used for the drained condition is given by Equation (24). The respective components of the element $L_{BA}$ matrix are expressed by Equation (27), where a value to be added differs whether the air condition between adjacent elements is exhausted condition or unexhausted condition. A value $B_j^{ai}$ used for the exhausted condition is given by Equation (28). With regard to the meanings of the respective symbols, refer to Fig. 9 for the two-dimensional plane strain condition, refer to Fig. 10 for the axisymmetric condition and refer to Fig. 11 for the three-dimensional condition.

[Math. 31]

$$L_{BW} = \begin{bmatrix} L_{BW}^{\;e1} \\ L_{BW}^{\;e2} \\ \vdots \\ L_{BW}^{\;ei} \\ \vdots \\ L_{BW}^{\;e(NE-1)} \\ L_{BW}^{\;e(NE)} \end{bmatrix} : \text{global } L_{BW} \text{ matrix (global water flow rate conversion matrix)} \quad (20)$$

$$L_{BW}^{\;ei} = \begin{bmatrix} L_{BW}^{\;i1}{}_1 & L_{BW}^{\;i1}{}_2 & L_{BW}^{\;i1}{}_r & \cdots & L_{BW}^{\;in}{}_1 & L_{BW}^{\;in}{}_2 & L_{BW}^{\;in}{}_r & \cdots & L_{BW}^{\;ip}{}_1 & L_{BW}^{\;ip}{}_2 & L_{BW}^{\;ip}{}_r \end{bmatrix} \quad (21)$$

: element $L_{BW}$ matrix for element $i$ (element water flow rate conversion matrix)

$n$ : local node number in element ($n$ = 1,2,···,$p$)

$p$ : number of nodes per element ($p$= 4 for 2D plane strain condition and axisymmetric condition, $p$= 8 for 3D condition)

$r$ : number of components per node ($r$= 2 for 2D plane strain condition and axisymmetric condition, $r$= 3 for 3D condition)

$$L_B^{\;l} = \int_{S^l} \{n^l\}^T [N] dS^l \quad (22)$$

$$L_{BW}^{\;in}{}_r = \frac{k^w_i}{\gamma_w} \left\{ \sum_{j=1}^{s}{}' \left( \beta_j^{\;wi} L_B^{\;j} \right) - \rho_0^{\;w} \sum_{k=1}^{s}{}'' \left( L_B^{\;k} \right) \right\} \quad (23)$$

$\rho_0^w$ : density of water at 4°C (standard density)

$s$ : number of planes per element (s= 6 for 3D condition) or number of sides per element (s= 4 for 2D plane strain condition and axisymmetric condition)

*l* : element plane number in element (for 3D condition) or element side number in element (for 2D plane strain condition or axisymmetric condition) (*l*=1,2,···,*s*)

*j* : element plane number in element (for 3D condition) or element side number in element (for 2D plane strain condition or axisymmetric condition) (*j*=1,2,···,*s*)

*k:* element plane number in element (for 3D condition) or element side number in element (for 2D plane strain condition or axisymmetric condition) (*k*=1,2,···,*s*)

$\sum_{j=1}^{s}{}'$ : addition operation based on mapping of local node number on *j* plane (or side) to global node number only when hydraulic condition relative to adjacent element on *j* plane (or side) is drained condition

$\sum_{k=1}^{s}{}''$ : addition operation based on mapping of local node number on *k* plane (or side) to global node number only when hydraulic condition relative to adjacent element on *k* plane (or side) is undrained condition

$$\beta_j^{wi} = \frac{\rho_0^{w} \cdot \dfrac{k^w_m - k^w_i}{l_{im}}}{\dfrac{k^w_m}{l_{mm}} + \dfrac{k^w_i}{l_{im}}} \qquad \text{(no summation)} \tag{24}$$

*m* : element number of element adjacent to element *i*

[Math. 32]

$$L_{BA} = \begin{bmatrix} L_{BA}^{e1} \\ L_{BA}^{e2} \\ \vdots \\ L_{BA}^{ei} \\ \vdots \\ L_{BA}^{e(NE-1)} \\ L_{BA}^{e(NE)} \end{bmatrix} : \text{global } L_{BA} \text{ matrix (global air flow rate conversion matrix)} \tag{25}$$

$$L_{BA}^{ei} = \begin{bmatrix} L_{BA}^{i1}{}_1 & L_{BA}^{iA1}{}_2 & L_{BA}^{iA1}{}_r & \cdots & L_{BA}^{in}{}_1 & L_{BA}^{in}{}_2 & L_{BA}^{in}{}_r & \cdots & L_{BA}^{iP}{}_1 & L_{BA}^{iP}{}_2 & L_{BA}^{iP}{}_r \end{bmatrix} \tag{26}$$

: element $L_{BA}$ matrix for element *i* (element air flow rate conversion matrix)

*n* : local node number in element ( *n*=1,2,···,*p* )

*p* : number of nodes per element (p= 4 for 2D plane strain condition and axisymmetric condition, *p*= 8 for 3D condition)

*r* : number of components per node (*r*= 2 for 2D plane strain condition and axisymmetric condition, *r*= 3 for 3D condition)

$$L_{BA}^{in}{}_r = \frac{k^a_i}{\gamma_w}\left\{ \sum_{j=1}^{s}{}'\left(\beta_j^{ai} L_B^{j}\right) - \rho^a_i \sum_{k=1}^{s}{}''\left(L_B^{k}\right) \right\} \tag{27}$$

*s* : number of planes per element (s= 6 for 3D condition) or number of sides per element (s= 4 for 2D plane strain condition and axisymmetric condition)

*j* : element plane number in element (for 3D condition) or element side number in element (for 2D plane strain condition or axisymmetric condition) ( *j*=1,2,···,*s* )

*k:* element plane number in element (for 3D condition) or element side number in element (for 2D plane strain condition or axisymmetric condition) (*k*=1,2,···,*s*)

$\sum_{j=1}^{s}{}'$ : addition operation based on mapping of local node number on *j* plane (or side) to global node number only when air condition relative to adjacent element on *j* plane (or side) is exhausted condition $\sum_{k=1}^{s}{}''$ : addition operation based on mapping of local node number on *k* plane (or side) to global node number only when air condition relative to adjacent element on *k* plane (or side) is unexhausted condition

$$\beta_j{}^{ai} = \frac{\dfrac{\rho^a{}_m{}^2 k^a{}_m - \rho^a{}_i{}^2 k^a{}_i}{l_{im}}}{\dfrac{\rho^a{}_m \cdot k^a{}_m}{l_{mm}} + \dfrac{\rho^a{}_i \cdot k^a{}_i}{l_{im}}} \qquad \text{(no summation)} \qquad (28)$$

*m* : element number of element adjacent to element i

The program subsequently calculates a total load increment vector, a total flow increment vector for pore water and a total flow increment vector for pore air at the time t=t+θΔt respectively according to Equation (29), Equation (44) and Equation (46) given below (step S170). A first term on the right side of Equation (29) is related to a nominal stress rate vector and is expressed by Equation (30). A second term on the right side of Equation (29) is related to the Green-Naghdi's rate and is expressed by Equation (31). An N matrix on the right side of Equation (30) and a B matrix on the right side of Equation (31) are expressed by Equations (32) and (33) for the two-dimensional plane strain condition, by Equations (34) and (35) for the axisymmetric condition and by Equations (36) and (37) for the three-dimensional condition. The term of the nominal stress rate vector in Equation (30) is rewritten as Equation (38) and is a sum of a surface traction rate vector externally given through a boundary defined by a first term on the right side and an increment of a surface traction vector due to a change in geometric configuration of the boundary defined by a second term on the right side. The surface traction rate vector at the time t=t+θΔt of the first term on the right side is given by conversion of a surface traction rate vector set as a boundary condition at a time t=t+Δt according to Equation (39) in conformity with the Wilson's θ method. An n matrix included in the second term on the right side of Equation (38) and related to an outward unit normal vector on a mechanical boundary of stress or stress rate is given by Equation (40) for the two-dimensional plane strain condition or the axisymmetric condition and by Equation (41) for the three-dimensional condition. A column vector including an effective stress component in a first term on the right side of Equation (31) is given by Equation (42) for the two-dimensional plane strain condition or the axisymmetric condition and by Equation (43) for the three-dimensional condition.

[Math. 33]

$$\left\{\dot{f}(\theta\Delta t)\right\}\Big|_{t+\theta\Delta t} = \sum_{j=1}^{NS}{}'\left\{\dot{f}^j(\theta\Delta t)\right\}\Big|_{t+\theta\Delta t} + \sum_{i=1}^{NE}{}''\left\{\dot{f}^{ei}(\theta\Delta t)\right\}\Big|_{t+\theta\Delta t} \quad : \text{ total load increment vector at time} \tag{29}$$

$$t = t + \theta\Delta t$$

$\sum_{j=1}^{NS}{}'$ : operation of adding value of node on *j*-th side (mechanical boundary of stress or stress rate) to value of corresponding global node, based on mapping of local node number to global node number *NS* : number of planes on mechanical boundary (for 3D condition) or number of sides on mechanical boundary (for 2D plane strain condition or axisymmetric condition) $\sum_{i=1}^{NE}{}''$ : operation of adding value of each node in *i*-th element to value of corresponding global node, based on mapping of local node number to global node number

$$\left\{\dot{f}^j(\theta\Delta t)\right\}\Big|_{t+\theta\Delta t} = \int_a [N]^T \left\{\dot{s}_t\right\}\Big|_{t+\theta\Delta t} da \times (\theta\Delta t) \tag{30}$$

$$\left\{\dot{f}^{ei}(\theta\Delta t)\right\}\Big|_{t+\theta\Delta t} = -\int_v [B]^T \left\{T'_\Omega\right\}\Big|_{t+\theta\Delta t} dv \times (\theta\Delta t) \tag{31}$$

[Math. 34]

$$[N] = \begin{bmatrix} N^1 & 0 & \cdots & N^m & 0 & \cdots & N^p & 0 \\ 0 & N^1 & \cdots & 0 & N^m & \cdots & 0 & N^p \end{bmatrix} \quad (p=4) \tag{32}$$

$$[B] = \begin{bmatrix} N^1_{,1} & 0 & \cdots & N^m_{,1} & 0 & \cdots & N^p_{,1} & 0 \\ 0 & N^1_{,2} & \cdots & 0 & N^m_{,2} & \cdots & 0 & N^p_{,2} \\ N^1_{,2} & N^1_{,1} & \cdots & N^m_{,2} & N^m_{,1} & \cdots & N^p_{,2} & N^p_{,1} \end{bmatrix} \tag{33}$$

[Math. 35]

$$[N] = \begin{bmatrix} N^1 & 0 & \cdots & N^m & 0 & \cdots & N^p & 0 \\ 0 & N^1 & \cdots & 0 & N^m & \cdots & 0 & N^p \end{bmatrix} \quad (p=4) \tag{34}$$

$$[B] = \begin{bmatrix} N^1_{,1} & 0 & \cdots & N^m_{,1} & 0 & \cdots & N^p_{,1} & 0 \\ 0 & N^1_{,2} & \cdots & 0 & N^m_{,2} & \cdots & 0 & N^p_{,2} \\ N^1_{,2} & N^1_{,1} & \cdots & N^m_{,2} & N^m_{,1} & \cdots & N^p_{,2} & N^p_{,1} \\ \dfrac{N^1}{x^G{}_1} & 0 & \cdots & \dfrac{N^m}{x^G{}_1} & 0 & \cdots & \dfrac{N^p}{x^G{}_1} & 0 \end{bmatrix} \tag{35}$$

[Math. 36]

$$[N] = \begin{bmatrix} N^1 & 0 & 0 & \cdots & N^m & 0 & 0 & \cdots & N^p & 0 & 0 \\ 0 & N^1 & 0 & \cdots & 0 & N^m & 0 & \cdots & 0 & N^p & 0 \\ 0 & 0 & N^1 & \cdots & 0 & 0 & N^m & \cdots & 0 & 0 & N^p \end{bmatrix} \quad (p=8) \tag{36}$$

$$[B] = \begin{bmatrix} N^1_{,1} & 0 & 0 & \cdots & N^m_{,1} & 0 & 0 & \cdots & N^p_{,1} & 0 & 0 \\ 0 & N^1_{,2} & 0 & \cdots & 0 & N^m_{,2} & 0 & \cdots & 0 & N^p_{,2} & 0 \\ 0 & 0 & N^1_{,3} & \cdots & 0 & 0 & N^m_{,3} & \cdots & 0 & 0 & N^p_{,3} \\ N^1_{,2} & N^1_{,1} & 0 & \cdots & N^m_{,2} & N^m_{,1} & 0 & \cdots & N^p_{,2} & N^p_{,1} & 0 \\ 0 & N^1_{,3} & N^1_{,2} & \cdots & 0 & N^m_{,3} & N^m_{,2} & \cdots & 0 & N^p_{,3} & N^p_{,2} \\ N^1_{,3} & 0 & N^1_{,1} & \cdots & N^m_{,3} & 0 & N^m_{,1} & \cdots & N^p_{,3} & 0 & N^p_{,1} \end{bmatrix} \tag{37}$$

[Math. 37]

$$\{\dot{s}_\mathrm{t}\}\big|_{t+\theta\Delta t} \times (\theta\Delta t) = \{\dot{t}\}\big|_{t+\theta\Delta t} \times (\theta\Delta t) + \left([B_v] - [n]\big|_{t+\theta\Delta t}[B]\right)\{v^N(\theta\Delta t)\}\big|_{t+\theta\Delta t}\{t\}\big|_{t+\theta\Delta t} \tag{38}$$

$$\{\dot{t}\}\big|_{t+\theta\Delta t} = \{\dot{t}\}\big|_t + \theta\left(\{\dot{t}\}\big|_{t+\Delta t} - \{\dot{t}\}\big|_t\right) \tag{39}$$

$\{\dot{s}_\mathrm{t}\}\big|_{t+\theta\Delta t}$ : nominal stress rate vector of $i$-th side specified as mechanical boundary at time $t = t + \theta\Delta t$ $\{t\}\big|_{t+\theta\Delta t}$ , $\{\dot{t}\}\big|_{t+\theta\Delta t}$ : surface traction vector acting on $i$-th side specified as mechanical boundary at time $t = t + \theta\Delta t$ and its rate

$[n]_{t+\theta\Delta t}$ : matrix of respective components (direction cosine, $n_r$: $r$-direction component) of outward unit normal vector at $i$-th side as mechanical boundary at time $t = t + \theta\Delta t$

[Math. 38]

$$\left[\boldsymbol{n}\right]_{t+\theta\Delta t} = \begin{bmatrix} n_1^{\,2} & n_2^{\,2} & n_1 n_2 \end{bmatrix} \tag{40}$$

$$\left[\boldsymbol{n}\right]_{t+\theta\Delta t} = \begin{bmatrix} n_1^{\,2} & n_2^{\,2} & n_3^{\,2} & n_1 n_2 & n_2 n_3 & n_3 n_1 \end{bmatrix} \tag{41}$$

[Math. 39]

$$\left\{T'_\Omega\right\}_{t+\theta\Delta t} = \Omega_c \left\{ \begin{array}{c} -2T'_{12} \\ 2T'_{12} \\ T'_{11} - T'_{22} \end{array} \right\} \tag{42}$$

$T'_{ij} : i,j$ components ($i$= 1, 2, 3, $j$= 1, 2, 3) of effective stress tensor $\boldsymbol{T'}$ at time $t = t + \theta\Delta t$

$\Omega_c$ : (2,1) component of material spin tensor $\Omega = \dot{\boldsymbol{R}}\boldsymbol{R}^T$ ( $\boldsymbol{R}$ : rotation tensor) at time $t = t + \theta\Delta t$ for axisymmetric condition: value of 4th component= 0

[Math. 40]

$$\left\{T'_\Omega\right\}_{t+\theta\Delta t} = \left\{ \begin{array}{c} 2(\Omega_2 T'_{13} - \Omega_3 T'_{12}) \\ 2(\Omega_3 T'_{12} - \Omega_1 T'_{23}) \\ 2(\Omega_1 T'_{23} - \Omega_2 T'_{13}) \\ -\Omega_1 T'_{13} + \Omega_2 T'_{23} + \Omega_3(T'_{11} - T'_{22}) \\ \Omega_1(T'_{22} - T'_{33}) - \Omega_2 T'_{12} + \Omega_3 T'_{13} \\ \Omega_1 T'_{12} + \Omega_2(T'_{33} - T'_{11}) - \Omega_3 T'_{23} \end{array} \right\} \tag{43}$$

$\Omega_1, \Omega_2\, \Omega_3$ : (3,2) component, (1,3) component and (2,1) component of material spin tensor $\Omega$ at time $t = t + \theta\Delta t$

The total flow increment vector for pore water at the time t = t + θΔt is expressed by Equation (44) and is obtained by multiplying a total flow rate vector expressed by Equation (45) by θΔt. The total flow increment vector for pore air at the time t=t+θΔt is expressed by Equation (46) and is obtained by multiplying a total flow rate vector for pore air expressed by Equation (47) by θΔt.

[Math. 41]

$$\left\{\dot{f}_w(\theta\Delta t)\right\}_{t+\theta\Delta t} = \left\{\dot{f}_w\right\}_{t+\theta\Delta t} \times (\theta\Delta t) \; : \text{total flow increment vector for pore water at time } t = t + \theta\Delta t \tag{44}$$

$$\left\{\dot{f}_w\right\}_{t+\theta\Delta t} = \left( \begin{array}{c} -\sum_{m=1}^{s} \gamma_w \alpha_W{}^{e1}{}_m \left(z_{cm}{}^{1} - z_c{}^{1}\right) \\ -\sum_{m=1}^{s} \gamma_w \alpha_W{}^{e2}{}_m \left(z_{cm}{}^{2} - z_c{}^{2}\right) \\ \vdots \\ -\sum_{m=1}^{s} \gamma_w \alpha_W{}^{ei}{}_m \left(z_{cm}{}^{i} - z_c{}^{i}\right) \\ \vdots \\ -\sum_{m=1}^{s} \gamma_w \alpha_W{}^{e(NE-1)}{}_m \left(z_{cm}{}^{(NE-1)} - z_c{}^{(NE-1)}\right) \\ -\sum_{m=1}^{s} \gamma_w \alpha_W{}^{e(NE)}{}_m \left(z_{cm}{}^{NE} - z_c{}^{NE}\right) \end{array} \right) \tag{45}$$

: total flow rate vector for pore water at time $t = t + \theta\Delta t$

$z_c{}^i$ : vertical coordinate of center of gravity of element *i* at time *t* = *t* + θΔ*t*

$z_{cm}{}^i$ : vertical coordinate of center of gravity of element *m* adjacent to element *i* at time *t* = *t* + θΔ*t*

*s* : number of planes per element *(s*= 6 for 3D condition) or number of sides per element *(s*= 4 for 2D plane strain condition and axisymmetric condition)

[Math. 42]

$$\left\{\dot{f}_{\mathrm{a}}(\theta\Delta t)\right\}\Big|_{t+\theta\Delta t} = \left\{\dot{f}_{\mathrm{a}}\right\}\Big|_{t+\theta\Delta t} \times (\theta\Delta t) \ : \text{total flow increment vector for pore air at time } \ t = t + \theta\Delta t \tag{46}$$

$$\left\{\dot{f}_{\mathrm{a}}\right\}\Big|_{t+\theta\Delta t} = \begin{pmatrix} -\sum_{m=1}^{s}\dfrac{k^{\mathrm{a}}{}_{1}}{\rho_0{}^{\mathrm{w}}}\left(\beta_m{}^{\mathrm{a}1}+\rho^{\mathrm{a}}{}_1\right)n^m{}_{1z}S^m \\[4pt] -\sum_{m=1}^{s}\dfrac{k^{\mathrm{a}}{}_{2}}{\rho_0{}^{\mathrm{w}}}\left(\beta_m{}^{\mathrm{a}2}+\rho^{\mathrm{a}}{}_2\right)n^m{}_{2z}S^m \\ \vdots \\ -\sum_{m=1}^{s}\dfrac{k^{\mathrm{a}}{}_{i}}{\rho_0{}^{\mathrm{w}}}\left(\beta_m{}^{\mathrm{a}i}+\rho^{\mathrm{a}}{}_i\right)n^m{}_{iz}S^m \\ \vdots \\ -\sum_{m=1}^{s}\dfrac{k^{\mathrm{a}}{}_{(NE-1)}}{\rho_0{}^{\mathrm{w}}}\left(\beta_m{}^{\mathrm{a}(NE-1)}+\rho^{\mathrm{a}}{}_{(NE-1)}\right)n^m{}_{(NE-1)z}S^m \\ -\sum_{m=1}^{s}\dfrac{k^{\mathrm{a}}{}_{NE}}{\rho_0{}^{\mathrm{w}}}\left(\beta_m{}^{\mathrm{a}(NE)}+\rho^{\mathrm{a}}{}_{NE}\right)n^m{}_{(NE)z}S^m \end{pmatrix} \tag{47}$$

: total flow rate vector for pore air at time $\ t = t + \theta\Delta t$

***b*** : body force vector per unit mass

*s* : number of planes per element (s= 6 for 3D condition) or number of sides per element *(s*= 4 for 2D plane strain condition and axisymmetric condition)

*m* : element plane number in element (for 3D condition) or element side number in element (for 2D plane strain condition or axisymmetric condition) ( *m* =1,2,⋯,*s* )

$n^m{}_{iz}$ : <for 2D plane strain condition and axisymmetric condition>

upward vertical component of outward unit normal vector (viewed from element *i*) perpendicular to boundary side between element *i* and element *m*

<for 3D condition>

upward vertical component of outward unit normal vector (viewed from element *i*) perpendicular to two sets of diagonal lines on boundary surface between element *i* and element *m*

The program subsequently computes a global M matrix (global mass matrix) according to Equation (48) (step S180). An element M matrix on the right side of Equation (48) is expressed by Equation (49). The global M matrix is a matrix of (NP×r) rows and (NPxr) columns.

[Math. 43]

$$\mathrm{M} = \sum_{i=1}^{NE}{}'\mathrm{M}^{ei} \ : \text{global M matrix (global mass matrix)} \tag{48}$$

$$\sum_{i=1}^{NE}{}' \ : \text{‹ operation of allocating local node numbers constituting matrix for each of elements from element number } i\text{= 1}$$

to element number *i*= NE to global node numbers and combining components from 0, based on mapping of local node number to global node number specified in setting solid soil model

$$\mathrm{M}^{ei} = \int_v \rho_i [N]^{\mathrm{T}}[N]\mathrm{d}v \ : \text{element M matrix (element mass matrix) for element } i \tag{49}$$

$\rho_i$ : density of element $i$ at time $t = t + \theta\Delta t$

The program then computes a global K matrix (global tangent stiffness matrix of soil skeleton) according to Equation (50) (step S190). The global K matrix is a matrix of (NPxr) rows and (NP×r) columns. An element K matrix constituting the global K matrix is expressed by Equation (51). In Equation (51), a first term on the right side represents a tangent stiffness providing mechanical behavior of an identified soil material (for example, sand or clay). A second term on the right side represents a contribution of a time change of soil configuration to the tangent stiffness. A third term on the right side represents a contribution to the tangent stiffness under significant influence of the acceleration to the soil skeleton, for example, impact load. A fourth term on the right side represents a contribution to the tangent stiffness under application of a body force. With regard to the material parameters, providing relative differences to these "structural degradation index", "normal consolidation index" and "rotational hardening limit constant" ensures the continuity of processing from sand, intermediate soil to clay. A D$^{ep}$ matrix (elasto-plastic matrix) included in the first term of Equation (51) is given by Equation (52) in the elasto-plastic state (in the loading state). Additionally, Equation (52) is defined by Equations (53) and (54) for the two-dimensional plane strain condition, by Equations (55) and (56) for the axisymmetric condition and by Equations (57) and (58) for the three-dimensional condition. The D$^{ep}$ matrix is given by Equation (59) in the elastic state. The symbols used in Equations (53) to (58) are defined by Equations (60). The symbols used in Equation (60) are defined by using one or a plurality of Equations (61).

[Math. 44]

$$\mathrm{K} = \sum_{i=1}^{NE} {}'\mathrm{K}^{ei} \ : \text{global K matrix (global tangent stiffness matrix) of soil skeleton} \tag{50}$$

$$\mathrm{K}^{ei} = \int_v [B]^{\mathrm{T}}[D^{ep}][B]\mathrm{d}v + \int_v [N']^{\mathrm{T}}[T_1][N']\mathrm{d}v$$
$$+ \int_v (\rho^w{}_i s^w{}_i + \rho^a{}_i s^a{}_i)[N]^{\mathrm{T}}[N]\{\dot{v}^{Ni}\}\big|_{t+\theta\Delta t}[B_v]\mathrm{d}v - \int_v (\rho^w{}_i s^w{}_i + \rho^a{}_i s^a{}_i)[N]^{\mathrm{T}}\{b\}[B_v]\mathrm{d}v \tag{51}$$

: element K matrix (element tangent stiffness matrix) for element $i$

$\{\dot{v}^{Ni}\}|_{t+\theta\Delta t}$ : column vector expressing acceleration vectors of respective nodes in element $i$ at time $t = t + \theta\Delta t$

$\{b\}$ : body force vector per unit mass

$s^w{}_i$ : degree of saturation of element $i$ at time $t = t + \theta\Delta t$

$s^a{}_i$ : air void ratio of element $i$ at time $t = t + \theta\Delta t$

[Math. 45]

$$[D^{ep}] = [D_1{}^{ep}] - [D_2{}^{ep}] \ : \text{elasto-plastic matrix} \tag{52}$$

[Math. 46]

$$[D_1{}^{ep}] = \begin{bmatrix} a' + b' & a' & 0 \\ a' & a' + b' & 0 \\ 0 & 0 & b'/2 \end{bmatrix} \tag{53}$$

$$[D_2{}^{ep}] = \frac{1}{e'}\begin{bmatrix} (c'\hat{\eta}_{11} - d')^2 & (c'\hat{\eta}_{11} - d')(c'\hat{\eta}_{22} - d') & c'\hat{\eta}_{12}(c'\hat{\eta}_{11} - d') \\ & (c'\hat{\eta}_{22} - d')^2 & c'\hat{\eta}_{12}(c'\hat{\eta}_{22} - d') \\ sym. & & (c'\hat{\eta}_{12})^2 \end{bmatrix} \tag{54}$$

$\hat{\eta}_{ij}$ : $i,j$ components of tensor $\hat{\eta} = \eta - \beta$ ( $i = 1,2, j = 1,2$ )

[Math. 47]

$$[D_1^{ep}] = \begin{bmatrix} a'+b' & a' & 0 & a' \\ a' & a'+b' & 0 & a' \\ 0 & 0 & b'/2 & 0 \\ a' & a' & 0 & a'+b' \end{bmatrix} \tag{55}$$

$$[D_2^{ep}] = \frac{1}{e'} \begin{bmatrix} (c'\hat{\eta}_{11}-d')^2 & (c'\hat{\eta}_{11}-d')(c'\hat{\eta}_{22}-d') & c'\hat{\eta}_{12}(c'\hat{\eta}_{11}-d') & (c'\hat{\eta}_{11}-d')(c'\hat{\eta}_{33}-d') \\ & (c'\hat{\eta}_{22}-d')^2 & c'\hat{\eta}_{12}(c'\hat{\eta}_{22}-d') & (c'\hat{\eta}_{22}-d')(c'\hat{\eta}_{33}-d') \\ & & (c'\hat{\eta}_{12})^2 & c'\hat{\eta}_{12}(c'\hat{\eta}_{33}-d') \\ sym. & & & (c'\hat{\eta}_{33}-d')^2 \end{bmatrix} \tag{56}$$

$\hat{\eta}_{ij}$ : $i,j$ components of tensor $\hat{\eta} = \eta - \beta$ ( $i$=1,2,3, $j$ = 1,2,3 )

[Math. 48]

$$[D_1^{ep}] = \begin{bmatrix} a'+b' & a' & a' & & & \\ a' & a'+b' & a' & & 0 & \\ a' & a' & a'+b' & & & \\ & & & b'/2 & & \\ & 0 & & & b'/2 & \\ & & & & & b'/2 \end{bmatrix} \tag{57}$$

$[D_2^{ep}]$

$$= \frac{1}{e'} \begin{bmatrix} (c'\hat{\eta}_{11}-d')^2 & (c'\hat{\eta}_{11}-d')(c'\hat{\eta}_{22}-d') & (c'\hat{\eta}_{11}-d')(c'\hat{\eta}_{33}-d') & c'\hat{\eta}_{12}(c'\hat{\eta}_{11}-d') & c'\hat{\eta}_{23}(c'\hat{\eta}_{11}-d') & c'\hat{\eta}_{31}(c'\hat{\eta}_{11}-d') \\ & (c'\hat{\eta}_{22}-d')^2 & (c'\hat{\eta}_{22}-d')(c'\hat{\eta}_{33}-d') & c'\hat{\eta}_{12}(c'\hat{\eta}_{22}-d') & c'\hat{\eta}_{23}(c'\hat{\eta}_{22}-d') & c'\hat{\eta}_{31}(c'\hat{\eta}_{22}-d') \\ & & (c'\hat{\eta}_{33}-d')^2 & c'\hat{\eta}_{12}(c'\hat{\eta}_{33}-d') & c'\hat{\eta}_{23}(c'\hat{\eta}_{33}-d') & c'\hat{\eta}_{31}(c'\hat{\eta}_{33}-d') \\ sym. & & & (c'\hat{\eta}_{12})^2 & (c'\hat{\eta}_{12})(c'\hat{\eta}_{23}) & (c'\hat{\eta}_{12})(c'\hat{\eta}_{31}) \\ & & & & (c'\hat{\eta}_{23})^2 & (c'\hat{\eta}_{23})(c'\hat{\eta}_{31}) \\ & & & & & (c'\hat{\eta}_{31})^2 \end{bmatrix}$$

$$\tag{58}$$

$\hat{\eta}_{ij}$ : $i,j$ components of tensor $\hat{\eta} = \eta - \beta$ ( $i$ = 1,2,3 , $j$ = 1,2,3 )

[Math. 49]

$$[D_2^{ep}] = [0] \tag{59}$$

[Math. 50]

$$a' = \widetilde{K} - \frac{2}{3}\widetilde{G}$$

$$b' = 2\widetilde{G}$$

$$c' = 6\widetilde{G}$$

$$d' = \widetilde{K}\alpha$$

$$e' = 12\eta^{*2}\widetilde{G} + \widetilde{K}\alpha^2 + h$$

$$\eta(= q/p') = \sqrt{\frac{3}{2}}\|\boldsymbol{\eta}\|\left(= \sqrt{\frac{3}{2}\boldsymbol{\eta}\cdot\boldsymbol{\eta}}\right) : \text{effective stress ratio}$$

(60)

β : rotational hardening variable tensor (tensor defining direction and magnitude of anisotropy: The greater norm $\|\beta\|$ represents the higher degree of anisotropy)

[Math. 51]

$$\widetilde{K} = \frac{1+e}{\kappa}p'\left(= \frac{J(1+e_0)}{\kappa}p'\right)$$

$$\widetilde{G} = \frac{3(1-2\nu)}{2(1+\nu)}\widetilde{K}$$

$$\alpha = M_a^2 - \eta^2$$

$$h = Jp'\frac{M^2 + \eta^{*2}}{MD}(M_s^2 - \eta^2)$$

(61)

$$D = \frac{\lambda - \kappa}{M(1+e_0)} : \text{dilatancy factor}$$

$$p' = -\frac{1}{3}\text{tr}\,\boldsymbol{T}' : \text{mean effective stress}$$

$$\boldsymbol{\eta} = \boldsymbol{T}'/p' + \boldsymbol{I}$$

$$\eta^* = \sqrt{\frac{3}{2}}\|\hat{\boldsymbol{\eta}}\|$$

$$M_a^2 = M^2 + \frac{3}{2}\boldsymbol{\beta}\cdot\boldsymbol{\beta}$$

: slope of threshold line between plastic compression and plastic expansion in $p' \sim q$ stress (62)

space

$$M_s{}^2 = M_a{}^2 + b_r \frac{4M\eta^{*2}}{M^2 + \eta^{*2}} \left( m_b \eta^* - \sqrt{\frac{3}{2}} \hat{\eta} \cdot \beta \right) - MD \left( \frac{U^*}{R^*} - \frac{U}{R} \right) \sqrt{6\eta^{*2} + \frac{1}{3}\alpha^2} \tag{63}$$

: slope of threshold line between hardening and softening in $p' \sim q$ stress space

e : void ratio at time $t = t + \theta\Delta t$

$e_0$ : void ratio at initial time $t = 0$

$J$ : determinant of deformation gradient tensor of soil skeleton (giving infinitesimal volume ratio of soil skeleton at time $t = t$ relative to initial time $t = 0$ )

$J = 1$ for small deformation analysis

$\lambda$ : compression index

$\kappa$ : swelling index

M : critical state constant

$v$ : *Poisson's* ratio

**I** : unit tensor

$q = \eta \times p'$ : shear stress

Equations (62) and (63) respectively give the slope of a threshold line between plastic compression and plastic expansion and the slope of a threshold line between hardening and softening in the p' -q stress space. Equations (62) and (63) show the case where a plastic stretching norm is employed as the plasticity measure as shown in Equations (64) and (65) given below in the evolution rule which specifies a process of lowering the degree of structure R* (0< R*≤1, the value of R* closer to 0 represents the more highly structured) and a process of losing the degree of overconsolidation R (0< R≤ 1, the value of R closer to 0 represents the higher degree of overconsolidation) in the soil skeleton structure. A shear component of the plastic stretching or a plastic power given as the inner product of the effective stress tensor and the plastic stretching may also be employed as the plasticity measure according to the type of soil. In small deformation analysis, the plastic stretching is specifiable by a plastic strain rate. Symbols "U" in Equation (65) and "U*" in Equation (66) are nonnegative functions and are given by, for example, Equations (66) and (67). Equation (68) given below is used for the evolution rule of the rotational hardening variable tensor representing the anisotropy.

[Math. 52]

$$\dot{R}^* = JU^* \left\| \boldsymbol{D}^p \right\| = JU^* \sqrt{6\eta^{*2} + \frac{1}{3}\alpha^2} \frac{6\widetilde{G}\hat{\eta} \cdot \boldsymbol{D} - \widetilde{K}\alpha(\mathrm{tr}\boldsymbol{D})}{12\eta^{*2}\widetilde{G} + \widetilde{K}\alpha^2 + h} \tag{64}$$

$$\dot{R} = JU \left\| \boldsymbol{D}^p \right\| = JU \sqrt{6\eta^{*2} + \frac{1}{3}\alpha^2} \frac{6\widetilde{G}\hat{\eta} \cdot \boldsymbol{D} - \widetilde{K}\alpha(\mathrm{tr}\boldsymbol{D})}{12\eta^{*2}\widetilde{G} + \widetilde{K}\alpha^2 + h} \tag{65}$$

$\dot{R}^*$ , $\dot{R}$ : rates of $R^*$ and $R$ (material time derivative)

$\boldsymbol{D}^p$ : plastic stretching tensor

$\boldsymbol{D}$ : stretching tensor

$$U^* = \frac{a}{D} R^{*b} \left( 1 - R^* \right)^c \tag{66}$$

$$U = -\frac{m}{D} \ln R \tag{67}$$

*a, b* and *c* : structural degradation indexes (material parameters for controlling the likelihood of degrading the structure)

*m* : normal consolidation index (material parameters for controlling the likelihood of loss of overconsolidation or the likelihood of normal consolidation)

[Math. 53]

$$\overset{\circ}{\boldsymbol{\beta}}(=\dot{\boldsymbol{\beta}}+\boldsymbol{\beta}\boldsymbol{\Omega}-\boldsymbol{\Omega}\boldsymbol{\beta})=J\frac{b_r}{\mathrm{D}}\sqrt{\frac{2}{3}}\left\|\boldsymbol{D}_s^{\ p}\right\|\|\hat{\boldsymbol{\eta}}\|\left(m_b\frac{\hat{\boldsymbol{\eta}}}{\|\hat{\boldsymbol{\eta}}\|}-\boldsymbol{\beta}\right)=J\frac{b_r}{\mathrm{D}}2\eta^{*2}\frac{6\widetilde{G}\hat{\boldsymbol{\eta}}\cdot\boldsymbol{D}-\widetilde{K}\alpha(\mathrm{tr}\boldsymbol{D})}{12\eta^{*2}\widetilde{G}+\widetilde{K}\alpha^2+h}\|\hat{\boldsymbol{\eta}}\|\left(m_b\frac{\hat{\boldsymbol{\eta}}}{\|\hat{\boldsymbol{\eta}}\|}-\boldsymbol{\beta}\right) \qquad (68)$$

β : Green-Nagdhi's rate of $\beta$

β : rate of β

$b_r$ : rotational hardening index (material parameter for controlling the likelihood of evolution of anisotropy β)

$m_b$ : rotational hardening limit constant (material parameter representing evolution limit of anisotropy)

An N' matrix and a T1 matrix of a second term on the right side of Equation (52) are given by Equations (69) and (70) for the two-dimensional plane strain condition, by Equations (71) and (72) for the axisymmetric condition and by Equations (73) and (74) for the three-dimensional condition.

[Math. 54]

$$[N']=\begin{bmatrix} N_{,1}^1 & 0 & \cdots & N_{,1}^m & 0 & \cdots & N_{,1}^p & 0 \\ 0 & N_{,2}^1 & \cdots & 0 & N_{,2}^m & \cdots & 0 & N_{,2}^p \\ N_{,2}^1 & 0 & \cdots & N_{,2}^m & 0 & \cdots & N_{,2}^p & 0 \\ 0 & N_{,1}^1 & \cdots & 0 & N_{,1}^m & \cdots & 0 & N_{,1}^p \end{bmatrix} \qquad (69)$$

$$[T_1]=\begin{bmatrix} 0 & T_{11} & -T_{12} & 0 \\ T_{22} & 0 & 0 & -T_{12} \\ T_{12} & 0 & 0 & -T_{11} \\ 0 & T_{12} & -T_{22} & 0 \end{bmatrix} \qquad (70)$$

$T_{ij}$ : $i,j$ components ($i$ = 1,2, $j$ = 1,2) of total stress tensor $T$ (= $T'$ - ($s^w p^w$ + $s^a p^a$)I) at time $t = t + \theta\Delta t$

[Math. 55]

$$[N']=\begin{bmatrix} N_{,1}^1 & 0 & \cdots & N_{,1}^m & 0 & \cdots & N_{,1}^p & 0 \\ 0 & N_{,2}^1 & \cdots & 0 & N_{,2}^m & \cdots & 0 & N_{,2}^p \\ N_{,2}^1 & 0 & \cdots & N_{,2}^m & 0 & \cdots & N_{,2}^p & 0 \\ 0 & N_{,1}^1 & \cdots & 0 & N_{,1}^m & \cdots & 0 & N_{,1}^p \\ \dfrac{N^1}{x^G_1} & 0 & \cdots & \dfrac{N^m}{x^G_1} & 0 & \cdots & \dfrac{N^p}{x^G_1} & 0 \end{bmatrix} \qquad (71)$$

$$[T_1]=\begin{bmatrix} 0 & T_{11} & -T_{12} & 0 & T_{11} \\ T_{22} & 0 & 0 & -T_{12} & T_{22} \\ T_{12} & 0 & 0 & -T_{11} & T_{12} \\ 0 & T_{12} & -T_{22} & 0 & T_{12} \\ T_{33} & T_{33} & 0 & 0 & 0 \end{bmatrix} \qquad (72)$$

$T_{ij}$ : $i,j$ components ($i$= 1,2, 3, j= 1,2,3) of total stress tensor $\boldsymbol{T}$ at time $t = t + \theta\Delta t$

[Math. 56]

$$[N'] = \begin{bmatrix} N_{,1}^1 & 0 & 0 & \cdots & N_{,1}^m & 0 & 0 & \cdots & N_{,1}^p & 0 & 0 \\ 0 & N_{,2}^1 & 0 & \cdots & 0 & N_{,2}^m & 0 & \cdots & 0 & N_{,2}^p & 0 \\ 0 & 0 & N_{,3}^1 & \cdots & 0 & 0 & N_{,3}^m & \cdots & 0 & 0 & N_{,3}^p \\ N_{,2}^1 & 0 & 0 & \cdots & N_{,2}^m & 0 & 0 & \cdots & N_{,2}^p & 0 & 0 \\ 0 & N_{,1}^1 & 0 & \cdots & 0 & N_{,1}^m & 0 & \cdots & 0 & N_{,1}^p & 0 \\ 0 & N_{,3}^1 & 0 & \cdots & 0 & N_{,3}^m & 0 & \cdots & 0 & N_{,3}^p & 0 \\ 0 & 0 & N_{,2}^1 & \cdots & 0 & 0 & N_{,2}^m & \cdots & 0 & 0 & N_{,2}^p \\ 0 & 0 & N_{,1}^1 & \cdots & 0 & 0 & N_{,1}^m & \cdots & 0 & 0 & N_{,1}^p \\ N_{,3}^1 & 0 & 0 & \cdots & N_{,3}^m & 0 & 0 & \cdots & N_{,3}^p & 0 & 0 \end{bmatrix} \tag{73}$$

$$[T_1] = \begin{bmatrix} 0 & T_{11} & T_{11} & -T_{12} & 0 & 0 & 0 & 0 & -T_{13} \\ T_{22} & 0 & T_{22} & 0 & -T_{12} & -T_{23} & 0 & 0 & 0 \\ T_{33} & T_{33} & 0 & 0 & 0 & 0 & -T_{23} & -T_{13} & 0 \\ T_{12} & 0 & T_{12} & 0 & -T_{11} & -T_{13} & 0 & 0 & 0 \\ 0 & T_{12} & T_{12} & -T_{22} & 0 & 0 & 0 & 0 & -T_{23} \\ T_{23} & T_{23} & 0 & 0 & 0 & 0 & -T_{22} & -T_{12} & 0 \\ T_{23} & 0 & T_{23} & 0 & -T_{13} & -T_{33} & 0 & 0 & 0 \\ 0 & T_{31} & T_{31} & -T_{23} & 0 & 0 & 0 & 0 & -T_{33} \\ T_{31} & T_{31} & 0 & 0 & 0 & 0 & -T_{12} & -T_{11} & 0 \end{bmatrix} \tag{74}$$

$T_{ij}$ : $i,j$ components ($i$= 1,2,3, $j$= 1,2,3) of total stress tensor **T** at time $t = t + \theta\Delta t$

The program subsequently computes a global $L_W{}^T$ matrix (global pore water pressure-related load change rate conversion matrix) and a global $L_A{}^T$ matrix (global pore air pressure-related load change rate conversion matrix) respectively according to Equation (75) and Equation (77) (step S200). An element $L_W{}^T$ matrix on the right side of Equation (75) is expressed by Equation (76), and an element $L_A{}^T$ matrix on the right side of Equation (77) is expressed by Equation (78). Both the global $L_W{}^T$ matrix and the global $L_A{}^T$ matrix are matrices of (NPxr) rows and NE columns.

[Math. 57]

$$L_W{}^T = \sum_{i=1}^{NE} {}^1 L_W{}^{ei\,T} \tag{75}$$

: global $L_W{}^T$ matrix (global pore water pressure-related load change rate conversion matrix) of

soil skeleton

$$L_W{}^{ei\,T} = \int_v n_i C_i \left( \rho^w{}_i - \rho^a{}_i \right) [N]^T \left( [N] \{ \dot{v}^{Ni} \} \Big|_{t+\theta\Delta t} - \{ b \} \right) dv + \int_v \left\{ s^w{}_i + C \left( p^a{}_i - p^w{}_i \right) \right\} [B_v]^T dv \tag{76}$$

: element $L_W{}^T$ matrix (element pore water pressure-related load change rate conversion matrix) of

element $i$

$n_i$ : porosity of element $i$ at time $t = t + \theta\Delta t$
$C_i$ : specific moisture capacity of element $i$ at time $t = t + \theta\Delta t$
$p^a{}_i$ : pore air pressure of element $i$ at time $t = t + \theta\Delta t$
$p^w{}_i$ : pore water pressure of element $i$ at time $t = t + \theta\Delta t$

[Math. 58]

$$L_A^T = \sum_{i=1}^{NE} {}' L_A^{ei\,T}$$

(77)

: global $L_A^T$ matrix (global pore air pressure-related load change rate conversion matrix) of soil

skeleton

$$L_A^{ei\,T} = -\int_v n_i \left\{ \frac{s_i^a}{\overline{R}\Theta_i} + C_i \left( \rho^w_i - \rho^a_i \right) \right\} [N]^T \left( [N] \{ \dot{v}^{Ni} \} \Big|_{t+\theta\Delta t} - \{b\} \right) dv + \int_v \left\{ s_i^a - C_i \left( p^a_i - p^w_i \right) \right\} [B_v]^T dv$$

(78)

: element $L_A^T$ matrix (element pore air pressure-related load change rate conversion matrix) of

element $i$

R : gas constant of the air

$\Theta_i$ : absolute temperature of air for element i

The program subsequently computes a global $S_{WA}$ matrix (global moisture characteristic matrix) and a global $S_{AC}$ matrix (global air compressibility matrix) respectively according to Equations (79) and (80) (step S210). Both these matrices are matrices of NE rows and NE columns.

[Math. 59]

$$S_{WA} = \begin{bmatrix} wa^{e1} & & & & & & \\ & wa^{e2} & & & & 0 & \\ & & \ddots & & & & \\ & & & wa^{ei} & & & \\ & & & & \ddots & & \\ 0 & & & & wa^{e(NE-1)} & \\ & & & & & wa^{e(NE)} \end{bmatrix}$$

(79)

: $S_{WA}$ matrix (global moisture characteristic matrix)

$$wa^{ei} = \int_{ve^i} n_i C_i \, dv$$

$$S_{AC} = \begin{bmatrix} ac^{e1} & & & & & & \\ & ac^{e2} & & & & 0 & \\ & & \ddots & & & & \\ & & & ac^{ei} & & & \\ & & & & \ddots & & \\ 0 & & & & ac^{e(NE-1)} & \\ & & & & & ac^{e(NE)} \end{bmatrix}$$

(80)

: $S_{AC}$ matrix (global air compressibility matrix)

$$ac^{ei} = \int_{ve^i} \frac{n_i s^a_i}{\rho^a_i \overline{R}\Theta_i} \, dv$$

The program then computes a modified global water permeability original L matrix, a modified global air permeability original L matrix, a modified global saturation degree original L matrix, a modified global air void ratio original L matrix, a modified global pore water original L matrix and a modified global pore air original L matrix respectively according to Equation (81), Equation (82), Equation (83), Equation (84), Equation (85) and Equation (86) (step S220). These matrices are matrices of NE rows and (NPxr) columns produced like the global L matrices. The modified global water permeability original L matrix and the modified global air permeability original L matrix are, however, respectively included in the modified global pore water original L matrix and the modified global pore air original L matrix in the process of establishment of global tangent stiffness equations (simultaneous linear equations), so that it is not required to actually calculate the modified global water permeability original L matrix and the modified global air permeability original L matrix.

[Math. 60]

$$
L_{W1} = \frac{1}{g} \begin{bmatrix} k^{w}_{1} L^{e1} \\ k^{w}_{2} L^{e2} \\ \vdots \\ k^{w}_{i} L^{ei} \\ \vdots \\ k^{w}_{(NE-1)} L^{e(NE-1)} \\ k^{w}_{(NE)} L^{e(NE)} \end{bmatrix} \tag{81}
$$

: modified global water permeability original L matrix (modified global water permeability original volume change rate matrix)

$$
L_{A1} = \frac{1}{g} \begin{bmatrix} k^{a}_{1} L^{e1} \\ k^{a}_{2} L^{e2} \\ \vdots \\ k^{a}_{i} L^{ei} \\ \vdots \\ k^{a}_{(NE-1)} L^{e(NE-1)} \\ k^{a}_{(NE)} L^{e(NE)} \end{bmatrix} \tag{82}
$$

: modified global air permeability original L matrix (modified global air permeability original volume change rate matrix)

$$
L_{W2} = \begin{bmatrix} s^{w}_{1} L^{e1} \\ s^{w}_{2} L^{e2} \\ \vdots \\ s^{w}_{i} L^{ei} \\ \vdots \\ s^{w}_{(NE-1)} L^{e(NE-1)} \\ s^{w}_{(NE)} L^{e(NE)} \end{bmatrix} \tag{83}
$$

: modified global saturation degree original L matrix (modified global saturation degree original volume change rate matrix)

$$L_{A2} = \begin{bmatrix} s^a{}_1 \, L^{e1} \\ s^a{}_2 \, L^{e2} \\ \vdots \\ s^a{}_i \, L^{ei} \\ \vdots \\ s^a{}_{(NE-1)} \, L^{e(NE-1)} \\ s^a{}_{(NE)} \, L^{e(NE)} \end{bmatrix} \qquad (84)$$

: modified global air void ratio original L matrix (modified global air void ratio original volume change rate matrix)

[Math. 61]

$$L_{\theta n}{}^{W} = \begin{bmatrix} \gamma_{\theta nW}{}^{1} L^{e1} \\ \gamma_{\theta nW}{}^{2} L^{e2} \\ \vdots \\ \gamma_{\theta nW}{}^{i} L^{ei} \\ \vdots \\ \gamma_{\theta nW}{}^{(NE-1)} L^{e(NE-1)} \\ \gamma_{\theta nW}{}^{(NE)} L^{e(NE)} \end{bmatrix} \qquad (n=1,2,3)$$

$$(85)$$

: modified global pore water original L matrix (modified global pore water original volume change rate matrix)

$$\gamma_{\theta 1W}{}^{i} = -\frac{1}{2\theta\Delta t}\frac{k^w{}_i}{g} + \frac{1}{6}s^w{}_i \quad , \quad \gamma_{\theta 2W}{}^{i} = s^w{}_i - \frac{1}{\theta\Delta t}\frac{k^w{}_i}{g} \quad , \quad \gamma_{\theta 3W}{}^{i} = \frac{1}{6}\left(2s^w{}_i - \frac{3}{\theta\Delta t}\frac{k^w{}_i}{g}\right)$$

$$L_{\theta n}{}^{A} = \begin{bmatrix} \gamma_{\theta nA}{}^{1} L^{e1} \\ \gamma_{\theta nA}{}^{2} L^{e2} \\ \vdots \\ \gamma_{\theta nA}{}^{i} L^{ei} \\ \vdots \\ \gamma_{\theta nA}{}^{(NE-1)} L^{e(NE-1)} \\ \gamma_{\theta nA}{}^{(NE)} L^{e(NE)} \end{bmatrix} \qquad (n=1,2,3)$$

: modified global pore air original L matrix (modified global pore air original volume change rate matrix) $\qquad (86)$

$$\gamma_{\theta 1A}{}^{i} = -\frac{1}{2\theta\Delta t}\frac{\rho^a{}_i}{\rho_0{}^w}\frac{k^a{}_i}{g} + \frac{1}{6}s^a{}_i \quad ,$$

$$\gamma_{\theta 2A}{}^{i} = s^a{}_i - \frac{1}{\theta\Delta t}\frac{\rho^a{}_i}{\rho_0{}^w}\frac{k^a{}_i}{g} \quad ,$$

$$\gamma_{\theta 3\mathrm{A}}{}^i = \frac{1}{6}\left(2s^{\mathrm{a}}{}_i - \frac{3}{\theta \Delta t}\frac{\rho^{\mathrm{a}}{}_i}{\rho_0{}^{\mathrm{w}}}\frac{k^{\mathrm{a}}{}_i}{g}\right)$$

The program subsequently determines the solutions of unknown "jerk field", pore water pressure field and pore air pressure field according to global tangent stiffness equations (simultaneous linear equations) shown by Equation (87) given below under geometric boundary condition with regard to the displacement, the displacement rate or the acceleration, mechanical boundary condition with regard to the stress or the stress rate, hydraulic boundary condition with regard to the pore water pressure or the total water head and the flow rate of pore water and air boundary condition with regard to the pore air pressure or the flow rate of pore air (step S230). This Equation (91) is given by employing an implicit method (finite difference method) to compute a time derivative term in simultaneous second-order ordinal differential equations (Equation (88)) which are eventually obtained by finite element discretization of the weak form of the rate-type equation of motion and modeling of the soil-water-air coupled equations in the light of the influence of an inertial term. Equation (87) is the simultaneous linear equations to be solved (eventually) with application of the Wilson's $\theta$ method to the deformation field and the trapezoidal rule to the pore water pressure and the pore air pressure. The total load increment vector, the total flow increment vector of pore water and the total flow increment vector of pore air, as well as the global M matrix, the global K matrix, the global $H_W$ matrix, the global $H_A$ matrix, the global $L_{BW}$ matrix, the global $L_{BA}$ matrix, the global $L_W{}^T$ matrix, the global $L_A{}^T$ matrix, the global $S_{WA}$ matrix, the global $S_{AC}$ matrix, the global $L_{W2}$ matrix, the global $L_{A2}$ matrix, the global $L_{\theta n}{}^W$ matrix and the global $L_{\theta n}{}^A$ matrix are updated through iterative computations in a time step between the time t=t and the time t=t+$\theta \Delta$t. Equation (88) further processes the equation of motion by material time derivation of the soil skeleton and is accordingly characterized by introduction of a second-order derivative term of a velocity vector as an explanatory variable with respect to time, i.e., a "jerk" term. This characteristic gives a rate-type constitutive equation of the soil skeleton and allows for consideration of a geometric change of soil such as soil foundation. This accordingly allows for large deformation analysis that needs accurate measurement of a volume change of the soil from deformation to failure or with regard to a behavior after the failure. For introduction of this jerk term, Equations (89) to (91) are derived with respect to deformation, based on the concept of linear acceleration method as the basis of the Wilson's $\theta$ method. When a response obtained has little effect of acceleration, Equation (88) is equal to simultaneous first-order ordinary differential equations obtained by static analysis. This means there is no need to selectively use dynamic analysis or static analysis. In the case that a constraint condition of, for example, a fixed length between two nodes or a fixed angle between three nodes is imposed on the internode, the Lagrange's method of undertermined multipliers is applied to solve Equation (88) with consideration of this constraint condition. The geometric boundary condition on the displacement, the displacement rate or the acceleration set at the time t=t+$\Delta$t is converted into a jerk vector at the time t=t+$\theta \Delta$t by Equation (92) when the geometric boundary condition is given by a difference of position vectors, i.e., a displacement vector, by Equation (93) when the geometric boundary condition is given by a velocity vector, and by Equation (94) when the geometric boundary condition is given by an acceleration vector.

[Math. 62]

$$\begin{bmatrix} \dfrac{1}{(\theta\Delta t)^2}M + \dfrac{1}{6}K & -2L_W{}^T & -2L_A{}^T \\[2ex] -L_{\theta 1}{}^W - \dfrac{1}{2(\theta\Delta t)}L_{BW} & H_W(\theta\Delta t)+2S_{WA} & -2S_{WA} \\[2ex] -L_{\theta 1}{}^A - \dfrac{1}{2(\theta\Delta t)}L_{BA} & -2S_{WA} & H_A(\theta\Delta t)+2S_{WA}-2S_{AC} \end{bmatrix} \begin{Bmatrix} \{\ddot{v}^N(\theta\Delta t)^3\}\big|_{t+\theta\Delta t} \\[1ex] \{p^w\}\big|_{t+\theta\Delta t} \\[1ex] \{p^a\}\big|_{t+\theta\Delta t} \end{Bmatrix}$$

$$= \begin{Bmatrix} \{f(\theta\Delta t)\}\big|_{t+\theta\Delta t} - K\left[\{v^N(\theta\Delta t)\}\big|_t + \{\dot{v}^N(\theta\Delta t)^2\}\big|_t + \dfrac{1}{3}\{\ddot{v}^N(\theta\Delta t)^3\}\big|_t\right] \\[2ex] \quad -2L_W{}^T\left[\{p^w\}\big|_t + \dfrac{1}{2}\{\dot{p}^w(\theta\Delta t)\}\big|_t\right] - 2L_A{}^T\left[\{p^a\}\big|_t + \dfrac{1}{2}\{\dot{p}^a(\theta\Delta t)\}\big|_t\right] \\[2ex] \{\dot{f}_w(\theta\Delta t)\}\big|_{t+\theta\Delta t} + L_{W2}\{v^N(\theta\Delta t)\}\big|_t + L_{\theta 2}{}^W\{\dot{v}^N(\theta\Delta t)^2\}\big|_t + L_{\theta 3}{}^W\{\ddot{v}^N(\theta\Delta t)^3\}\big|_t \\[2ex] \quad + 2S_{WA}\left[\{p^w\}\big|_t + \dfrac{1}{2}\{\dot{p}^w(\theta\Delta t)\}\big|_t\right] - 2S_{WA}\left[\{p^a\}\big|_t + \dfrac{1}{2}\{\dot{p}^a(\theta\Delta t)\}\big|_t\right] \\[2ex] \quad + \dfrac{1}{2(\theta\Delta t)}L_{BW}\left[2\{\dot{v}^N(\theta\Delta t)^2\}\big|_t + \{\ddot{v}^N(\theta\Delta t)^3\}\big|_t\right] \\[2ex] \{\dot{f}_a(\theta\Delta t)\}\big|_{t+\theta\Delta t} + L_{A2}\{v^N(\theta\Delta t)\}\big|_t + L_{\theta 2}{}^A\{\dot{v}^N(\theta\Delta t)^2\}\big|_t + L_{\theta 3}{}^A\{\ddot{v}^N(\theta\Delta t)^3\}\big|_t \\[2ex] \quad + (2S_{WA}-2S_{AC})\left[\{p^a\}\big|_t + \dfrac{1}{2}\{\dot{p}^a(\theta\Delta t)\}\big|_t\right] - 2S_{WA}\left[\{p^w\}\big|_t + \dfrac{1}{2}\{\dot{p}^w(\theta\Delta t)\}\big|_t\right] \\[2ex] \quad + \dfrac{1}{2(\theta\Delta t)}L_{BA}\left[2\{\dot{v}^N(\theta\Delta t)^2\}\big|_t + \{\ddot{v}^N(\theta\Delta t)^3\}\big|_t\right] \end{Bmatrix} \tag{87}$$

$\{\ddot{v}^N(\theta\Delta t)^3\}|_{t+\theta\Delta t}$ : total increment vector concerning jerk at time $t = t + \theta\Delta t$

[Math. 63]

$$\begin{bmatrix} M & 0 & 0 \\ 0 & 0 & 0 \\ 0 & 0 & 0 \end{bmatrix}\dfrac{d^2}{dt^2}\begin{Bmatrix} v^N \\ p^w \\ p^a \end{Bmatrix} + \begin{bmatrix} 0 & -L_W{}^T & -L_A{}^T \\ L_{W1}-L_{BW} & S_{WA} & -S_{WA} \\ L_{A1}-L_{BA} & -S_{WA} & S_{WA}-S_{AC} \end{bmatrix}\dfrac{d}{dt}\begin{Bmatrix} v^N \\ p^w \\ p^a \end{Bmatrix}$$
$$+ \begin{bmatrix} K & 0 & 0 \\ -L_{W2} & H_W & 0 \\ -L_{A2} & 0 & H_A \end{bmatrix}\begin{Bmatrix} v^N \\ p^w \\ p^a \end{Bmatrix} = \begin{Bmatrix} \dot{f} \\ \dot{f}_w \\ \dot{f}_a \end{Bmatrix} \tag{88}$$

[Math. 64]

$$\{\dot{v}^N\}\big|_{t+\theta\Delta t} = \{\dot{v}^N\}\big|_t + \dfrac{1}{2}\{\ddot{v}^N\}\big|_t(\theta\Delta t) + \dfrac{1}{2}\{\ddot{v}^N\}\big|_{t+\theta\Delta t}(\theta\Delta t) \tag{89}$$

$$\{v^N\}\big|_{t+\theta\Delta t} = \{v^N\}\big|_t + \{\dot{v}^N\}\big|_t(\theta\Delta t) + \dfrac{1}{3}\{\ddot{v}^N\}\big|_t(\theta\Delta t)^2 + \dfrac{1}{6}\{\ddot{v}^N\}\big|_{t+\theta\Delta t}(\theta\Delta t)^2 \tag{90}$$

$$\{x^N\}\big|_{t+\theta\Delta t} = \{x^N\}\big|_t + \{v^N\}\big|_t(\theta\Delta t) + \dfrac{1}{2}\{\dot{v}^N\}\big|_t(\theta\Delta t)^2 + \dfrac{1}{8}\{\ddot{v}^N\}\big|_t(\theta\Delta t)^3 + \dfrac{1}{24}\{\ddot{v}^N\}\big|_{t+\theta\Delta t}(\theta\Delta t)^3 \tag{91}$$

[Math. 65]

$$\ddddot{v}^{Nj}{}_r\Big|_{t+\theta\Delta t}\left(\theta\Delta t\right)^3 = \ddddot{v}^{Nj}{}_r\Big|_t\left(\theta\Delta t\right)^3 + 24\theta^4\left(x^{Nj}{}_r\Big|_{t+\Delta t} - x^{Nj}{}_r\Big|_t\right) - 24\theta^3 v^{Nj}{}_r\Big|_t\left(\theta\Delta t\right) - 12\theta^2 \dot{v}^{Nj}{}_r\Big|_t\left(\theta\Delta t\right)^2 - 4\theta \ddot{v}^{Nj}{}_r\Big|_t\left(\theta\Delta t\right)^3 \quad (92)$$

$$\ddddot{v}^{Nj}{}_r\Big|_{t+\theta\Delta t}\left(\theta\Delta t\right)^3 = \ddddot{v}^{Nj}{}_r\Big|_t\left(\theta\Delta t\right)^3 + 6\theta^3\left\{v^{Nj}{}_r\Big|_{t+\Delta t}\left(\theta\Delta t\right) - v^{Nj}{}_r\Big|_t\left(\theta\Delta t\right)\right\} - 6\theta^2 \dot{v}^{Nj}{}_r\Big|_t\left(\theta\Delta t\right)^2 - 3\theta \ddot{v}^{Nj}{}_r\Big|_t\left(\theta\Delta t\right)^3 \quad (93)$$

$$\ddddot{v}^{Nj}{}_r\Big|_{t+\theta\Delta t}\left(\theta\Delta t\right)^3 = \ddddot{v}^{Nj}{}_r\Big|_t\left(\theta\Delta t\right)^3 + 2\theta^2\left\{\dot{v}^{Nj}{}_r\Big|_{t+\Delta t}\left(\theta\Delta t\right)^2 - \dot{v}^{Nj}{}_r\Big|_t\left(\theta\Delta t\right)^2\right\} - 2\theta \ddot{v}^{Nj}{}_r\Big|_t\left(\theta\Delta t\right)^3 \quad (94)$$

$x^{Nj}{}_r\Big|_{t+\Delta t}$ : $r$-direction component of position vector of $j$-th node at time $t = t + \Delta t$ set as boundary condition

$v^{Nj}{}_r\Big|_{t+\Delta t}$ : $r$-direction component of velocity vector of $j$-th node at time $t = t + \Delta t$ set as boundary condition

$\dot{v}^{Nj}{}_r\Big|_{t+\Delta t}$ : $r$-direction component of acceleration vector of $j$-th node at time $t = t + \Delta t$ set as boundary condition

$x^{Nj}{}_r\Big|_t$ : $r$-direction component of position vector of $j$-th node at time $t = t$

$v^{Nj}{}_r\Big|_t$ : $r$-direction component of velocity vector of $j$-th node at time $t = t$

$\dot{v}^{Nj}{}_r\Big|_t$ : $r$-direction component of acceleration vector of $j$-th node at time $t = t$

$\ddot{v}^{Nj}{}_r\Big|_t$ : $r$-direction component of jerk vector of $j$-th node at time $t = t$

$\ddot{v}^{Nj}{}_r\Big|_{t+\theta\Delta t}$ : $r$-direction component of converted jerk vector of $j$-th node at time $t = t + \theta\Delta t$

The program subsequently calculates an acceleration field, a velocity field, coordinates and various state quantities at the time t=t+θΔt and identifies a loading state of each Gauss point (step S240). A total increment vector concerning acceleration, a total increment vector concerning velocity and a coordinate vector at the time t=t+θΔt are calculated according to Equations (95) to (97) derived from Equations (89) to (91) using the total increment vector concerning jerk at the time t=t+θΔt obtained by Equation (87) and various geometric quantities at the time t=t. Respective state quantities A, for example, effective stress, degree of structure R*, degree of overconsolidation R, and anisotropy, at the time t=t+θΔ are calculated by Equation (98). For example, an effective stress rate at each Gauss point in a certain element at the time t=t+θΔ is calculated according to Equation (99) from velocity vectors of nodes in the certain element out of the total increment vectors concerning velocity given by Equation (96). An effective stress is subsequently calculated according to Equation (98). The rates, such as the degree of structure, the degree of overconsolidation and the anisotropy are determined according to Equations (64), (65) and (68) for the Gauss points in the elasto-plastic state and are set equal to 0 for the Gauss points in the elastic state. In the modified Cam-clay model with super-sub-loading yield surfaces and rotational hardening, the "plastic volume strain" at the time t=t+θΔt is calculated by Equation (103) for the Gauss points in the elasto-plastic state and is kept at a previous value calculated in the last elasto-plastic state for the Gauss points in the elastic state. In the elastic state, the degree of overconsolidation R at the time t=t+θΔ is accordingly calculable using an equation obtained by solving Equation (103) with respect to R. The pore water pressure and the pore air pressure at the time t=t+θΔ are directly determined by the global tangent stiffness equations of Equation (87). The rates of the pore water pressure and the pore air pressure are determined by inversely applying Equation (98). With regard to whether the state of each Gauss point of the soil element is the elasto-plastic state or the elastic state, it is determined that the state of a Gauss point is the elasto-plastic state upon satisfaction of a loading criterion shown by Equation (104) obtained from the numerator of the plastic multiplier using, for example, effective stress and stretching of the Gauss point calculated from each node of the soil element. A column vector of the stretching is obtained by Equation (105). The components of the column vector at the time t=t+θΔt are expressed by Equation (106) for the two-dimensional plane strain condition, by Equation (107) for the axisymmetric condition and by Equation (108) for the three-dimensional condition.

[Math. 66]

$$\left\{\dot{v}^{N}\left(\theta\Delta t\right)^{2}\right\}\Big|_{t+\theta\Delta t} = \left\{\dot{v}^{N}\left(\theta\Delta t\right)^{2}\right\}\Big|_{t} + \frac{1}{2}\left\{\ddot{v}^{N}\left(\theta\Delta t\right)^{3}\right\}\Big|_{t} + \frac{1}{2}\left\{\ddot{v}^{N}\left(\theta\Delta t\right)^{3}\right\}\Big|_{t+\theta\Delta t} \tag{95}$$

$$\left\{v^{N}\left(\theta\Delta t\right)\right\}\Big|_{t+\theta\Delta t} = \left\{v^{N}\left(\theta\Delta t\right)\right\}\Big|_{t} + \left\{\dot{v}^{N}\left(\theta\Delta t\right)^{2}\right\}\Big|_{t} + \frac{1}{3}\left\{\ddot{v}^{N}\left(\theta\Delta t\right)^{3}\right\}\Big|_{t} + \frac{1}{6}\left\{\ddot{v}^{N}\left(\theta\Delta t\right)^{3}\right\}\Big|_{t+\theta\Delta t} \tag{96}$$

$$\left\{x^{N}\right\}\Big|_{t+\theta\Delta t} = \left\{x^{N}\right\}\Big|_{t} + \left\{v^{N}\left(\theta\Delta t\right)\right\}\Big|_{t} + \frac{1}{2}\left\{\dot{v}^{N}\left(\theta\Delta t\right)^{2}\right\}\Big|_{t} + \frac{1}{8}\left\{\ddot{v}^{N}\left(\theta\Delta t\right)^{3}\right\}\Big|_{t} + \frac{1}{24}\left\{\ddot{v}^{N}\left(\theta\Delta t\right)^{3}\right\}\Big|_{t+\theta\Delta t} \tag{97}$$

[Math. 67]

$$A\Big|_{t+\theta\Delta t} = A\Big|_{t} + \frac{1}{2}\left(\dot{A}\Big|_{t+\theta\Delta t} + \dot{A}\Big|_{t}\right)\left(\theta\Delta t\right) \tag{98}$$

$A|_{t}$, $A|_{t+\theta\Delta t}$ : state quantities $A$ at time $t=t$ and at time $t = t + \theta\Delta t$
$\dot{A}|_{t}$, $\dot{A}|_{t+\theta\Delta t}$ : rates of state quantities $A$ at time $t=t$ and at time $t = t + \theta\Delta t$
[Math. 68]

$$\left\{\dot{T}'\right\}\Big|_{t+\theta\Delta t} \times \left(\theta\Delta t\right) = \left[D^{\mathrm{ep}}\right]\left[B\right]\left\{v^{Ni}\left(\theta\Delta t\right)\right\}\Big|_{t+\theta\Delta t} - \left\{T'_{\Omega}\right\}\Big|_{t+\theta\Delta t} \times \left(\theta\Delta t\right) \tag{99}$$

$\{v^{Ni}(\theta\Delta t)\}|_{t+\theta\Delta t}$ : column vector expression of velocity vectors at respective nodes in element $i$ at time $t = t + \theta\Delta t$
$\{T'\}|_{t+\theta\Delta t}$ : column vector expression of components of effective stress rate tensor $\dot{T}'$ at respective Gauss points in element $i$ at time $t = t + \theta\Delta t$

<2D plane strain condition>

$$\left\{\dot{T}'\right\}\Big|_{t+\theta\Delta t} = \left\{\begin{matrix} \dot{T}'_{11} \\ \dot{T}'_{22} \\ \dot{T}'_{12} \end{matrix}\right\}\Bigg|_{t+\theta\Delta t} \tag{100}$$

<axisymmetric condition>

$$\left\{\dot{T}'\right\}\Big|_{t+\theta\Delta t} = \left\{\begin{matrix} \dot{T}'_{11} \\ \dot{T}'_{22} \\ \dot{T}'_{12} \\ \dot{T}'_{33} \end{matrix}\right\}\Bigg|_{t+\theta\Delta t} \tag{101}$$

<3D condition>

$$\{\dot{T}'\}\Big|_{t+\theta\Delta t} = \left\{\begin{matrix} \dot{T}'_{11} \\ \dot{T}'_{22} \\ \dot{T}'_{33} \\ \dot{T}'_{12} \\ \dot{T}'_{23} \\ \dot{T}'_{31} \end{matrix}\right\}\Bigg|_{t+\theta\Delta t} \tag{102}$$

[Math. 69]

$$\varepsilon_v^{\text{p}}\left(=-\int_0^t J \text{tr}\boldsymbol{D}^{\text{p}} \text{d}\tau\right) = \text{MD}\ln\frac{p'}{p'_0} + \text{MD}\ln\frac{\text{M}^2+\eta^{*2}}{\text{M}^2} + \text{MD}\ln R^* - \text{MD}\ln R \tag{103}$$

: sub-loading yield surface in modified Cam-clay model

$\varepsilon_v^{\text{p}}$ : "plastic volume strain" (compression: positive)

$p'_0$ : $p'(>0)$ value at which the normal yield surface (modified Cam-clay yield surface) at initial time $t = 0$ gives $\eta^*=0$ in $p' \sim q$ stress space

[Math. 70]

$$\frac{\text{MD}}{p'\left(\text{M}^2+\eta^{*2}\right)}\left\{6\tilde{G}\hat{\boldsymbol{\eta}}\cdot\boldsymbol{D} - \tilde{K}\alpha(\text{tr}\boldsymbol{D})\right\} > 0 \tag{104}$$

$$\{\boldsymbol{D}\} = [B]\{v^{Ni}\} \quad : \text{ column vector expression of components of stretching tensor } \boldsymbol{D} \text{ in element } i \tag{105}$$

<2D plane strain condition>

$$\{\boldsymbol{D}\}\Big|_{t+\theta\Delta t} = \left\{\begin{matrix} D_{11} \\ D_{22} \\ 2D_{12} \end{matrix}\right\}\Bigg|_{t+\theta\Delta t} \tag{106}$$

<axisymmetric condition>

$$\{\boldsymbol{D}\}\Big|_{t+\theta\Delta t} = \left\{\begin{matrix} D_{11} \\ D_{22} \\ 2D_{12} \\ D_{33} \end{matrix}\right\}\Bigg|_{t+\theta\Delta t} \tag{107}$$

<3D condition>

$$\{\boldsymbol{D}\}\big|_{t+\theta\Delta t} = \left\{\begin{array}{c} D_{11} \\ D_{22} \\ D_{33} \\ 2D_{12} \\ 2D_{23} \\ 2D_{31} \end{array}\right\}\Bigg|_{t+\theta\Delta t} \tag{108}$$

The program subsequently detects convergence using the effective stresses of the respective Gauss points in the respective elements (step S250). The procedure of the first embodiment detects convergence, based on whether Equation (109) given below is satisfied for all the Gauss points in all the elements. In Equation (109), "ε" is a sufficiently small positive value. Upon no detection of the convergence, the program returns to the computation of the global $H_W$ matrix and the global $H_A$ matrix of step S150 and repeats the processing of steps S150 to S250.

[Math. 71]

$$\left| \frac{T_e'^{(n)} - T_e'^{(n-1)}}{T_e'^{(n)}} \right| \leq \varepsilon \tag{109}$$

$$T_e'^{(n)} = \left\| \boldsymbol{T}'^{(n)} \right\| = \sqrt{\boldsymbol{T}'^{(n)} \cdot \boldsymbol{T}'^{(n)}} \tag{110}$$

: norm of effective stress tensor $\boldsymbol{T}'^{(n)}$ at Gauss point obtained by n-th iterative computation at time $t = t + \theta\Delta t$

Upon detection of the convergence at step S250, on the other hand, the program subsequently calculates an acceleration field, a velocity field, coordinates and various state quantities at a time t=t+Δt and identifies a loading state of each Gauss point (step S260). The acceleration field, the velocity field and the coordinates at the time t=t+Δt are calculated from the total increment vector concerning jerk at the time t=t+θΔt and the total increment vector concerning acceleration and the total increment vector concerning velocity and the coordinate vector at the time t=t according to Equations (111) to (114) given below. Respective state quantities and their rates at the time t=t+Δt are calculated from the state quantities at the time t=t and at the time t=t+θΔt according to Equations (115) and (116). The loading state of each Gauss point at the time t=t+Δt is identified, based on the respective state quantities at the time t=t+θΔt according to Equations (104) and (105).

[Math. 72]

$$\left\{\dddot{v}^N(\theta\Delta t)^3\right\}\big|_{t+\Delta t} = \left\{\dddot{v}^N(\theta\Delta t)^3\right\}\big|_t + \frac{1}{\theta}\left[\left\{\dddot{v}^N(\theta\Delta t)^3\right\}\big|_{t+\theta\Delta t} - \left\{\dddot{v}^N(\theta\Delta t)^3\right\}\big|_t\right] \tag{111}$$

$$\left\{\ddot{v}^N(\theta\Delta t)^2\right\}\big|_{t+\Delta t} = \left\{\ddot{v}^N(\theta\Delta t)^2\right\}\big|_t + \frac{1}{\theta}\left\{\dddot{v}^N(\theta\Delta t)^3\right\}\big|_t + \frac{1}{2\theta^2}\left[\left\{\dddot{v}^N(\theta\Delta t)^3\right\}\big|_{t+\theta\Delta t} - \left\{\dddot{v}^N(\theta\Delta t)^3\right\}\big|_t\right] \tag{112}$$

$$\left\{\dot{v}^N(\theta\Delta t)\right\}\big|_{t+\Delta t} = \left\{\dot{v}^N(\theta\Delta t)\right\}\big|_t + \frac{1}{\theta}\left\{\ddot{v}^N(\theta\Delta t)^2\right\}\big|_t + \frac{1}{2\theta^2}\left\{\dddot{v}^N(\theta\Delta t)^3\right\}\big|_t + \frac{1}{6\theta^3}\left[\left\{\dddot{v}^N(\theta\Delta t)^3\right\}\big|_{t+\theta\Delta t} - \left\{\dddot{v}^N(\theta\Delta t)^3\right\}\big|_t\right] \tag{113}$$

$$\left\{x^N\right\}\big|_{t+\Delta t} = \left\{x^N\right\}\big|_t + \frac{1}{\theta}\left\{\dot{v}^N(\theta\Delta t)\right\}\big|_t + \frac{1}{2\theta^2}\left\{\ddot{v}^N(\theta\Delta t)^2\right\}\big|_t + \frac{1}{6\theta^3}\left\{\dddot{v}^N(\theta\Delta t)^3\right\}\big|_t + \frac{1}{24\theta^4}\left[\left\{\dddot{v}^N(\theta\Delta t)^3\right\}\big|_{t+\theta\Delta t} - \left\{\dddot{v}^N(\theta\Delta t)^3\right\}\big|_t\right] \tag{114}$$

[Math. 73]

43

$$\dot{A}\Big|_{t+\Delta t}(\theta\Delta t) = \dot{A}\Big|_{t}(\theta\Delta t) + \frac{2}{\theta}\left\{A\Big|_{t+\theta\Delta t} - A\Big|_{t} - \dot{A}\Big|_{t}(\theta\Delta t)\right\} \qquad (115)$$

$$A\Big|_{t+\Delta t} = A\Big|_{t} + \frac{1}{2\theta}\left\{\dot{A}\Big|_{t+\Delta t}(\theta\Delta t) + \dot{A}\Big|_{t}(\theta\Delta t)\right\} \qquad (116)$$

$A|_{t+\Delta t}$: state quantity $A$ at time $t = t + \Delta t$

$\dot{A}|_{t+\Delta t}$: rate of state quantity $A$ at time $t = t + \Delta t$

The program then determines whether the current computation flow reaches the number of computation steps input at step S100 (step S270). When the current computation flow does not still reach the input number of computation steps, the program returns to the estimation of the various conditions (for example, effective stress, pore water pressure, pore air pressure and structure/ overconsolidation/ anisotropy) at the time t=t+θΔt of step S130 as a next computation step. When the current computation flow reaches the input number of computation steps, on the other hand, the soil-water-air coupled analyzing program 30 outputs the computation results in the form of a specified output file (step S280) and is then terminated.

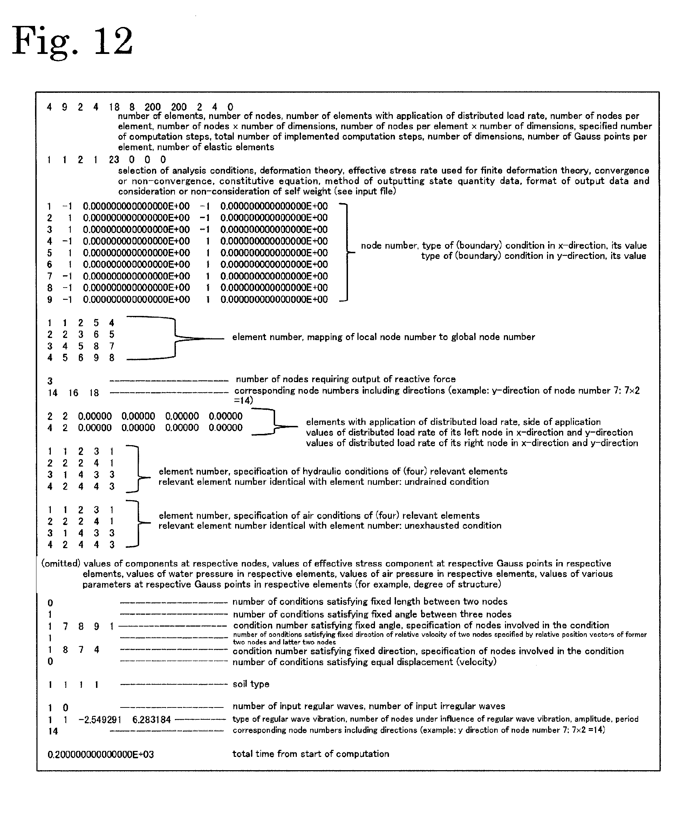

One example of the output file is shown in Fig. 12. In this illustrated example, data on the 1st line successively show, from the left, the number of elements, the number of nodes, the number of elements with application of distributed load rate, the number of nodes, the number of nodes × the number of dimensions, the number of nodes per element × the number of dimensions, the specified number of computation steps, the total number of implemented computation steps, the number of dimensions, the number of Gauss points per element and the number of elastic elements. Data on the 2nd line successively show, from the left, selection of analysis conditions, a deformation theory, an effective stress rate used for the finite deformation theory, convergence or non-convergence, a constitutive equation, a method of outputting state quantity data, a format of output data and consideration or non-consideration of self weight. Data on the 3rd to the 11th lines successively show, from the left in each line, a node number, a type of boundary condition in the x-direction and its value and a type of boundary condition in the y-direction and its value. Data on the 12th to the 15th lines show an element number at the left most end in each line and global node numbers mapped to local node numbers with regard to the corresponding element. Data on the 16th line shows the number of nodes requiring output of reactive force. Data on the 17th line show node numbers requiring output of reactive force as values including directions at the corresponding nodes. Data on the 18th and 19th lines successively show, from the left in each line, an element number with application of distributed load rate, a side of application (internode of application), a value in the x-direction of the distributed load rate of its left node, a value in the y-direction of the distributed load rate of the left node, a value in the x-direction of the distributed load rate of its right node and a value in the y-direction of the distributed load rate of the right node. Data on the 20th to the 23rd lines show the hydraulic boundary conditions. Data on the 24th to the 27th lines show the air boundary conditions. The output file also includes, for example, material parameters at respective Gauss points in respective elements, values of components at respective nodes, values of pore water pressure in respective elements, values of pore air pressure in respective elements, values of effective stress component at respective Gauss points in respective elements and values representing degree of structure, although they are "omitted" from the illustration of Fig. 12. Data on the 1st line after the "omission" shows the number of conditions satisfying a fixed length between two nodes. When the number of conditions satisfying the fixed length between two nodes is equal to or greater than 1, its condition number and specification of nodes involved in the condition are shown on a next line. Data on the 2nd line after the "omission" shows the number of conditions satisfying a fixed angle between three nodes. Data on the subsequent 3rd line show a condition number satisfying the fixed angle between three nodes and specification of nodes (three) involved in the condition. Data on the 4th line after the "omission" show the number of conditions satisfying a fixed direction of a relative velocity of two nodes specified by relative position vectors of former two nodes and latter two nodes. Data on the subsequent 5th line show its condition number and specification of nodes involved in the condition. Data on the 6th line after the "omission" shows the number of conditions satisfying an equal displacement (velocity) . When there is any condition satisfying the equal displacement (velocity), its condition number and specification of nodes are shown on a next line. Data on the 7th line after the "omission" shows a soil type. Data on the 8th line after the "omission" shows the number of input regular waves and the number of input irregular waves. With regard to the regular wave, data on the 9th and 10th lines show a type of regular wave vibration, the number of nodes under influence of the regular wave vibration, the amplitude and the period of the regular wave vibration and node numbers under influence of the regular wave vibration as values including directions. When there is any input irregular wave, a type of irregular wave vibration, the number of nodes under influence of the irregular wave vibration, node numbers under influence of the irregular wave vibration as values including directions and other data are similarly shown on next two lines. Data on the last line shows a total time since start of computation.

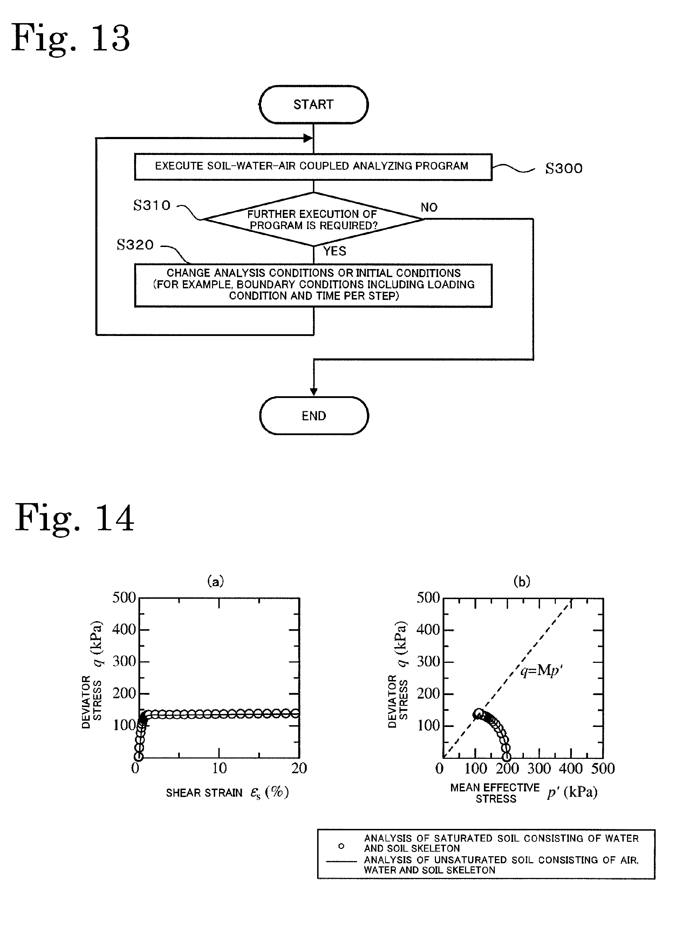

The soil-water-air coupled analyzing program 30 according to the first embodiment described above inputs and sets a plurality of different soil types to elements and estimates the elasto-plastic deformation behaviors of the soil skeleton in the soil-water-air coupled field under various loading conditions including dynamic loading conditions and static loading conditions with regard to the naturally-deposited, highly structured and overconsolidated ground or specimen, as well as the artificially made remolded soil. The soil-water-air coupled analyzing program 30 of the first embodiment accordingly addresses the following issues: deformation behaviors (including bifurcation behavior and post-peak behavior) and self weight consolidation analysis of the specimen; consolidation settlement and lateral movement behaviors (including "secondary consolidation"-like, long-term continuous settlement behavior) of soil under loading like road embankment; soil deformation behaviors in excavation; water pumping-induced soil subsidence; soil bearing capacity; liquefaction and liquefaction-induced settlement (shaking settlement) behaviors under repetitive loading like earthquake; stability/ instability by filtration flow; compacted soil improvement due to cylindrical cavity expansion by sand piles; interaction with an elastic structure (deformation behavior of piled-raft foundation); deformation behaviors of reinforced soil; soil improvement by improvement of mass permeability; and stability of an elasto-plastic embankment structure. Additionally, the broad scope from saturated soil to unsaturated soil expands the coverage of issues to be addressed to deformation and failure behaviors of embankment or slope due to rainfall seepage, deformation and failure behaviors on embankment or slope under the combined condition of torrential rain with earthquake, seismic assessment of river bank and slaking phenomenon of mudstone. In other words, this allows for deformation analysis of the soil skeleton, whether static analysis or dynamic analysis, with regard to unsaturated soil having the air in addition to water in voids of the soil skeleton as well as saturated soil having water saturated in voids of the soil skeleton. When these issues are to be addressed, the soil-water-air coupled analyzing program shown in Fig. 2 should be executed a plurality of times as needed with setting the results of the previous analysis as new analysis conditions or initial conditions. In this case, as shown in the flowchart of Fig. 13, after execution of the soil-water-air coupled analyzing program (step S300), it is determined whether further execution of the program is required (strep S310). Upon requirement for further execution, the soil-water-air coupled analyzing program is executed again at step S300 after change of the analysis conditions or the initial conditions (step S320). Upon no requirement for further execution, the soil-water-air coupled analyzing program is terminated.

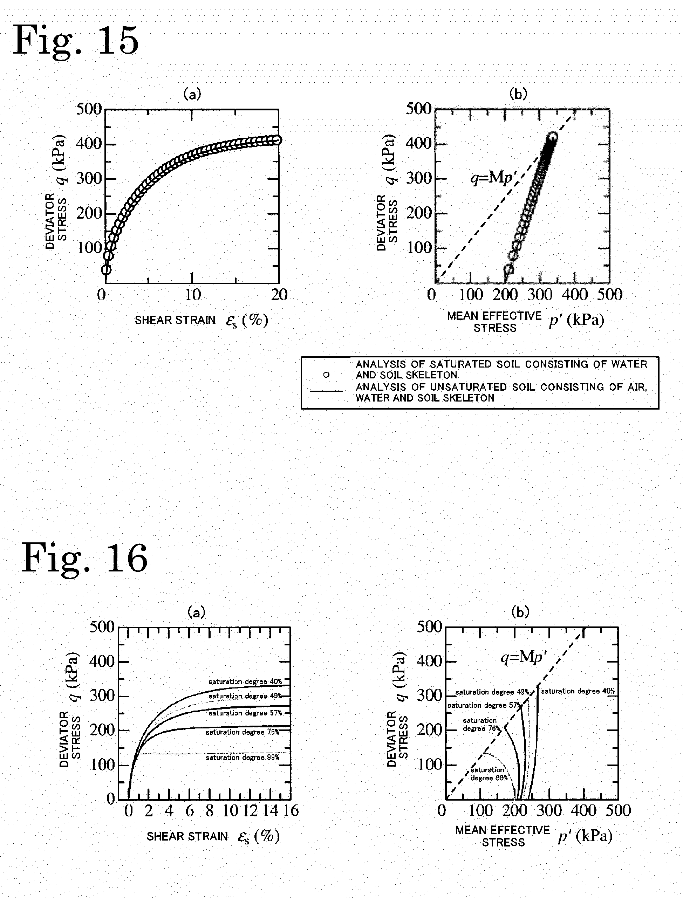

Figs. 14a and 14b respectively show the relation between the deviator stress and the shear strain and the relationship between the deviator stress and the mean effective stress in simulation of an undrained and unexhausted triaxial compression test with setting an initial degree of saturation to a maximum degree of saturation, in comparison with simulation of an undrained triaxial compression test by analysis of saturated soil consisting of water and soil skeleton. Figs. 15a and 15b respectively show the relation between the deviator stress and the shear strain and the relationship between the deviator stress and the mean effective stress in simulation of a drained and exhausted triaxial compression test with setting an initial degree of saturation to a maximum degree of saturation, in comparison with simulation of a drained triaxial compression test by analysis of saturated soil consisting of water and soil skeleton. In both the simulations, the results by the analysis of unsaturated soil consisting of the air, water and soil skeleton are substantially equivalent to the results by the analysis of saturated soil consisting of water and soil skeleton. This indicates that soil-water-air coupled analyzer, the soil-water-air coupled analyzing method and the soil-water-air coupled analyzing program of the invention can continuously deal with unsaturated soil to saturated soil.

Figs. 16a and 16b respectively show the relation between the deviator stress and the shear strain and the relationship between the deviator stress and the mean effective stress as the results of simulation of an undrained and unexhausted triaxial compression test with regard to five different initial degrees of saturation. These results indicate the typical mechanical behaviors of unsaturated soil, i.e. , the strength and the stiffness increase and the effective stress path shifts to the right with a decrease in initial degree of saturation.

The soil-water-air coupled analyzer 20 of the first embodiment described above computes the global tangent stiffness equations (simultaneous linear equations) using the global L matrix, the global $L_{W2}$ matrix, the global $L_{A2}$ matrix, the global $L_{\theta n}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $S_{WA}$ matrix and the global $S_{AC}$ matrix to provide the geometric boundary condition with regard to the displacement, the displacement rate or the acceleration, the mechanical boundary condition with regard to the stress or the stress rate, the hydraulic boundary condition with regard to the pore water pressure or the total water head and the flow rate of pore water and the air boundary condition with regard to the pore air pressure or the flow rate of pore air to determine the solutions of the unknown "jerk field", pore water pressure field and pore air pressure field. This allows for consideration of a geometric change of soil such as soil foundation and accordingly allows for large deformation analysis that needs accurate measurement of a volume change of the soil from deformation to failure or with regard to a behavior after the failure. In other words, this allows for deformation analysis of the soil skeleton, whether static analysis or dynamic analysis, with regard to unsaturated soil having the air in addition to water in voids of the soil skeleton as well as saturated soil having water saturated in voids of the soil skeleton. The first embodiment also allows for setting of a soil type and more specifically allows for setting of a soil type for each element and is thus applicable to compute the mechanical behaviors in a wide range of soil skeletons having various skeleton structures (structure, overconsolidation and anisotropy) including a continuously varying soil structure of sand, intermediate soil to clay, unusual or problematic soils like peat and volcanic ash soil and a layered soil structure of sand, clay and intermediate soil. Additionally, the broad scope from saturated soil to unsaturated soil expands the coverage of issues to be addressed to deformation and failure behaviors of embankment or slope due to rainfall seepage, deformation and failure behaviors on embankment or slope under the combined condition of torrential rain with earthquake, seismic assessment of river bank and slaking phenomenon of mudstone. When an obtained response has little effect of the acceleration, the global tangent stiffness equations are equal to global stiffness equations obtained by static analysis. This allows for computation of the mechanical behaviors of the soil skeleton, whether static of dynamic. More specifically, this allows for static deformation analysis subsequent to dynamic deformation analysis or dynamic deformation analysis subsequent to static deformation analysis, such as consolidation deformation behaviors after earthquake-induced liquefaction as well as seismic deformation behaviors during consolidation deformation.

The first embodiment uses the equation of average soil skeleton stress (equation of $X= s^w$ in the Bishop's effective stress equation), but $X= s^w$ is not restrictive and another effective stress equation may be employed.

The first embodiment describes on the assumption that the pore water is incompressible fluid and the pore air is compressible fluid. The pore water may alternatively be compressible fluid.

The first embodiment does not consider a temperature change but may consider a temperature change.

The first embodiment does not consider a phase change between three phases (solid phase, liquid phase and gas phase) but may consider a phase change between the respective phases.

The soil-water-air coupled analyzer 20 of the first embodiment allows for setting of a soil type and more specifically allows for setting of a soil type for each element. The available type of soils may be limited, or the soil type may be set for each element block, instead of each element.

The soil-water-air coupled analyzer 20 of the first embodiment performs rigorous calculation using the global L matrix, the global $L_{W2}$ matrix, the global $L_{A2}$ matrix, the global $L_{\theta n}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $S_{WA}$ matrix and the global $S_{AC}$ matrix as the respective element matrices, but the calculation may be approximate calculation.

The soil-water-air coupled analyzer 20 of the first embodiment calculates the global L matrix, the global $L_{W2}$ matrix, the global $L_{A2}$ matrix, the global $L_{\theta n}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $S_{WA}$ matrix and the global $S_{AC}$ matrix and establishes the global tangent stiffness equations (simultaneous linear equations) using all these plural different types of matrices. Alternatively the global tangent stiffness equations (simultaneous linear equations) may be established using part of the global L matrix, the global $L_{W2}$ matrix, the global $L_{A2}$ matrix, the global $L_{\theta n}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $S_{WA}$ matrix and the global $S_{AC}$ matrix. In this case, any other suitable element but these plural different types of matrices may also be used.

The soil-water-air coupled analyzer 20 of the first embodiment employs the finite element method for deformation analysis of the soil skeleton but may perform deformation analysis of the soil skeleton without using the finite element method.

The soil-water-air coupled analyzer 20 of the first embodiment inputs the settings of the following analysis conditions as shown in the specific example of Figs. 5 and 6: the setting of "plane strain", "axisymmetric" or "three-dimensional" as the computation condition; selection of either "small deformation theory" or "finite deformation theory" as the deformation theory; the setting of either "Jaumann's rate of Cauchy stress" or "Green-Naghdi's rate" as the effective stress rate with objectivity used in the finite deformation theory; the setting of convergence or non-convergence; selection of either "original Cam-clay model with super-sub-loading yield surfaces and rotational hardening" or "modified Cam-clay model with super-sub-loading yield surfaces and rotational hardening" as the constitutive equation; the setting of either "element average of Gauss points" or "value of a specific Gauss point" as the state quantity output data; setting of either "Text" or "Binary" as the output data format; the setting of consideration or non-consideration of self weight; the current program number; the number of computation steps; the computation time DT per computation step (sec/step) ; the number of divisions of loading during embankment or excavation; the number of divisions of distributed load under application of distributed load; the input file for soil material parameters and initial values; the input file for mesh data; and specification of the output file for initial computation conditions used in execution of a next program. The analysis conditions to be set are, however, not limited to these parameters. Only part of these parameters may be input as the analysis conditions, or any other suitable parameter but the above parameters may be input. Additionally, any suitable format may be employed as the input format.

The soil-water-air coupled analyzer 20 of the first embodiment employs the van Genuchten's equation as the moisture characteristic equation of soil and the Mualem model as the water permeability coefficient/ air permeability coefficient model but may employ another moisture characteristic-related equation of soil which takes into account hysteresis, a void ratio change and temperature dependency and uses the volume water content and the moisture content or another water permeability coefficient/ air permeability coefficient model.

The soil-water-air coupled analyzer 20 of the first embodiment inputs the following settings of soils, such as clay, intermediate soil and sand, for the respective elements constituting the soil foundation as shown in the specific example of Fig. 7: the number of different types of soils as the elasto-plastic bodies, the number of different types of elastic elements, material numbers, specification of the progress scale of plastic deformation adopted in the evolution rule of overconsolidation, specification of the progress scale of plastic deformation adopted in the evolution rule of structure, the intercept N of the isotropic normal consolidation line of remolded soil, the critical state constant M, the compression index $\lambda$, the swelling index $\kappa$, the Poisson's ratio v, the coefficient of saturated water permeability $k_s^w$, the specific gravity $G_s$ of soil particles, the initial lateral pressure coefficient $K_0$, the initial degree of overconsolidation $1/R_0$, the initial degree of structure $1/R^*_0$, the normal consolidation index m, the U* shape, the structural degradation index a, the structural degradation index b, the structural degradation index c, the structural degradation index $c_s$, the rotational hardening index $b_r$, the rotational hardening limit surface $m_b$, the initial degree of anisotropy, the initial void ratio, the coefficient of dry air permeability $k_d^a$, the parameters $s^w_{max}$, $s^w_{min}$, $\alpha_v$, $m_v$ and $n_v$ of the moisture characteristic curve, the parameters $\xi$ and $\gamma$ of the water permeability coefficient/ air permeability coefficient model, the temperature of the air, the initial overburden pressure of the soil foundation, the cell pressure (tensile: positive), the air pressure, the suction, the gas constant of the air, the settings of the unit system (time, force and length), the density of the viscous boundary, the compression wave velocity and the shear wave velocity. The settings of soils are, however, not limited to these parameters. Only part of these parameters may be input as the settings, or any other suitable parameter but the above parameters may be input. Additionally, any suitable format may be employed as the input format.

The soil-water-air coupled analyzer 20 of the first embodiment inputs the following settings of the solid soil model as shown in the specific example of Fig. 8: the number of elements, the number of nodes and the number of elements with application of distributed load rate in the solid soil model, the number of nodes per element, the number of computation steps and the number of elastic elements as the data on the 1st line; the node number, the type of boundary condition in then x-direction, the value of the boundary condition in the x-direction, the type of boundary condition in the y-direction and the value of the boundary condition in the y-direction as the data on each of the 2nd to the 10th lines; the element number and the global node numbers mapped to the local node numbers with regard to the corresponding element as the data on each of the 11th to the 14th lines; the node number, the coordinate in the x-direction and the coordinate in the y-direction as the data on each of the 15th to the 23rd lines; the number of nodes requiring output of reactive force and their node numbers as the data on the 24th and the 25th lines; the element number with application of distributed load rate, the side of application (internode of application), the value in the x-direction of the distributed load rate of its left node, the value in the y-direction of the distributed load rate of the left node, the value in the x-direction of the distributed load rate of its right node and the value in the y-direction of the distributed load rate of the right node as the data on each of the 26th and the 27th lines; the element number, the element number adjacent to the internode 1 of the corresponding element, the element number adjacent to the internode 2 of the corresponding element, the element number adjacent to the internode 3 of the corresponding element and the element number adjacent to the internode 4 of the corresponding element (settings of hydraulic boundary) as the data on each of the 28th to the 31st lines; the element number, the element number adjacent to the internode 1 of the corresponding element, the element number adjacent to the internode 2 of the corresponding element, the element number adjacent to the internode 3 of the corresponding element and the element number adjacent to the internode 4 of the corresponding element (settings of air boundary) as the data on each of the 32nd to the 35th lines; the number of conditions satisfying the fixed length between two nodes (and the settings of the condition) as the data on the 36th line; the number of conditions satisfying the fixed angle between three nodes and the settings of the condition as the data on the 37th and the 38th lines; the number of conditions satisfying the fixed direction of the relative velocity of two nodes specified by relative position vectors of former two nodes and latter two nodes and the settings of the condition as the data on the 39th and the 40th lines; the number of conditions satisfying the equal displacement (velocity) (and the settings of the condition) as the data on the 41st line; the soil type as the data on the 42nd line; the number of regular waves input into the model and the number of irregular waves input into the model as the data on the 43rd line; the type of regular wave vibration input into the model, the number of nodes under influence of the regular wave vibration input into the model and the amplitude and the period of the regular wave vibration as the data on the 44th line; the node number under influence of the regular wave vibration as the data on the 45th line; and the specification of the irregular waves as the data on a new line below the 45th line. The settings of the solid soil model are, however, not limited to these parameters. Only part of these parameters may be input as the settings of the solid soil model, or any other suitable parameter but the above parameters may be input. Additionally, any suitable format may be employed as the input format.

The soil-water-air coupled analyzer 20 of the first embodiment is allowed to execute the soil-water-air coupled analyzing program a plurality of times using the results of computation of the soil-water-air coupled analyzing program as the new computation conditions or initial conditions. The number of times of repeatedly executing the soil-water-air coupled analyzing program may be limited to a specific number or may be limited to only once.

The soil-water-air coupled analyzer 20 of the first embodiment calculates the element K matrix according to Equation (51) including: the term representing the tangent stiffness providing the mechanical behavior of the identified soil material (for example, sand or clay) (first term on the right side) ; the term representing the contribution of the time change of soil configuration to the tangent stiffness (second term on the right side) ; the term representing the contribution to the tangent stiffness under significant influence of the acceleration to the soil skeleton, for example, impact load (third term on the right side) ; and the term representing the contribution to the tangent stiffness under application of the body force (fourth term on the right side) . The element K matrix may, however, be calculated without considering part of these terms under some analysis conditions.

The soil-water-air coupled analyzer 20 of the first embodiment is configured to perform the analysis for any of the two-dimensional plane strain condition, the axisymmetric condition and the three-dimensional condition. The soil-water-air coupled analyzer may, however, be configured to perform the analysis only for the two-dimensional plane strain condition or may be configured to perform the analysis for only the two-dimensional plane strain condition and the axisymmetric condition.

The soil-water-air coupled analyzer 20 of the first embodiment is configured to address the issues of: deformation behaviors (including bifurcation behavior and post-peak behavior) and self weight consolidation analysis of the specimen; consolidation settlement and lateral movement behaviors (including "secondary consolidation"-like, long-term continuous settlement behavior) of soil under loading like road embankment; soil deformation behaviors in excavation; water pumping-induced soil subsidence; soil bearing capacity; liquefaction and liquefaction-induced settlement (shaking settlement) behaviors under repetitive loading like earthquake; stability/ instability by filtration flow; compacted soil improvement due to cylindrical cavity expansion by sand piles; interaction with an elastic structure (deformation behavior of piled-raft foundation); deformation behaviors of reinforced soil; soil improvement by improvement of mass permeability; stability of an elasto-plastic embankment structure; deformation and failure behaviors of embankment or slope due to rainfall seepage; deformation and failure behaviors on embankment or slope under the combined condition of torrential rain with earthquake; seismic assessment of river bank; and slaking phenomenon of mudstone. The soil-water-air coupled analyzer may, however, be configured to address only part of these issues or may be configured to address only one of these issues.

The first embodiment describes the soil-water-air coupled analyzer 20 in which the soil-water-air coupled analyzing program 30 is installed. The invention may be implemented as the soil-water-air coupled analyzing program 30 or may be implemented as the computation module 34 of the soil-water-air coupled analyzing program 30.

Second Embodiment

The following describes a soil-water-air coupled analyzer 220 according to a second embodiment of the invention. The first embodiment describes the formulation on the assumption that the pore water is incompressible. The second embodiment takes into account the compressibility of the pore water and additionally gives more detailed consideration on the degree of saturation. The soil-water-air coupled analyzer 220 of the second embodiment has the similar hardware configuration to that of the soil-water-air coupled analyzer 20 of the first embodiment shown in Fig. 1 and is configured by installing a soil-water-air coupled analyzing program 230 as application software in a general-purpose computer 222. Like the soil-water-air analyzing program 30 of the first embodiment, the soil-water-air coupled analyzing program 230 of the second embodiment includes: a data input module 232 configured to input data for setting the analysis conditions, the soil profiles and a solid soil model; a computation module 234 configured to analyze the mechanical behaviors of unsaturated soil from the input data according to an elasto-plastic constitutive equation; and a result output module 236 configured to output the result of the analysis. The respective components of the soil-water-air coupled analyzer 220 of the second embodiment are similar to those shown in Fig. 1, except that these components are expressed by the corresponding numerals in 200s, and are thus not specifically illustrated herein.

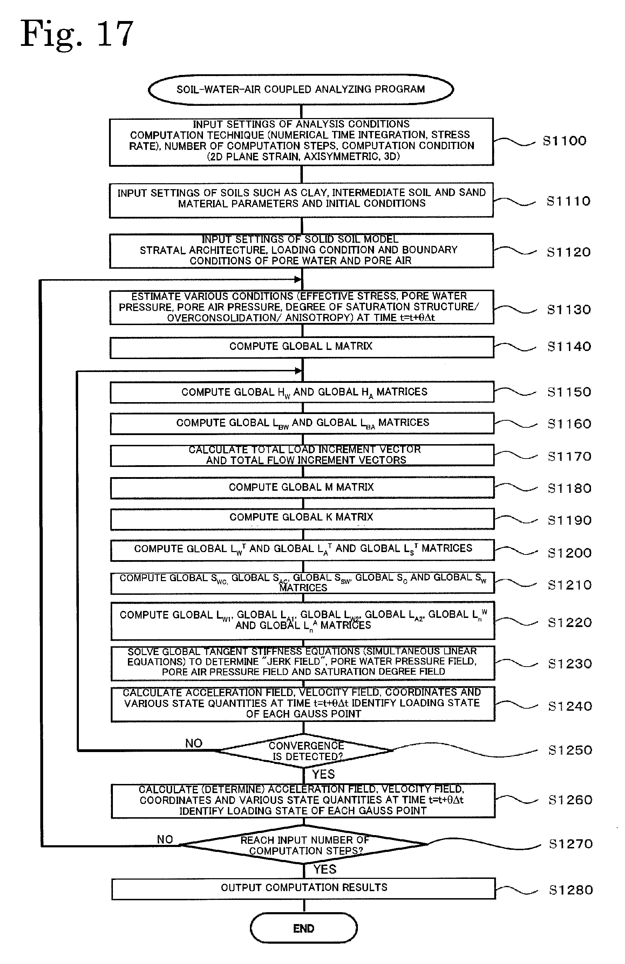

Fig. 17 is a flowchart showing one exemplary process according to the soil-water-air coupled analyzing program 230 of the second embodiment. Like the first embodiment, for the simplicity of explanation, the second embodiment also describes the process of the soil-water-air coupled analyzing program 230 with regard to a specific example on the assumption that a soil foundation as the analysis object has four elements expressed by a 2x2 matrix (see Figs. 3 and 4). The element numbers, the node number and the internode numbers are the same as those described with regard to the first embodiment.

The soil-water-air coupled analyzing program 230 of the second embodiment is triggered to first input settings of the analysis conditions (step S1100). Like the first embodiment, the settings of analysis conditions input herein may include, for example, selection of a technique of numerical time integration based on the difference method, an update frequency of time (number of computation steps), and selection of a computation condition, such as two-dimensional plane strain, axisymmetric or three-dimensional. Like the first embodiment, Figs. 5 and 6 should be referred to as the exemplary files of the specific example to input the settings of analysis conditions. The details of the setting of analysis conditions shown in Figs. 5 and 6 have been described above.

The program subsequently inputs the settings of soils, such as clay, intermediate soil and sand with regard to the respective elements of the soil foundation (step S1110). Like the first embodiment, the settings of soil include material parameters and initial conditions. Like the first embodiment, Fig. 7 should be referred to as the exemplary file of the specific example to input the settings of soils. The details of the settings of analysis conditions shown in Fig. 7 have been described above.

The program then inputs the settings of a solid soil model (step S1120). Like the first embodiment, the settings of the solid soil model include conditions with regard to elements of the soil model. Like the first embodiment, Fig. 8 should be referred to as the exemplary file for inputting the settings of the solid soil model as the specific example shown in Figs. 3 and 4. The details of the settings of the solid soil model shown in Fig. 8 have been described above.

After the data input, the program performs computations using the input data (steps S1130 to S1270). The computation process of the second embodiment employ the Wilson's θ method for time integration, but another technique such as the Newmark's P method may be applied alternatively. The computation process of the second embodiment also employs the model like the Tamura's model or the Christian's model of the finite element method of allocating the pore water pressure, the pore air pressure and the degree of saturation to the element center for spatial discretization, but another technique such as the Sandhu's model of the finite element method of allocating the pore water pressure to the node or the mesh-free method may be applied alternatively. The following describes computations according to the Wilson's θ method and the Tamura's model of the finite element method. The program first estimates a "total position vector", a "total increment vector concerning velocity" and a "total increment vector concerning acceleration" as well as various conditions (for example, effective stress, pore water pressure, pore air pressure, degree of saturation and structure/overconsolidation/anisotropy) according to Equations (201) to (205) given below (step S1130). These predictive calculations are performed for the purpose of convergence in a shorter time period and are not essential. In the case of omission of such predictive calculations, the values eventually determined at a time t=t or the initial values at an initial time are set to the values at the time t= t+θΔt. Each set of vector quantities of all nodes as in the left side or in each term of the right side of Equation (201) is expressed by a column vector having components as shown in Equation (206). Each set of scalar quantities of all elements as in the left side or in each term of the right side of Equation (204) is expressed by a column vector having components as shown in Equation (207).

[Math. 74]

$$\left\{x^{N}\right\}\Big|_{t+\theta\Delta t} = \left\{x^{N}\right\}\Big|_{t} + \left\{v^{N}(\theta\Delta t)\right\}\Big|_{t} + \frac{1}{2}\left\{\dot{v}^{N}(\theta\Delta t)^{2}\right\}\Big|_{t} + \frac{1}{6}\left\{\ddot{v}^{N}(\theta\Delta t)^{3}\right\}\Big|_{t} \qquad (201)$$

$$\left\{v^{N}(\theta\Delta t)\right\}\Big|_{t+\theta\Delta t} = \left\{v^{N}(\theta\Delta t)\right\}\Big|_{t} + \left\{\dot{v}^{N}(\theta\Delta t)^{2}\right\}\Big|_{t} + \frac{1}{2}\left\{\ddot{v}^{N}(\theta\Delta t)^{3}\right\}\Big|_{t} \qquad (202)$$

$$\left\{\dot{v}^{N}(\theta\Delta t)^{2}\right\}\Big|_{t+\theta\Delta t} = \left\{\dot{v}^{N}(\theta\Delta t)^{2}\right\}\Big|_{t} + \left\{\ddot{v}^{N}(\theta\Delta t)^{3}\right\}\Big|_{t} \qquad (203)$$

$$\left\{p^{w}\right\}\Big|_{t+\theta\Delta t} = \left\{p^{w}\right\}\Big|_{t} + \left\{\dot{p}^{w}(\theta\Delta t)\right\}\Big|_{t} \qquad (204)$$

$$A\Big|_{t+\theta\Delta t} = A\Big|_{t} + \dot{A}\Big|_{t}(\theta\Delta t) \qquad (205)$$

$\{x^{N}\}|_{t}$, $\{x^{N}\}|_{t+\theta\Delta t}$ : column vector (total position vector) expressing set of position vectors of all nodes at time $t = t$ or at time $t = t + \theta\Delta t$

$\{v^{N}(\theta\Delta t)\}|_{t}$, $\{v^{N}(\theta\Delta t)\}|_{t+\theta\Delta t}$ : column vector (total increment vector concerning velocity) given as production of $\theta\Delta t$ and column vector $\{v^{N}\}$(total velocity vector) expressing set of velocity vectors of all nodes at time $t = t$ or at time $t = t + \theta\Delta t$

$\{\dot{v}N(\theta\Delta t)^{2}\}|_{t}$, $\{\dot{v}^{N}(\theta\Delta t)^{2}\}|_{t+\theta\Delta t}$ : column vector (total increment vector concerning acceleration) given as production of $(\theta\Delta t)^{2}$ and column vector $\{\dot{v}^{N}\}$ (total acceleration vector) expressing set of acceleration vectors of all nodes at time $t = t$ or at time $t = t + \theta\Delta t$

$\{\ddot{v}^{N}(\theta\Delta t)^{3}\}|_{t}$ : column vector (total increment vector concerning jerk) given as production of $(\theta\Delta t)^{3}$ and column vector $\{\ddot{v}^{N}\}$ (total jerk vector) expressing set of jerk vectors of all nodes at time $t=t$

$A$ : state quantity, for example, effective stress tensor $T'$, rotational hardening variable tensor $\beta$ (tensor quantity), surface force vector $t$ (vector quantity), pore water pressure $p^{w}$, pore air pressure $p^{a}$, degree of saturation $s^{w}$, degree of structure

$R*$, degree of overconsolidation $R$ (scalar quantity)

$A|_t$, $A|_{t+\theta\Delta t}$ : state quantity $A$ at time $t = t$ or at time $t = t + \theta\Delta t$

$\dot{A}|_t$ : rate of state quantity $A$ at time $t = t$

[Math. 75]

$$\{a^N\} = \begin{Bmatrix} a^1{}_1 \\ a^1{}_2 \\ a^1{}_r \\ \vdots \\ a^j{}_1 \\ a^j{}_2 \\ a^j{}_r \\ \vdots \\ a^{(NP)}{}_1 \\ a^{(NP)}{}_2 \\ a^{(NP)}{}_r \end{Bmatrix} \tag{206}$$

$a^j{}_r$ : $r$-direction component of $a^N$ at $j$-th node

$j$ : node number ($j = 1,2,\cdots$, $NP$, $NP$: total number of nodes)

$r$: number of elements per node ($r$= 2 for 2D plane strain condition and axisymmetric condition, $r$= 3 for 3D condition)

[Math. 76]

$$\{\alpha\} = \begin{Bmatrix} \alpha^1 \\ \alpha^2 \\ \vdots \\ \alpha^i \\ \vdots \\ \alpha^{(NE-1)} \\ \alpha^{(NE)} \end{Bmatrix} \tag{207}$$

$\alpha^i$ : value of $\alpha$ at $i$-th element

$i$ : element number ($i = 1,2,\cdots,NE$, $NE$ : total number of elements)

The program subsequently computes a global L matrix (global volume change rate matrix) according to Equation (208) given below as a matrix for converting the displacement rate of each node to the time rate of volume change of the soil skeleton (step S1140). The respective elements of the global L matrix are shown by Equation (209). The global L matrix is a matrix of NE rows and (NPxr) columns (r: the number of components per node). According to the mapping of the global node number to the local node number specified in setting the solid soil model, a value "0" is set to any column component having an m-th node number different from the global node number in an element L matrix for an element i shown in Equation (209) . A Bv matrix constituting the element L matrix is expressed by Equation (210) for the two-dimensional plane strain condition, by Equation (211) for the axisymmetric condition and by Equation (212) for the three-dimensional condition.

[Math. 77]

$$
L = \begin{bmatrix} L^{e1} \\ L^{e2} \\ \vdots \\ L^{ei} \\ \vdots \\ L^{e(NE-1)} \\ L^{e(NE)} \end{bmatrix} \quad : \text{global L matrix (global volume change rate matrix)} \tag{208}
$$

$$
L^{ei} = \begin{bmatrix} L^{i1}_1 & L^{i1}_2 & L^{i1}_r & \cdots & L^{im}_1 & L^{im}_2 & L^{im}_r & \cdots & L^{ip}_1 & L^{ip}_2 & L^{ip}_r \end{bmatrix} = \int_{ve^i} [B_v] dv \tag{209}
$$

: element L matrix for element $i$ (element volume change rate matrix)

$m$ : local node number in element ($m$= 1, 2, ..., $p$)
$p$ : number of nodes per element ($p$= 4 for 2D plane strain condition and axisymmetric condition, $p$ = 8 for 3D condition)
$ve^i$ : volume area occupied by element $i$

[Math. 78]

$$
[B_v] = \begin{bmatrix} N^1_{,1} & N^1_{,2} & \cdots & N^m_{,1} & N^m_{,2} & \cdots & N^p_{,1} & N^p_{,2} \end{bmatrix} \quad (p{=}4) \tag{210}
$$

$N^m$: shape function regarding $m$-th node of element $i$

$$
[B_v] = \begin{bmatrix} N^1_{,1} + \dfrac{N^1}{x^G_1} & N^1_{,2} & \cdots & N^m_{,1} + \dfrac{N^m}{x^G_1} & N^m_{,2} & \cdots & N^p_{,1} + \dfrac{N^p}{x^G_1} & N^p_{,2} \end{bmatrix} \quad (p{=}4) \tag{211}
$$

$x^G_i$ : distance in radial direction rotational axis to Gauss point.

$$
[B_v] = \begin{bmatrix} N^1_{,1} & N^1_{,2} & N^1_{,3} & \cdots & N^m_{,1} & N^m_{,2} & N^m_{,3} & \cdots & N^p_{,1} & N^p_{,2} & N^p_{,3} \end{bmatrix} \quad (p{=}8) \tag{212}
$$

The integrals like Equation (209) are performed according to the Gauss numeral integration method after conversion of a target analysis area in a global coordinate system into a local coordinate system as shown in Equation (213). Equation (213) herein shows volume integration for the three-dimensional condition. The integration is performed in the light of a unit thickness in a direction perpendicular to the target analysis plane for the two-dimensional plane strain condition, while being performed in the light of the rotational symmetry about the axis for the axisymmetric condition.
[Math. 79]

$$
\iiint f(x, y, z)\,dxdydz
$$
$$
= \sum_{i=1}^{NG} \sum_{j=1}^{NG} \sum_{k=1}^{NG} w_i w_j w_k f(x(\xi_i, \eta_j, \zeta_k), y(\xi_i, \eta_j, \zeta_k), z(\xi_i, \eta_j, \zeta_k)) J'(\xi_i, \eta_j, \zeta_k) \tag{213}
$$

$f = f(x,y,z)$ : integrand in integral area in o-xyz coordinate system (for 3D condition)
$J'$ : Jacobian determinant for conversion from o-xyz coordinate system into o-$\xi\eta\zeta$ local coordinate system having domain of [-1,1]
$NG$ : number of Gauss points
$\xi_i(i=1,\cdots,NG)$, $\eta_j(j=1,\cdots,NG)$, $\zeta_k(k=1,\cdots,NG)$ : coordinates of Gauss points on $\xi,\eta,\zeta$, axes
$w_i(i=1,\cdots,NG)$, $w_j(j=1,\cdots,NG)$, $w_k(k=1,\cdots,NG)$ : weights of respective Gauss points
The program subsequently computes a global $H_W$ matrix (global water permeability matrix) representing water permeability in the soil according to Equation (214) and a global $H_A$ matrix (global air permeability matrix) representing air permeability in the soil according to Equation (215) (step S1150). Both the global $H_W$ matrix and global $H_A$ matrix are matrices of NE rows and NE columns. According to the mapping of the internode number of the element to the global element number specified in setting the solid soil model, a value "0" is set to any component in the global $H_W$ matrix of Equation (214) and to any component in the global $H_A$ matrix of Equation (215) having an m-th internode number in an element $H_W$ matrix and in an element $H_A$ matrix for an element i different from the global element number. The last component in the element $H_W$ matrix for the element i of Equation (216) or the last component in the element $H_A$ matrix for the element i of Equation (217) is substituted into an i-th column component of the element i (i.e., i-th row). The respective components on the right side of Equation (216) are expressed by Equation (218). The respective components on the right side of Equation (217) are expressed by Equation (219). The hydraulic boundary condition, such as drain or undrain and the air boundary condition such as exhaust or unexhaust are respectively obtained by changing the coefficient of water permeability in Equation (218) and by changing the coefficient of air permeability in Equation (219). For example, in the case of the undrained condition, a value "0" is set to the coefficient of water permeability corresponding to an internode under this condition. In the case of the unexhausted condition, a value "0" is set to the coefficient of air permeability corresponding to an internode under this condition. With regard to the meanings of the respective symbols, like the first embodiment, refer to Fig. 9 for the two-dimensional plane strain condition, refer to Fig. 10 for the axisymmetric condition and refer to Fig. 11 for the three-dimensional condition.

[Math. 80]

$$
H_W = \begin{bmatrix} H_W{}^{e1} \\ H_W{}^{e2} \\ \vdots \\ H_W{}^{ei} \\ \vdots \\ H_W{}^{e(NE-1)} \\ H_W{}^{e(NE)} \end{bmatrix}
\tag{214}
$$

: global $H_W$ matrix (global water permeability matrix)

$$
H_A = \begin{bmatrix} H_A{}^{e1} \\ H_A{}^{e2} \\ \vdots \\ H_A{}^{ei} \\ \vdots \\ H_A{}^{e(NE-1)} \\ H_A{}^{e(NE)} \end{bmatrix}
\tag{215}
$$

: global $H_A$ matrix (global air permeability matrix)

$$
H_W{}^{ei} = \begin{bmatrix} \alpha_W{}^{ei}{}_1 & \cdots & \alpha_W{}^{ei}{}_m & \cdots & \alpha_W{}^{ei}{}_s & -\sum_{m=1}^{s} \alpha_W{}^{ei}{}_m \end{bmatrix}
\tag{216}
$$

: element $H_W$ matrix for element $i$ (element water permeability matrix)

$$
H_A{}^{ei} = \begin{bmatrix} \alpha_A{}^{ei}{}_1 & \cdots & \alpha_A{}^{ei}{}_m & \cdots & \alpha_A{}^{ei}{}_s & -\sum_{m=1}^{s} \alpha_A{}^{ei}{}_m \end{bmatrix}
\tag{217}
$$

: element $H_A$ matrix for element $i$ (element air permeability matrix)

$m$ : element plane number in element (for 3D condition) or element side number in element (for 2D plane strain condition or axisymmetric condition) ($m$ =1,2,···,$s$)

$s$ : number of planes per element *(s*= 6 for 3D condition) or number of sides per element (*s*= 4 for 2D plane strain condition and axisymmetric condition)

[Math. 81]

$$\alpha_W{}^{ei}{}_m = \frac{1}{\gamma_w} \cdot \frac{\dfrac{\rho^w{}_m \cdot k^w{}_m \cdot k^w{}_i}{l_{mm} \cdot l_{im}}}{\dfrac{\rho^w{}_m \cdot k^w{}_m}{l_{mm}} + \dfrac{\rho^w{}_i \cdot k^w{}_i}{l_{im}}} \cdot \frac{\boldsymbol{l}_m}{l_m} \cdot \boldsymbol{n}^m S^m \qquad \text{(no summation)} \qquad (218)$$

$$\alpha_A{}^{ei}{}_m = \frac{1}{\gamma_w} \cdot \frac{\dfrac{\rho^a{}_m \cdot k^a{}_m \cdot k^a{}_i}{l_{mm} \cdot l_{im}}}{\dfrac{\rho^a{}_m \cdot k^a{}_m}{l_{mm}} + \dfrac{\rho^a{}_i \cdot k^a{}_i}{l_{im}}} \cdot \frac{\boldsymbol{l}_m}{l_m} \cdot \boldsymbol{n}^m S^m \qquad \text{(no summation)} \qquad (219)$$

$m$ : element number of element adjacent to element $i$

$k^w{}_m$ : coefficient of water permeability of element $m$ at time $t = t + \theta\Delta t$, equal to 0 in the case of undrained boundary

$k^w{}_i$ : coefficient of water permeability of element $i$ at time $t = t + \theta\Delta t$

$k^a{}_m$ : coefficient of air permeability of *element $m$* at time $t = t + \theta\Delta t$, equal to 0 in the case of unexhausted boundary

$k^a{}_i$ : coefficient of air permeability of element $i$ at time $t = t + \theta\Delta t$

$\rho^w m$ : density of water of element $m$ at time $t = t + \theta\Delta t$

$\rho^w{}_i$ : density of water of element $i$ at time $t = t + \theta\Delta t$

$\rho^a{}_m$ : density of air of element $m$ at time $t = t + \theta\Delta t$

$\rho^a{}_i$ : density of air of element $i$ at time $t = t + \theta\Delta t$

$\gamma_w$ : unit volume weight of water at 4°C (standard unit volume weight)

$\boldsymbol{n}^m$ : <for 2D plane strain condition and axisymmetric condition>

outward (viewed from element $i$) unit normal vector perpendicular to boundary side between element $i$ and element $m$

<for 3D condition>

outward (viewed from element $i$) unit normal vector perpendicular to two diagonal lines on boundary surface between element $i$ and element $m$

$S^m$ : <for 2D plane strain condition>

distance between two nodes on boundary surface between element $i$ and element $m$

<for axisymmetric condition>

(distance between two nodes) on boundary surface between element $i$ and element $m$ $\times$

(distance $r'$ from axis of rotational symmetry to center of gravity on side)

<for 3D condition>

area of boundary surface between element $i$ and element $m$

$l_{im}$ : distance from center of gravity of element $i$ to center of gravity on boundary surface of element $m$

$l_{mm}$ : distance from center of gravity of element $m$ to center of gravity on boundary surface of element $m$

$l_m$ : distance from center or gravity of element $i$ to center of gravity of element $m$

$\boldsymbol{l}_m$ : relative position vector from center of gravity of element $i$ to center of gravity of element $m$

The program subsequently computes a global $L_{BW}$ matrix (global water flow rate conversion matrix) according to Equation (220) and a global $L_{BA}$ matrix (global air flow rate conversion matrix) according to Equation (225) (step S1160). The respective elements of the global $L_{BW}$ matrix are expressed by Equation (221), and the respective elements of the global $L_{BA}$ matrix are expressed by Equation (226). Both the global $L_{BW}$ matrix and the global $L_{BA}$ matrix are matrices of NE rows and (NPxr) columns. According to the mapping of the global node number to the local number specified in setting the solid soil mode, a value "0" is set to any column component having an n-th node number different from the global node number in an element $L_{BW}$ matrix for an element i for Equation (221) or in an element $L_{BA}$ matrix for the element i of Equation (226). An $L_B$ matrix constituting the element $L_{BW}$ matrix or the element $L_{BA}$ matrix is expressed by Equation (222). The respective components of the element $L_{BW}$ matrix are expressed by Equation (223), and a value $\beta_j{}^{wi}$ is given by Equation (224). The respective components of the element $L_{BA}$ matrix are expressed by Equation (227), and a value $B_j{}^{ai}$ is given by Equation (228). The hydraulic boundary condition, such as drain or undrain and the air boundary condition such as exhaust or unexhaust are respectively obtained by changing the coefficient of water permeability in Equation (224) and by changing the coefficient of air permeability in Equation (228). For example, in the case of the undrained condition, a value "0" is set to the coefficient of water permeability corresponding to an internode under this condition. In the case of the unexhausted condition, a value "0" is set to the coefficient of air permeability corresponding to an internode under this condition. With regard to the meanings of the respective symbols, like the first embodiment, refer to Fig. 9 for the two-dimensional plane strain condition, refer to Fig. 10 for the axisymmetric condition and refer to Fig. 11 for the three-dimensional condition.

[Math. 82]

$$
L_{BW} = \begin{bmatrix} L_{BW}{}^{e1} \\ L_{BW}{}^{e2} \\ \vdots \\ L_{BW}{}^{ei} \\ \vdots \\ L_{BW}{}^{e(NE-1)} \\ L_{BW}{}^{e(NE)} \end{bmatrix} \quad : \text{global } L_{BW} \text{ matrix (global water flow rate conversion matrix)} \tag{220}
$$

$$
L_{BW}{}^{ei} = \begin{bmatrix} L_{BW}{}^{i1}{}_1 & L_{BW}{}^{i1}{}_2 & L_{BW}{}^{i1}{}_r & \cdots & L_{BW}{}^{in}{}_1 & L_{BW}{}^{in}{}_2 & L_{BW}{}^{in}{}_r & \cdots & L_{BW}{}^{ip}{}_1 & L_{BW}{}^{ip}{}_2 & L_{BW}{}^{ip}{}_r \end{bmatrix} \tag{221}
$$

: element $L_{BW}$ matrix for element $i$ (element water flow rate conversion matrix)

$n$ : local node number in element ($n$ = 1,2,$\cdots$,$p$)
$p$ : number of nodes per element ($p$= 4 for 2D plane strain condition and axisymmetric condition, $p$= 8 for 3D condition)
$r$ : number of components per node ($r$= 2 for 2D plane strain condition and axisymmetric condition, $r$= 3 for 3D condition)

$$
L_B{}^l = \int_{S^l} \{n^l\}^T [N] dS^l \tag{222}
$$

$$
L_{BW}{}^{in}{}_r = \frac{k^w{}_i}{\gamma_w} \sum_{j=1}^s \left( \beta_j{}^{wi} L_B{}^j \right) \tag{223}
$$

$s$ : number of planes per element ($s$= 6 for 3D condition) or number of sides per element ($s$= 4 for 2D plane strain condition and axisymmetric condition)
$l$ : element plane number in element (for 3D condition) or element side number in element (for 2D plane strain condition or axisymmetric condition) ($l$ = 1,2,$\cdots$,$s$ )
$j$ : element plane number in element (for 3D condition) or element side number in element (for 2D plane strain condition or axisymmetric condition) ( $j$=1,2,$\cdots$,$s$ )
$k$ : element plane number in element (for 3D condition) or element side number in element (for 2D plane strain condition or axisymmetric condition) ( $k$ = 1,2,$\cdots$,$s$ )

$$
\beta_j{}^{wi} = \frac{\dfrac{\rho^w{}_m{}^2 \cdot k^w{}_m - \rho^w{}_i{}^2 \cdot k^w{}_i}{l_{im}}}{\dfrac{\rho^w{}_m \cdot k^w{}_m}{l_{mm}} + \dfrac{\rho^w{}_i \cdot k^w{}_i}{l_{im}}} \quad \text{(no summation)} \tag{224}
$$

$m$ : element number of element adjacent to element $i$
[Math. 83]

$$L_{BA} = \begin{bmatrix} L_{BA}{}^{e1} \\ L_{BA}{}^{e2} \\ \vdots \\ L_{BA}{}^{ei} \\ \vdots \\ L_{BA}{}^{e(NE-1)} \\ L_{BA}{}^{e(NE)} \end{bmatrix} \quad : \text{global } L_{BA} \text{ matrix (global air flow rate conversion matrix)} \tag{225}$$

$$L_{BA}{}^{ei} = \begin{bmatrix} L_{BA}{}^{i^1}{}_1 & L_{BA}{}^{iA^1}{}_2 & L_{BA}{}^{iA^1}{}_r & \cdots & L_{BA}{}^{i^n}{}_1 & L_{BA}{}^{i^n}{}_2 & L_{BA}{}^{i^n}{}_r & \cdots & L_{BA}{}^{i^p}{}_1 & L_{BA}{}^{i^p}{}_2 & L_{BA}{}^{i^p}{}_r \end{bmatrix} \tag{226}$$

: element $L_{BA}$ matrix for element $i$ (element air flow rate conversion matrix)

$n$ : local node number in element ( $n=1,2,\cdots,p$ )
$p$ : number of nodes per element ($p=4$ for 2D plane strain condition and axisymmetric condition, $p = 8$ for 3D condition)
$r$ : number of components per node ($r= 2$ for 2D plane strain condition and axisymmetric condition, $r= 3$ for 3D condition)

$$L_{BA}{}^{i^n}{}_r = \frac{k^a{}_i}{\gamma_w} \sum_{j=1}^{s} \left( \beta_j{}^{ai} L_B{}^j \right) \tag{227}$$

$s$ : number of planes per element ($s= 6$ for 3D condition) or number of sides per element ($s= 4$ for 2D plane strain condition and axisymmetric condition)
$j$ : element plane number in element (for 3D condition) or element side number in element (for 2D plane strain condition or axisymmetric condition) ( $j=1,2,\cdots,s$ )
$k$ : element plane number in element (for 3D condition) or element side number in element (for 2D plane strain condition or axisymmetric condition) ( $k =1,2,\cdots,s$ )

$$\beta_j{}^{ai} = \frac{\dfrac{\rho^a{}_m{}^2 \cdot k^a{}_m - \rho^a{}_i{}^2 \cdot k^a{}_i}{l_{im}}}{\dfrac{\rho^a{}_m \cdot k^a{}_m}{l_{mm}} + \dfrac{\rho^a{}_i \cdot k^a{}_i}{l_{im}}} \quad \text{(no summation)} \tag{228}$$

$m$ : element number of element adjacent to element $i$

The program subsequently calculates a total load increment vector, a total flow increment vector for pore water and a total flow increment vector for pore air at the time $t=t+\theta\Delta t$ respectively according to Equation (229), Equation (244) and Equation (246) given below (step S1170). A first term on the right side of Equation (229) is related to a nominal stress rate vector and is expressed by Equation (230). A second term on the right side of Equation (229) is related to the Green-Naghdi's rate and is expressed by Equation (231). An N matrix on the right side of Equation (230) and a B matrix on the right side of Equation (231) are expressed by Equations (232) and (233) for the two-dimensional plane strain condition, by Equations (234) and (235) for the axisymmetric condition and by Equations (236) and (237) for the three-dimensional condition. The term of the nominal stress rate vector in Equation (230) is rewritten as Equation (238) and is a sum of a surface traction rate vector externally given through a boundary defined by a first term on the right side and an increment of a surface traction vector due to a change in geometric configuration of the boundary defined by a second term on the right side. The surface traction rate vector at the time $t=t+\theta\Delta t$ of the first term on the right side is given by conversion of a surface traction rate vector set as a boundary condition at a time $t=t+\Delta t$ according to Equation (239) in conformity with the Wilson's $\theta$ method. An n matrix included in the second term on the right side of Equation (238) and related to an outward unit normal vector on a mechanical boundary of stress or stress rate is given by Equation (240) for the two-dimensional plane strain condition or the axisymmetric condition and by Equation (241) for the three-dimensional condition. A column vector including an effective stress component in a first term on the right side of Equation (231) is given by Equation (242) for the two-dimensional plane strain condition or the axisymmetric condition and by Equation (243) for the three-dimensional condition.

[Math. 84]

$$\left\{\dot{f}\left(\theta\Delta t\right)\right\}\Big|_{t+\theta\Delta t} = \sum_{j=1}^{NS}{}'\left\{\dot{f}^{\,j}\left(\theta\Delta t\right)\right\}\Big|_{t+\theta\Delta t} + \sum_{i=1}^{NE}{}''\left\{\dot{f}^{\,ei}\left(\theta\Delta t\right)\right\}\Big|_{t+\theta\Delta t} \quad : \text{ total load increment vector at time} \tag{229}$$

$$t = t + \theta\Delta t$$

$\displaystyle\sum_{j=1}^{NS}{}'$ : operation of adding value of node on *j*-th side (mechanical boundary of stress or stress rate) to value of corresponding global node, based on mapping of local node number to global node number *NS* : number of planes on mechanical boundary (for 3D condition) or number of sides on mechanical boundary (for 2D plane strain condition or axisymmetric condition)

$\displaystyle\sum_{i=1}^{NE}{}''$ : operation of adding value of each node in *i*-th element to value of corresponding global node, based on mapping of local node number to global node number

$$\left\{\dot{f}^{\,j}\left(\theta\Delta t\right)\right\}\Big|_{t+\theta\Delta t} = \int_{a}\left[N\right]^{\mathrm{T}}\left\{\dot{s}_{\iota}\right\}\Big|_{t+\theta\Delta t}\,\mathrm{d}a \times \left(\theta\Delta t\right) \tag{230}$$

$$\left\{\dot{f}^{\,ei}\left(\theta\Delta t\right)\right\}\Big|_{t+\theta\Delta t} = -\int_{v}\left[B\right]^{\mathrm{T}}\left\{T'_{\Omega}\right\}\Big|_{t+\theta\Delta t}\,\mathrm{d}v \times \left(\theta\Delta t\right) \tag{231}$$

[Math. 85]

$$\left[N\right] = \begin{bmatrix} N^{1} & 0 & \cdots & N^{m} & 0 & \cdots & N^{p} & 0 \\ 0 & N^{1} & \cdots & 0 & N^{m} & \cdots & 0 & N^{p} \end{bmatrix} \qquad (p=4) \tag{232}$$

$$\left[B\right] = \begin{bmatrix} N^{1}_{,1} & 0 & \cdots & N^{m}_{,1} & 0 & \cdots & N^{p}_{,1} & 0 \\ 0 & N^{1}_{,2} & \cdots & 0 & N^{m}_{,2} & \cdots & 0 & N^{p}_{,2} \\ N^{1}_{,2} & N^{1}_{,1} & \cdots & N^{m}_{,2} & N^{m}_{,1} & \cdots & N^{p}_{,2} & N^{p}_{,1} \end{bmatrix} \tag{233}$$

[Math. 86]

$$\left[N\right] = \begin{bmatrix} N^{1} & 0 & \cdots & N^{m} & 0 & \cdots & N^{p} & 0 \\ 0 & N^{1} & \cdots & 0 & N^{m} & \cdots & 0 & N^{p} \end{bmatrix} \qquad (p=4) \tag{234}$$

$$\left[B\right] = \begin{bmatrix} N^{1}_{,1} & 0 & \cdots & N^{m}_{,1} & 0 & \cdots & N^{p}_{,1} & 0 \\ 0 & N^{1}_{,2} & \cdots & 0 & N^{m}_{,2} & \cdots & 0 & N^{p}_{,2} \\ N^{1}_{,2} & N^{1}_{,1} & \cdots & N^{m}_{,2} & N^{m}_{,1} & \cdots & N^{p}_{,2} & N^{p}_{,1} \\ \dfrac{N^{1}}{x^{G}_{1}} & 0 & \cdots & \dfrac{N^{m}}{x^{G}_{1}} & 0 & \cdots & \dfrac{N^{p}}{x^{G}_{1}} & 0 \end{bmatrix} \tag{235}$$

[Math. 87]

$$\left[N\right] = \begin{bmatrix} N^{1} & 0 & 0 & \cdots & N^{m} & 0 & 0 & \cdots & N^{p} & 0 & 0 \\ 0 & N^{1} & 0 & \cdots & 0 & N^{m} & 0 & \cdots & 0 & N^{p} & 0 \\ 0 & 0 & N^{1} & \cdots & 0 & 0 & N^{m} & \cdots & 0 & 0 & N^{p} \end{bmatrix} \qquad (p=8) \tag{236}$$

$$[B] = \begin{bmatrix} N_{,1}^1 & 0 & 0 & \cdots & N_{,1}^m & 0 & 0 & \cdots & N_{,1}^p & 0 & 0 \\ 0 & N_{,2}^1 & 0 & \cdots & 0 & N_{,2}^m & 0 & \cdots & 0 & N_{,2}^p & 0 \\ 0 & 0 & N_{,3}^1 & \cdots & 0 & 0 & N_{,3}^m & \cdots & 0 & 0 & N_{,3}^p \\ N_{,2}^1 & N_{,1}^1 & 0 & \cdots & N_{,2}^m & N_{,1}^m & 0 & \cdots & N_{,2}^p & N_{,1}^p & 0 \\ 0 & N_{,3}^1 & N_{,2}^1 & \cdots & 0 & N_{,3}^m & N_{,2}^m & \cdots & 0 & N_{,3}^p & N_{,2}^p \\ N_{,3}^1 & 0 & N_{,1}^1 & \cdots & N_{,3}^m & 0 & N_{,1}^m & \cdots & N_{,3}^p & 0 & N_{,1}^p \end{bmatrix} \tag{237}$$

[Math. 88]

$$\{\dot{s}_\mathrm{t}\}\big|_{t+\theta\Delta t} \times (\theta\Delta t) = \{\dot{t}\}\big|_{t+\theta\Delta t} \times (\theta\Delta t) + \left([B_v] - [n]\big|_{t+\theta\Delta t}[B]\right)\{v^N(\theta\Delta t)\}\big|_{t+\theta\Delta t}\{t\}\big|_{t+\theta\Delta t} \tag{238}$$

$$\{t\}\big|_{t+\theta\Delta t} = \{t\}\big|_t + \theta\left(\{t\}\big|_{t+\Delta t} - \{t\}\big|_t\right) \tag{239}$$

$[\dot{s}_t]|_{t+\theta\Delta t}$ : nominal stress rate vector of $i$-th side specified as mechanical boundary at time $t = t + \theta\Delta t$

$\{t\}_{t+\theta\Delta t}, \{t\}|_{t+\theta\Delta t}$ : surface traction vector acting on i-th side specified as mechanical boundary at time $t = t + \theta\Delta t$ and its rate

$[n]_{t+\theta\Delta t}$ : matrix of respective components (direction cosine, $n_r$: r-direction component) of outward unit normal vector at i-th side as mechanical boundary at time $t = t + \theta\Delta t$

[Math. 89]

$$[n]_{t+\theta\Delta t} = \begin{bmatrix} n_1^2 & n_2^2 & n_1 n_2 \end{bmatrix} \tag{240}$$

$$[n]_{t+\theta\Delta t} = \begin{bmatrix} n_1^2 & n_2^2 & n_3^2 & n_1 n_2 & n_2 n_3 & n_3 n_1 \end{bmatrix} \tag{241}$$

[Math. 90]

$$\{T'_\Omega\}\big|_{t+\theta\Delta t} = \Omega_\mathrm{c}\begin{Bmatrix} -2T'_{12} \\ 2T'_{12} \\ T'_{11} - T'_{22} \end{Bmatrix} \tag{242}$$

$T'_{ij}$ : $i,j$ components ($i$= 1, 2, 3, $j$= 1, 2, 3) of effective stress tensor $T'$ at time $t = t + \theta\Delta t$ ($i$ =1,2,3, $j$ = 1,2,3)

$\Omega_c$ : (2,1) component of material spin tensor $\Omega = \boldsymbol{R R}^\mathrm{T}$ ($\boldsymbol{R}$ : rotation tensor) at time $t = t + \theta\Delta t$ for axisymmetric condition: value of 4th component= 0

[Math. 91]

$$\{T'_\Omega\}\big|_{t+\theta\Delta t} = \begin{Bmatrix} 2(\Omega_2 T'_{13} - \Omega_3 T'_{12}) \\ 2(\Omega_3 T'_{12} - \Omega_1 T'_{23}) \\ 2(\Omega_1 T'_{23} - \Omega_2 T'_{13}) \\ -\Omega_1 T'_{13} + \Omega_2 T'_{23} + \Omega_3 (T'_{11} - T'_{22}) \\ \Omega_1 (T'_{22} - T'_{33}) - \Omega_2 T'_{12} + \Omega_3 T'_{13} \\ \Omega_1 T'_{12} + \Omega_2 (T'_{33} - T'_{11}) - \Omega_3 T'_{23} \end{Bmatrix} \tag{243}$$

$\Omega_1, \Omega_2$ $\Omega_3$ : (3,2) component, (1,3) component and (2,1) component of material spin tensor $\Omega$ at time $t = t + \theta\Delta t$

The total flow increment vector for pore water at the time t=t+θΔt is expressed by Equation (244) and is obtained by multiplying a total flow rate vector expressed by Equation (245) by θΔt. A value $\zeta^\mathrm{ei}_\mathrm{m}$ as a component of the total flow rate vector is shown by Equation (246). The hydraulic boundary condition, such as drain or undrain is obtained by changing the coefficient of water permeability in Equation (246). For example, in the case of the undrained condition, a value "0" is set to the coefficient of water permeability corresponding to an internode under this condition. With regard to the meanings of the respective symbols, refer to Fig. 9 for the two-dimensional plane strain condition, refer to Fig. 10 for the axisymmetric condition and refer to Fig. 11 for the three-dimensional condition. The total flow increment vector for pore air at the time t=t+θΔt is expressed by Equation (247) and is obtained by multiplying a total flow rate vector for pore air expressed by Equation (248) by θΔt.

[Math. 92]

$$\left\{\dot{f}_{\mathrm{w}}\left(\theta\Delta t\right)\right\}\Big|_{t+\theta\Delta t} = \left\{\dot{f}_{\mathrm{w}}\right\}\Big|_{t+\theta\Delta t} \times \left(\theta\Delta t\right) \;:\; \text{total flow increment vector for pore water at time } \; t = t + \theta\Delta t \tag{244}$$

$$\left\{\dot{f}_{\mathrm{w}}\right\}\Big|_{t+\theta\Delta t} = \begin{pmatrix} -\sum_{m=1}^{s}\gamma_{\mathrm{W}}\alpha_{\mathrm{W}}{}^{\mathrm{e}1}{}_{m}\left(z_{cm}{}^{1}-z_{\mathrm{c}}{}^{1}\right)-k^{\mathrm{w}}{}_{1}\sum_{m=1}^{s}\zeta^{\mathrm{e}1}{}_{m} \\ -\sum_{m=1}^{s}\gamma_{\mathrm{W}}\alpha_{\mathrm{W}}{}^{\mathrm{e}2}{}_{m}\left(z_{cm}{}^{2}-z_{\mathrm{c}}{}^{2}\right)-k^{\mathrm{w}}{}_{2}\sum_{m=1}^{s}\zeta^{\mathrm{e}2}{}_{m} \\ \vdots \\ -\sum_{m=1}^{s}\gamma_{\mathrm{W}}\alpha_{\mathrm{W}}{}^{\mathrm{e}i}{}_{m}\left(z_{cm}{}^{i}-z_{\mathrm{c}}{}^{i}\right)-k^{\mathrm{w}}{}_{i}\sum_{m=1}^{s}\zeta^{\mathrm{e}i}{}_{m} \\ \vdots \\ -\sum_{m=1}^{s}\gamma_{\mathrm{W}}\alpha_{\mathrm{W}}{}^{\mathrm{e}(NE-1)}{}_{m}\left(z_{cm}{}^{(NE-1)}-z_{\mathrm{c}}{}^{(NE-1)}\right)-k^{\mathrm{w}}{}_{(NE-1)}\sum_{m=1}^{s}\zeta^{\mathrm{e}(NE-1)}{}_{m} \\ -\sum_{m=1}^{s}\gamma_{\mathrm{W}}\alpha_{\mathrm{W}}{}^{\mathrm{e}(NE)}{}_{m}\left(z_{cm}{}^{NE}-z_{\mathrm{c}}{}^{NE}\right)-k^{\mathrm{w}}{}_{(NE)}\sum_{m=1}^{s}\zeta^{\mathrm{e}(NE)}{}_{m} \end{pmatrix} \tag{245}$$

: total flow rate vector for pore water at time $\; t = t + \theta\Delta t$

$z_{\mathrm{c}}{}^{i}$ : vertical coordinate of center of gravity of element $i$ at time $t = t + \theta\Delta t$

$z_{cm}{}^{i}$ : vertical coordinate of center of gravity of element $m$ adjacent to element $i$ at time $t = t + \theta\Delta t$

s : number of planes per element *(s= 6 for 3D condition)* or number of sides per element (*s= 4 for 2D plane strain condition and axisymmetric condition)

$$\zeta^{\mathrm{e}i}{}_{m} = \left[\frac{1}{l_{im}}\cdot\frac{\left\{\rho^{\mathrm{w}}{}_{m}\cdot k^{\mathrm{w}}{}_{m}\left(\frac{\rho^{\mathrm{w}}{}_{m}}{\rho_{0}}-1\right)-\rho^{\mathrm{w}}{}_{i}\cdot k^{\mathrm{w}}{}_{i}\left(\frac{\rho^{\mathrm{w}}{}_{i}}{\rho_{0}}-1\right)\right\}}{\dfrac{\rho^{\mathrm{w}}{}_{m}\cdot k^{\mathrm{w}}{}_{m}}{l_{mm}}+\dfrac{\rho^{\mathrm{w}}{}_{i}\cdot k^{\mathrm{w}}{}_{i}}{l_{im}}}+\left(\frac{\rho^{\mathrm{w}}{}_{i}}{\rho_{0}}-1\right)\right]n^{m}{}_{iz}S^{m} \tag{246}$$

$\rho_{0}$ : density of water at 4°C (standard density)

$m$ : element number of element adjacent to element $i$

$n^{m}{}_{iz}$ : <for 2D plane strain condition and axisymmetric condition>

upward vertical component of outward unit normal vector (viewed from element $i$) perpendicular to boundary side between element $i$ and element $m$

<for 3D condition>

upward vertical component of outward unit normal vector (viewed from element $i$) perpendicular to two sets of diagonal lines on boundary surface between element $i$ and element $m$

[Math. 93]

$$\left\{\dot{f}_{\mathrm{a}}\left(\theta\Delta t\right)\right\}\Big|_{t+\theta\Delta t} = \left\{\dot{f}_{\mathrm{a}}\right\}\Big|_{t+\theta\Delta t} \times \left(\theta\Delta t\right) \;:\; \text{total flow increment vector for pore air at time } \; t = t + \theta\Delta t \tag{247}$$

$$\{\dot{f}_a\}_{t+\theta\Delta t} = \begin{pmatrix} -\sum_{m=1}^{s} \frac{k^a_1}{\rho_0}\left(\beta_m^{a1} + \rho^a_1\right)n^m_{1z}S^m \\ -\sum_{m=1}^{s} \frac{k^a_2}{\rho_0}\left(\beta_m^{a2} + \rho^a_2\right)n^m_{2z}S^m \\ \vdots \\ -\sum_{m=1}^{s} \frac{k^a_i}{\rho_0}\left(\beta_m^{ai} + \rho^a_i\right)n^m_{iz}S^m \\ \vdots \\ -\sum_{m=1}^{s} \frac{k^a_{(NE-1)}}{\rho_0}\left(\beta_m^{a(NE-1)} + \rho^a_{(NE-1)}\right)n^m_{(NE-1)z}S^m \\ -\sum_{m=1}^{s} \frac{k^a_{NE}}{\rho_0}\left(\beta_m^{a(NE)} + \rho^a_{(NE)}\right)n^m_{(NE)z}S^m \end{pmatrix} \tag{248}$$

: total flow rate vector for pore air at time $t = t + \theta\Delta t$

The program subsequently computes a global M matrix (global mass matrix) according to Equation (249) (step S1180). An element M matrix on the right side of Equation (249) is expressed by Equation (250). The global M matrix is a matrix of (NPxr) rows and (NPxr) columns.

[Math. 94]

$$M = \sum_{i=1}^{NE}{}' M^{ei} \quad : \text{global M matrix (global mass matrix)} \tag{249}$$

$\sum_{i=1}^{NE}{}'$ : operation of allocating local node numbers constituting matrix for each of elements from element number $i = 1$ to element number $i = NE$ to global node numbers and combining components from 0, based on mapping of local node number to global node number specified in setting solid soil model

$$M^{ei} = \int_v \rho_i [N]^T [N] dv \quad : \text{element M matrix (element mass matrix) for element } i \tag{250}$$

$\rho_i$ : density of element $i$ at time $t = t + \theta\Delta t$

The program then computes a global K matrix (global tangent stiffness matrix of soil skeleton) according to Equation (251) (step S1190). The global K matrix is a matrix of (NPxr) rows and (NPxr) columns. An element K matrix constituting the global K matrix is expressed by Equation (252). In Equation (252), a first term on the right side represents a tangent stiffness providing mechanical behavior of an identified soil material (for example, sand or clay). A second term on the right side represents a contribution of a time change of soil configuration to the tangent stiffness. A third term on the right side represents a contribution to the tangent stiffness under significant influence of the acceleration to the soil skeleton, for example, impact load. A fourth term on the right side represents a contribution to the tangent stiffness under application of a body force. With regard to the material parameters, providing relative differences to these "structural degradation index", "normal consolidation index" and "rotational hardening limit constant" ensures the continuity of processing from sand, intermediate soil to clay. A $D^{ep}$ matrix (elasto-plastic matrix) included in the first term of Equation (252) is given by Equation (253) in the elasto-plastic state (in the loading state). Additionally, Equation (253) is defined by Equations (254) and (255) for the two-dimensional plane strain condition, by Equations (256) and (257) for the axisymmetric condition and by Equations (258) and (259) for the three-dimensional condition. The $D^{eP}$ matrix is given by Equation (260) in the elastic state. The symbols used in Equations (254) to (259) are defined by Equations (261). The symbols used in Equation (261) are defined by using one or a plurality of Equations (262).

[Math. 95]

$$K = \sum_{i=1}^{NE}{}' K^{ei} \quad : \text{global K matrix (global tangent stiffness matrix) of soil skeleton} \tag{251}$$

$$\mathbf{K}^{ei} = \int_v [B]^T [D^{ep}][B]dv + \int_v [N']^T[T_1][N']dv$$

$$+ \int_v (\rho^w{}_i s^w{}_i + \rho^a{}_i s^a{}_i)[N]^T[N]\{\dot{v}^{Ni}\}\big|_{t+\theta\Delta t}[B_v]dv - \int_v (\rho^w{}_i s^w{}_i + \rho^a{}_i s^a{}_i)[N]^T\{b\}[B_v]dv \qquad (252)$$

: element K matrix (element tangent stiffness matrix) for element $i$

$\{\dot{v}^{Ni}\}\big|_{t+\theta\Delta t}$ : column vector expressing acceleration vectors of respective nodes in element $i$ at time $t = t + \theta\Delta t$
$\{b\}$ : body force vector per unit mass
$s^w{}_i$ : degree of saturation of element $i$ at time $t = t + \theta\Delta t$
$s^a{}_i$ : air void ratio of element $i$ at time $t = t + \theta\Delta t$
[Math. 96]

$$[D^{ep}] = [D_1{}^{ep}] - [D_2{}^{ep}] \quad : \text{elasto-plastic matrix} \qquad (253)$$

[Math. 97]

$$[D_1{}^{ep}] = \begin{bmatrix} a'+b' & a' & 0 \\ a' & a'+b' & 0 \\ 0 & 0 & b'/2 \end{bmatrix} \qquad (254)$$

$$[D_2{}^{ep}] = \frac{1}{e'} \begin{bmatrix} (c'\hat{\eta}_{11} - d')^2 & (c'\hat{\eta}_{11} - d')(c'\hat{\eta}_{22} - d') & c'\hat{\eta}_{12}(c'\hat{\eta}_{11} - d') \\ & (c'\hat{\eta}_{22} - d')^2 & c'\hat{\eta}_{12}(c'\hat{\eta}_{22} - d') \\ sym. & & (c'\hat{\eta}_{12})^2 \end{bmatrix} \qquad (255)$$

$\hat{\eta}_{ij}$ : $i,j$ components of tensor $\hat{\eta} = \eta - \beta$ ( $i$=1,2, $j$=1,2 )
[Math. 98]

$$[D_1{}^{ep}] = \begin{bmatrix} a'+b' & a' & 0 & a' \\ a' & a'+b' & 0 & a' \\ 0 & 0 & b'/2 & 0 \\ a' & a' & 0 & a'+b' \end{bmatrix} \qquad (256)$$

$$[D_2{}^{ep}] = \frac{1}{e'} \begin{bmatrix} (c'\hat{\eta}_{11} - d')^2 & (c'\hat{\eta}_{11} - d')(c'\hat{\eta}_{22} - d') & c'\hat{\eta}_{12}(c'\hat{\eta}_{11} - d') & (c'\hat{\eta}_{11} - d')(c'\hat{\eta}_{33} - d') \\ & (c'\hat{\eta}_{22} - d')^2 & c'\hat{\eta}_{12}(c'\hat{\eta}_{22} - d') & (c'\hat{\eta}_{22} - d')(c'\hat{\eta}_{33} - d') \\ & & (c'\hat{\eta}_{12})^2 & c'\hat{\eta}_{12}(c'\hat{\eta}_{33} - d') \\ sym. & & & (c'\hat{\eta}_{33} - d')^2 \end{bmatrix} \qquad (257)$$

$\hat{\eta}_{ij}$ : $i\,j$ components of tensor $\hat{\eta} = \eta - \beta$ ( $i$=1,2,3, $j$=1,2,3 )
[Math. 99]

$$[D_1{}^{\mathrm{ep}}] = \begin{bmatrix} a'+b' & a' & a' & & & \\ a' & a'+b' & a' & & 0 & \\ a' & a' & a'+b' & & & \\ & & & b'\!\big/\!2 & & \\ & 0 & & & b'\!\big/\!2 & \\ & & & & & b'\!\big/\!2 \end{bmatrix} \tag{258}$$

$$[D_2{}^{\mathrm{ep}}]$$

$$= \frac{1}{e'} \begin{bmatrix} (c'\hat{\eta}_{11}-d')^2 & (c'\hat{\eta}_{11}-d')(c'\hat{\eta}_{22}-d') & (c'\hat{\eta}_{11}-d')(c'\hat{\eta}_{33}-d') & c'\hat{\eta}_{12}(c'\hat{\eta}_{11}-d') & c'\hat{\eta}_{23}(c'\hat{\eta}_{11}-d') & c'\hat{\eta}_{31}(c'\hat{\eta}_{11}-d') \\ & (c'\hat{\eta}_{22}-d')^2 & (c'\hat{\eta}_{22}-d')(c'\hat{\eta}_{33}-d') & c'\hat{\eta}_{12}(c'\hat{\eta}_{22}-d') & c'\hat{\eta}_{23}(c'\hat{\eta}_{22}-d') & c'\hat{\eta}_{31}(c'\hat{\eta}_{22}-d') \\ & & (c'\hat{\eta}_{33}-d')^2 & c'\hat{\eta}_{12}(c'\hat{\eta}_{33}-d') & c'\hat{\eta}_{23}(c'\hat{\eta}_{33}-d') & c'\hat{\eta}_{31}(c'\hat{\eta}_{33}-d') \\ & \mathit{sym.} & & (c'\hat{\eta}_{12})^2 & (c'\hat{\eta}_{12})(c'\hat{\eta}_{23}) & (c'\hat{\eta}_{12})(c'\hat{\eta}_{31}) \\ & & & & (c'\hat{\eta}_{23})^2 & (c'\hat{\eta}_{23})(c'\hat{\eta}_{31}) \\ & & & & & (c'\hat{\eta}_{31})^2 \end{bmatrix}$$

$$\tag{259}$$

$\hat{\eta}_{ij}$ : $i,j$ components of tensor $\hat{\eta}=\eta=\beta$ ( $i$ =1,2,3, $J$=1,2,3 )

[Math. 100]

$$[D_2{}^{\mathrm{ep}}] = [0] \tag{260}$$

[Math. 101]

$$a' = \widetilde{K} - \frac{2}{3}\widetilde{G}$$

$$b' = 2\widetilde{G}$$

$$c' = 6\widetilde{G}$$

$$d' = \widetilde{K}\alpha$$

$$e' = 12\eta^{*2}\widetilde{G} + \widetilde{K}\alpha^2 + h$$

$$\eta(=q/p') = \sqrt{\frac{3}{2}}\|\eta\|\left(=\sqrt{\frac{3}{2}\eta\cdot\eta}\right) \ : \text{effective stress ratio}$$

$$\left.\begin{matrix}\\ \\ \\ \\ \\ \\ \\ \\ \\ \end{matrix}\right\} \tag{261}$$

$\beta$ : rotational hardening variable tensor (tensor defining direction and magnitude of anisotropy: The greater norm $\|\beta\|$ represents the higher degree of anisotropy)

[Math. 102]

$$\widetilde{K} = \frac{1+e}{\kappa} p' \left(= \frac{J(1+e_0)}{\kappa} p'\right)$$

$$\widetilde{G} = \frac{3(1-2v)}{2(1+v)} \widetilde{K}$$

$$\alpha = M_a^2 - \eta^2$$

$$h = Jp' \frac{M^2 + \eta^{*2}}{MD} (M_s^2 - \eta^2)$$

$$D = \frac{\lambda - \kappa}{M(1+e_0)} \quad : \text{dilatancy factor}$$

$$p' = -\frac{1}{3} \operatorname{tr} T' \quad : \text{mean skeleton stress}$$

$$\eta = T'/p' + I$$

$$\eta^* = \sqrt{\frac{3}{2}} \|\hat{\eta}\|$$

(262)

$$M_a^2 = M^2 + \frac{3}{2} \beta \cdot \beta$$

(263)

: slope of threshold line between plastic compression and plastic expansion in $p'\sim q$ stress space

$$M_s^2 = M_a^2 + b_r \frac{4M\eta^{*2}}{M^2 + \eta^{*2}} \left( m_b \eta^* - \sqrt{\frac{3}{2}} \hat{\eta} \cdot \beta \right) - MD \left( \frac{U^*}{R^*} - \frac{U}{R} \right) \sqrt{6\eta^{*2} + \frac{1}{3} \alpha^2}$$

(264)

: slope of threshold line between hardening and softening in $p' \sim q$ stress space

e : void ratio at time $t = t + \theta \Delta t$

$e_0$ : void ratio at initial time $t = 0$

$J$ : determinant of deformation gradient tensor of soil skeleton (giving infinitesimal volume ratio of soil skeleton at time $t = t$ relative to initial time $t = 0$ )

$J = 1$ for small deformation analysis

$\lambda$ : compression index

$\kappa$ : swelling index

M : critical state constant

$v$ : Poisson's ratio

I : unit tensor

$q = \eta \times p'$ : shear stress

Equations (263) and (264) respectively give the slope of a threshold line between plastic compression and plastic expansion and the slope of a threshold line between hardening and softening in the p'-q stress space. Equations (263) and (264) show the case where a plastic stretching norm is employed as the plasticity measure as shown in Equations (265) and (266) given below in the evolution rule which specifies a process of lowering the degree of structure R* (0< R≤ 1, the value of R* closer to 0 represents the more highly structured) and a process of losing the degree of overconsolidation R (0< R≤ 1, the value of R closer to 0 represents the higher degree of overconsolidation) in the soil skeleton structure. A shear component of the plastic stretching or a plastic power given as the inner product of the effective stress tensor and the plastic stretching may also be employed as the plasticity measure according to the type of soil. In small deformation analysis, the plastic stretching is specifiable by a plastic strain rate. Symbols "U" in Equation (265) and "U*" in Equation (266) are nonnegative functions and are given by, for example, Equations (267) and (268). Equation (269) given below is used for the evolution rule of the rotational hardening variable tensor representing the anisotropy.

[Math. 103]

$$\dot{R}^* = JU^*\|\boldsymbol{D}^{\mathrm{p}}\| = JU^* \sqrt{6\eta^{*2} + \frac{1}{3}\alpha^2}\, \frac{6\widetilde{G}\hat{\boldsymbol{\eta}}\cdot\boldsymbol{D} - \widetilde{K}\alpha(\mathrm{tr}\boldsymbol{D})}{12\eta^{*2}\widetilde{G} + \widetilde{K}\alpha^2 + h} \tag{265}$$

$$\dot{R} = JU\|\boldsymbol{D}^{\mathrm{p}}\| = JU \sqrt{6\eta^{*2} + \frac{1}{3}\alpha^2}\, \frac{6\widetilde{G}\hat{\boldsymbol{\eta}}\cdot\boldsymbol{D} - \widetilde{K}\alpha(\mathrm{tr}\boldsymbol{D})}{12\eta^{*2}\widetilde{G} + \widetilde{K}\alpha^2 + h} \tag{266}$$

$\dot{R}^*$, $\dot{R}$ : rates of $R^*$ and R (material time derivative)
$\boldsymbol{D}^P$ : plastic stretching tensor
$D$ : stretching tensor

$$U^* = \frac{a}{\mathrm{D}}R^{*b}\left(1 - R^*\right)^c \tag{267}$$

$$U = -\frac{m}{\mathrm{D}}\ln R \tag{268}$$

*a, b* and *c* : structural degradation indexes (material parameters for controlling the likelihood of degrading the structure)
*m* : normal consolidation index (material parameters for controlling the likelihood of loss of overconsolidation or the likelihood of normal consolidation)

[Math. 104]

$$\overset{\circ}{\boldsymbol{\beta}}(= \dot{\boldsymbol{\beta}} + \boldsymbol{\beta}\boldsymbol{\Omega} - \boldsymbol{\Omega}\boldsymbol{\beta}) = J\frac{b_r}{\mathrm{D}}\sqrt{\frac{2}{3}}\|\boldsymbol{D}_s{}^p\|\|\hat{\boldsymbol{\eta}}\|\left(m_b\frac{\hat{\boldsymbol{\eta}}}{\|\hat{\boldsymbol{\eta}}\|} - \boldsymbol{\beta}\right) = J\frac{b_r}{\mathrm{D}}2\eta^{*2}\frac{6\widetilde{G}\hat{\boldsymbol{\eta}}\cdot\boldsymbol{D} - \widetilde{K}\alpha(\mathrm{tr}\boldsymbol{D})}{12\eta^{*2}\widetilde{G} + \widetilde{K}\alpha^2 + h}\|\hat{\boldsymbol{\eta}}\|\left(m_b\frac{\hat{\boldsymbol{\eta}}}{\|\hat{\boldsymbol{\eta}}\|} - \boldsymbol{\beta}\right) \tag{269}$$

$\overset{\circ}{\beta}$ : Green-Nagdhi's rate of β
$\dot{\beta}$ : rate of β
$b_r$ : rotational hardening index (material parameter for controlling the likelihood of evolution of anisotropy β)
$m_b$ : rotational hardening limit constant (material parameter representing evolution limit of anisotropy)

An N' matrix and a T1 matrix of a second term on the right side of Equation (252) are given by Equations (270) and (271) for the two-dimensional plane strain condition, by Equations (272) and (273) for the axisymmetric condition and by Equations (274) and (275) for the three-dimensional condition.

[Math. 105]

$$\left[N'\right] = \begin{bmatrix} N_{,1}^1 & 0 & \cdots & N_{,1}^m & 0 & \cdots & N_{,1}^p & 0 \\ 0 & N_{,2}^1 & \cdots & 0 & N_{,2}^m & \cdots & 0 & N_{,2}^p \\ N_{,2}^1 & 0 & \cdots & N_{,2}^m & 0 & \cdots & N_{,2}^p & 0 \\ 0 & N_{,1}^1 & \cdots & 0 & N_{,1}^m & \cdots & 0 & N_{,1}^p \end{bmatrix} \tag{270}$$

$$[T_1] = \begin{bmatrix} 0 & T_{11} & -T_{12} & 0 \\ T_{22} & 0 & 0 & -T_{12} \\ T_{12} & 0 & 0 & -T_{11} \\ 0 & T_{12} & -T_{22} & 0 \end{bmatrix} \qquad (271)$$

$T_{ij}$ : $i,j$ components ($i$= 1,2, $j$= 1,2) of total stress tensor $\boldsymbol{T}(=\boldsymbol{T'}-(s^w p^w + s^a p^a)\boldsymbol{I})$ at time $t = t + \theta\Delta t$

[Math. 106]

$$[N'] = \begin{bmatrix} N_{,1}^1 & 0 & \cdots & N_{,1}^m & 0 & \cdots & N_{,1}^p & 0 \\ 0 & N_{,2}^1 & \cdots & 0 & N_{,2}^m & \cdots & 0 & N_{,2}^p \\ N_{,2}^1 & 0 & \cdots & N_{,2}^m & 0 & \cdots & N_{,2}^p & 0 \\ 0 & N_{,1}^1 & \cdots & 0 & N_{,1}^m & \cdots & 0 & N_{,1}^p \\ \dfrac{N^1}{x^G_1} & 0 & \cdots & \dfrac{N^m}{x^G_1} & 0 & \cdots & \dfrac{N^p}{x^G_1} & 0 \end{bmatrix} \qquad (272)$$

$$[T_1] = \begin{bmatrix} 0 & T_{11} & -T_{12} & 0 & T_{11} \\ T_{22} & 0 & 0 & -T_{12} & T_{22} \\ T_{12} & 0 & 0 & -T_{11} & T_{12} \\ 0 & T_{12} & -T_{22} & 0 & T_{12} \\ T_{33} & T_{33} & 0 & 0 & 0 \end{bmatrix} \qquad (273)$$

$T_{ij}$ : $i,j$ components ($i$= 1,2, 3, $j$= 1,2,3) of total stress tensor $T$ at time $t = t + \theta\Delta t$

[Math. 107]

$$[N'] = \begin{bmatrix} N_{,1}^1 & 0 & 0 & \cdots & N_{,1}^m & 0 & 0 & \cdots & N_{,1}^p & 0 & 0 \\ 0 & N_{,2}^1 & 0 & \cdots & 0 & N_{,2}^m & 0 & \cdots & 0 & N_{,2}^p & 0 \\ 0 & 0 & N_{,3}^1 & \cdots & 0 & 0 & N_{,3}^m & \cdots & 0 & 0 & N_{,3}^p \\ N_{,2}^1 & 0 & 0 & \cdots & N_{,2}^m & 0 & 0 & \cdots & N_{,2}^p & 0 & 0 \\ 0 & N_{,1}^1 & 0 & \cdots & 0 & N_{,1}^m & 0 & \cdots & 0 & N_{,1}^p & 0 \\ 0 & N_{,3}^1 & 0 & \cdots & 0 & N_{,3}^m & 0 & \cdots & 0 & N_{,3}^p & 0 \\ 0 & 0 & N_{,2}^1 & \cdots & 0 & 0 & N_{,2}^m & \cdots & 0 & 0 & N_{,2}^p \\ 0 & 0 & N_{,1}^1 & \cdots & 0 & 0 & N_{,1}^m & \cdots & 0 & 0 & N_{,1}^p \\ N_{,3}^1 & 0 & 0 & \cdots & N_{,3}^m & 0 & 0 & \cdots & N_{,3}^p & 0 & 0 \end{bmatrix} \qquad (274)$$

$$[T_1] = \begin{bmatrix} 0 & T_{11} & T_{11} & -T_{12} & 0 & 0 & 0 & 0 & -T_{13} \\ T_{22} & 0 & T_{22} & 0 & -T_{12} & -T_{23} & 0 & 0 & 0 \\ T_{33} & T_{33} & 0 & 0 & 0 & 0 & -T_{23} & -T_{13} & 0 \\ T_{12} & 0 & T_{12} & 0 & -T_{11} & -T_{13} & 0 & 0 & 0 \\ 0 & T_{12} & T_{12} & -T_{22} & 0 & 0 & 0 & 0 & -T_{23} \\ T_{23} & T_{23} & 0 & 0 & 0 & 0 & -T_{22} & -T_{12} & 0 \\ T_{23} & 0 & T_{23} & 0 & -T_{13} & -T_{33} & 0 & 0 & 0 \\ 0 & T_{31} & T_{31} & -T_{23} & 0 & 0 & 0 & 0 & -T_{33} \\ T_{31} & T_{31} & 0 & 0 & 0 & 0 & -T_{12} & -T_{11} & 0 \end{bmatrix} \qquad (275)$$

$T_{ij}$ : $i,j$ components ($i$= 1,2, 3, $j$= 1,2,3) of total stress tensor $T$ at time $t = t + \theta\Delta t$

The program subsequently computes a global $L_W^T$ matrix (global pore water pressure-related load change rate conversion matrix), a global $L_A{}^T$ matrix (global pore air pressure-related load change rate conversion matrix) and a global $L_S{}^T$ matrix (global saturation degree-related load change rate conversion matrix) respectively according to Equation (276), Equation (278) and Equation (280) (step S1200). An element $L_W{}^T$ matrix on the right side of Equation (276) is expressed by Equation (277), an element $L_A{}^T$ matrix on the right side of Equation (278) is expressed by Equation (279), and an element $L_S{}^T$ matrix on the right side of Equation (280) is expressed by Equation (281). All the global $L_W{}^T$ matrix, the global $L_A{}^T$ matrix and the global $L_S{}^T$ matrix are matrices of (NPxr) rows and NE columns.

[Math. 108]

$$L_W{}^T = \sum_{i=1}^{NE} {}^{\prime} L_W{}^{ei\,T} \tag{276}$$

: global $L_W{}^T$ matrix (global pore water pressure-related load change rate conversion matrix) of

soil skeleton

$$L_W{}^{ei\,T} = \int_v s^w{}_i [B_v]^T \, dv - \int_v \frac{n_i s^w{}_i \rho^w{}_i}{K_w} [N]^T \left( [N]\{\dot{v}^{Ni}\}\big|_{t+\theta\Delta t} - \{b\} \right) dv \tag{277}$$

: element $L_W{}^T$ matrix (element pore water pressure-related load change rate conversion matrix)

of element $i$

$n_i$ : porosity of element $i$ at time $t = t + \theta\Delta t$
$K_w$ : volume modulus of water

[Math. 109]

$$L_A{}^T = \sum_{i=1}^{NE} {}^{\prime} L_A{}^{ei\,T} \tag{278}$$

: global $L_A{}^T$ matrix (global pore air pressure-related load change rate conversion matrix) of soil

skeleton

$$L_A{}^{ei\,T} = \int_v s^a{}_i [B_v]^T \, dv - \int_v \frac{n_i s^a{}_i}{R\Theta_i} [N]^T \left( [N]\{\dot{v}^{Ni}\}\big|_{t+\theta\Delta t} - \{b\} \right) dv \tag{279}$$

: element $L_A{}^T$ matrix (element pore air pressure-related load change rate conversion matrix) of

element $i$

R : gas constant of the air
$\Theta_i$ : absolute temperature of air for element $i$

[Math. 110]

$$L_S{}^T = \sum_{i=1}^{NE} {}^{\prime} L_S{}^{ei\,T} \tag{280}$$

: global $L_A{}^T$ matrix (global saturation degree-related load change rate conversion matrix) of soil

skeleton

$$\mathbf{L_S}^{ei\,\mathrm{T}} = -\int_v \left( p^{\mathrm{a}}_i - p^{\mathrm{w}}_i \right) \left[ B_v \right]^{\mathrm{T}} \mathrm{d}v - \int_v n_i \left( \rho^{\mathrm{w}}_i - \rho^{\mathrm{a}}_i \right) \left[ N \right]^{\mathrm{T}} \left( \left[ N \right] \{ \dot{v}^{Ni} \} \big|_{t+\theta\Delta t} - \{ b \} \right) \mathrm{d}v$$

$$(281)$$

: element $\mathbf{L_S}^{\mathrm{T}}$ matrix (element saturation degree-related load change rate conversion matrix) of

element $i$

$p^{\mathrm{a}}_i$ : pore air pressure of element $i$ at time $t = t + \theta\Delta t$

$p^{\mathrm{w}}_i$ : pore water pressure of element $i$ at time $t = t + \theta\Delta t$

The program subsequently computes a global $S_{WC}$ matrix (global water compressibility matrix), a global $S_{AC}$ matrix (global air compressibility matrix) and a global $S_{SW}$ matrix (global saturation degree change rate matrix) respectively according to Equation (282), Equation (283) and Equation (284). The program also computes a global $S_C$ matrix (global suction-contributing moisture characteristic matrix) and a global $S_W$ matrix (global saturation degree-contributing moisture characteristic matrix) respectively according to Equation (285) and (286). For such computation, the van Genuchten's equation is used. All these matrices are matrices of NE rows and NE columns (step S1210).

[Math. 111]

$$S_{WC} = \begin{bmatrix} wc^{\mathrm{e}1} & & & & & \\ & wc^{\mathrm{e}2} & & & 0 & \\ & & \ddots & & & \\ & & & wc^{\mathrm{e}i} & & \\ & & & & \ddots & \\ & 0 & & & wc^{\mathrm{e}(NE-1)} & \\ & & & & & wc^{\mathrm{e}(NE)} \end{bmatrix}$$

$$(282)$$

: $S_{WC}$ matrix (global water compressibility matrix)

$$wc^{ei} = \int_{v^{ei}} \frac{n_i s^{\mathrm{w}}_i}{\mathrm{K_w}} \mathrm{d}v$$

$$S_{AC} = \begin{bmatrix} ac^{\mathrm{e}1} & & & & & \\ & ac^{\mathrm{e}2} & & & 0 & \\ & & \ddots & & & \\ & & & ac^{\mathrm{e}i} & & \\ & & & & \ddots & \\ & 0 & & & ac^{\mathrm{e}(NE-1)} & \\ & & & & & ac^{\mathrm{e}(NE)} \end{bmatrix}$$

$$(283)$$

: $S_{AC}$ matrix (global air compressibility matrix)

$$ac^{ei} = \int_{v^{ei}} \frac{n_i s^{\mathrm{a}}_i}{\rho^{\mathrm{a}}_i \overline{\overline{R}} \Theta_i} \mathrm{d}v$$

$$S_{SW} = \begin{bmatrix} ssw^{e1} & & & & & \\ & ssw^{e2} & & & 0 & \\ & & \ddots & & & \\ & & & ssw^{ei} & & \\ & & & & \ddots & \\ & 0 & & & ssw^{e(NE-1)} & \\ & & & & & ssw^{e(NE)} \end{bmatrix}$$

(284)

: $S_{SW}$ matrix (global saturation degree change rate matrix)

$$ssw^{ei} = \int_{ve^i} n_i \mathrm{d}v$$

[Math. 112]

$$S_C = \begin{bmatrix} sc^{e1} & & & & & \\ & sc^{e2} & & & 0 & \\ & & \ddots & & & \\ & & & sc^{ei} & & \\ & & & & \ddots & \\ & 0 & & & sc^{e(NE-1)} & \\ & & & & & sc^{e(NE)} \end{bmatrix}$$

: $S_C$ matrix (global suction-contributing moisture characteristic matrix)

$$sc^{ei} = \mathrm{m}^{v}{}_i \mathrm{n}^{v}{}_i \alpha^{v}{}_i \left(\alpha^{v}{}_i p^{s}{}_i\right)^{\mathrm{n}^{v}{}_i - 1} \left(s^{w}{}_{\max_i} - s^{w}{}_{\min_i}\right)$$

(285)

$\mathrm{m}^{v}{}_i$ : moisture characteristic parameter m in van Genuchten's equation of material of element $i$
$\mathrm{n}^{v}{}_i$ : moisture characteristic parameter n in van Genuchten's equation of material of element $i$
$\alpha^{v}{}_i$ : moisture characteristic parameter $\alpha$ in van Genuchten's equation of material of element $i$
$p^{s}{}_i$ : suction (difference between air pressure and water pressure) of element $i$ at time $t = t + \theta\Delta t$
$s^{w}{}_{\max_i}$ : maximum degree of saturation of material of element i
$s^{w}{}_{\min_i}$ : residual degree of saturation of material of element i

$$S_W = \begin{bmatrix} sw^{e1} & & & & & \\ & sw^{e2} & & & 0 & \\ & & \ddots & & & \\ & & & sw^{ei} & & \\ & & & & \ddots & \\ & 0 & & & sw^{e(NE-1)} & \\ & & & & & sw^{e(NE)} \end{bmatrix}$$

(286)

: $S_W$ matrix (global saturation degree-contributing moisture characteristic matrix)

$$sw^{ei} = -\left\{1 + \left(\alpha^{v}{}_i p^{s}{}_i\right)^{\mathrm{n}^{v}{}_i}\right\}^{\mathrm{m}^{v}{}_i + 1}$$

The program then computes a modified global water permeability original L matrix, a modified global air permeability original L matrix, a modified global saturation degree original L matrix, a modified global air void ratio original L matrix, a modified global pore water original L matrix and a modified global pore air original L matrix respectively according to Equation (287), Equation (288), Equation (289), Equation (290), Equation (291) and Equation (292) (step S1220). These matrices are matrices of NE rows and (NP×r) columns produced like the global L matrices. The modified global water permeability original L matrix and the modified global air permeability original L matrix are, however, respectively included in the modified global pore water original L matrix and the modified global pore air original L matrix in the process of establishment of global tangent stiffness equations (simultaneous linear equations), so that it is not required to actually calculate the modified global water permeability original L matrix and the modified global air permeability original L matrix.

[Math. 113]

$$
L_{W1} = \frac{1}{g}
\begin{bmatrix}
k^{w}_{1} \, L^{e1} \\
k^{w}_{2} \, L^{e2} \\
\vdots \\
k^{w}_{i} \, L^{ei} \\
\vdots \\
k^{w}_{(NE-1)} \, L^{e(NE-1)} \\
k^{w}_{(NE)} \, L^{e(NE)}
\end{bmatrix}
\tag{287}
$$

: modified global water permeability original L matrix (modified global water permeability original volume change rate matrix)

$$
L_{A1} = \frac{1}{g}
\begin{bmatrix}
k^{a}_{1} \, L^{e1} \\
k^{a}_{2} \, L^{e2} \\
\vdots \\
k^{a}_{i} \, L^{ei} \\
\vdots \\
k^{a}_{(NE-1)} \, L^{e(NE-1)} \\
k^{a}_{(NE)} \, L^{e(NE)}
\end{bmatrix}
\tag{288}
$$

: modified global air permeability original L matrix (modified global air permeability original volume change rate matrix)

$$
L_{W2} =
\begin{bmatrix}
s^{w}_{1} L^{e1} \\
s^{w}_{2} \, L^{e2} \\
\vdots \\
s^{w}_{i} \, L^{ei} \\
\vdots \\
s^{w}_{(NE-1)} \, L^{e(NE-1)} \\
s^{w}_{(NE)} \, L^{e(NE)}
\end{bmatrix}
\tag{289}
$$

: modified global saturation degree original L matrix (modified global saturation degree original volume change rate matrix)

$$L_{A2} = \begin{bmatrix} s^a_1 L^{e1} \\ s^a_2 L^{e2} \\ \vdots \\ s^a_i L^{ei} \\ \vdots \\ s^a_{(NE-1)} L^{e(NE-1)} \\ s^a_{(NE)} L^{e(NE)} \end{bmatrix} \tag{290}$$

: modified global air void ratio original L matrix (modified global air void ratio original volume

change rate matrix)

[Math. 114]

$$L_{\theta n}^{W} = \begin{bmatrix} \gamma_{\theta n W}^{1} L^{e1} \\ \gamma_{\theta n W}^{2} L^{e2} \\ \vdots \\ \gamma_{\theta n W}^{i} L^{ei} \\ \vdots \\ \gamma_{\theta n W}^{(NE-1)} L^{e(NE-1)} \\ \gamma_{\theta n W}^{(NE)} L^{e(NE)} \end{bmatrix} \qquad (n = 1,2,3) \tag{291}$$

: modified global pore water original L matrix (modified global pore water original volume change rate matrix)

$$\gamma_{\theta 1 W}^{i} = -\frac{1}{2\theta\Delta t}\frac{\rho^{W}_i}{\rho_0}\frac{k^{W}_i}{g} + \frac{1}{6}s^{W}_i \qquad ,$$

$$\gamma_{\theta 2 W}^{i} = s^{W}_i - \frac{1}{\theta\Delta t}\frac{\rho^{W}_i}{\rho_0}\frac{k^{W}_i}{g} \qquad ,$$

$$\gamma_{\theta 3 W}^{i} = \frac{1}{6}\left(2s^{W}_i - \frac{3}{\theta\Delta t}\frac{\rho^{W}_i}{\rho_0}\frac{k^{W}_i}{g}\right)$$

$$L_{\theta n}^{A} = \begin{bmatrix} \gamma_{\theta n A}^{1} L^{e1} \\ \gamma_{\theta n A}^{2} L^{e2} \\ \vdots \\ \gamma_{\theta n A}^{i} L^{ei} \\ \vdots \\ \gamma_{\theta n A}^{(NE-1)} L^{e(NE-1)} \\ \gamma_{\theta n A}^{(NE)} L^{e(NE)} \end{bmatrix} \qquad (n = 1,2,3) \tag{292}$$

: modified global pore air original L matrix (modified global pore air original volume change rate matrix)

$$\gamma_{\theta 1 A}{}^{i} = -\frac{1}{2\theta\Delta t} \frac{\rho^{a}{}_{i}}{\rho_{0}} \frac{k^{a}{}_{i}}{g} + \frac{1}{6} s^{a}{}_{i} \qquad ,$$

$$\gamma_{\theta 2 A}{}^{i} = s^{a}{}_{i} - \frac{1}{\theta\Delta t} \frac{\rho^{a}{}_{i}}{\rho_{0}} \frac{k^{a}{}_{i}}{g} \qquad ,$$

$$\gamma_{\theta 3 A}{}^{i} = \frac{1}{6}\left( 2 s^{a}{}_{i} - \frac{3}{\theta\Delta t} \frac{\rho^{a}{}_{i}}{\rho_{0}} \frac{k^{a}{}_{i}}{g} \right)$$

The program subsequently determines the solutions of unknown "jerk field", pore water pressure field, pore air pressure field and saturation degree field according to global tangent stiffness equations (simultaneous linear equations) shown by Equation (293) given below under geometric boundary condition with regard to the displacement, the displacement rate or the acceleration, mechanical boundary condition with regard to the stress or the stress rate, hydraulic boundary condition with regard to the pore water pressure or the total water head and the flow rate of pore water and air boundary condition with regard to the pore air pressure or the flow rate of pore air (step S1230). This Equation (297) is given by employing an implicit method (finite difference method) to compute a time derivative term of simultaneous second-order ordinal differential equations (Equation (294)) which is eventually obtained by finite element discretization of the weak form of the rate-type equation of motion and modeling of the soil-water-air coupled equations in the light of the influence of an inertial term. Equation (293) is the simultaneous linear equations to be solved (eventually) with application of the Wilson's θ method to the deformation field and the trapezoidal rule to the pore water pressure, the pore air pressure and the degree of saturation. The total load increment vector, the total flow increment vector of pore water and the total flow increment vector of pore air, as well as the global M matrix, the global K matrix, the global $H_{W}$ matrix, the global $H_{A}$ matrix, the global $L_{BW}$ matrix, the global $L_{BA}$ matrix, the global $L_{W}{}^{T}$ matrix, the global $L_{A}{}^{T}$ matrix, the global $L_{S}{}^{T}$ matrix, the global $S_{WC}$ matrix, the global $S_{AC}$ matrix, the global $S_{SW}$ matrix, the global $S_{C}$ matrix, the global $S_{W}$ matrix, the global $L_{W2}$ matrix, the global $L_{A2}$ matrix, the global $L_{\theta n}{}^{W}$ matrix and the global $L_{\theta n}{}^{A}$ matrix are updated through iterative computations in a time step between the time t=t and the time t=t+θΔt. Equation (294) further processes the equation of motion by material time derivation of the soil skeleton and is accordingly characterized by introduction of a second-order derivative term of a velocity vector as an explanatory variable with respect to time, i.e., a "jerk" term. This characteristic gives a rate-type constitutive equation of the soil skeleton and allows for consideration of a geometric change of soil such as soil foundation. This accordingly allows for large deformation analysis that needs accurate measurement of a volume change of the soil from deformation to failure or with regard to a behavior after the failure. For introduction of this jerk term, Equations (295) to (297) are derived with respect to deformation, based on the concept of linear acceleration method as the basis of the Wilson's θ method. When a response obtained has little effect of acceleration, Equation (294) is equal to simultaneous first-order ordinary differential equations obtained by static analysis. This means there is no need to selectively use dynamic analysis or static analysis. In the case that a constraint condition of, for example, a fixed length between two nodes or a fixed angle between three nodes is imposed on the internode, the Lagrange's method of undertermined multipliers is applied to solve Equation (294) with consideration of this constraint condition. The geometric boundary condition on the displacement, the displacement rate or the acceleration set at the time t=t+Δt is converted into a jerk vector at the time t=t+θΔt by Equation (298) when the geometric boundary condition is given by a difference of position vectors, i.e., a displacement vector, by Equation (299) when the geometric boundary condition is given by a velocity vector, and by Equation (300) when the geometric boundary condition is given by an acceleration vector.

[Math. 115]

$$\left[\begin{array}{cccc} \dfrac{1}{(\theta\Delta t)^2}M+\dfrac{1}{6}K & -2L_W{}^T & -2L_A{}^T & -2L_S{}^T \\ -L_{\theta 1}{}^W+\dfrac{1}{2(\theta\Delta t)}L_{BW} & H_W(\theta\Delta t)-2S_{WC} & 0 & -2S_{SW} \\ -L_{\theta 1}{}^A+\dfrac{1}{2(\theta\Delta t)}L_{BA} & 0 & H_A(\theta\Delta t)-2S_{AC} & 2S_{SW} \\ 0 & 2S_C & -2S_C & 2S_W \end{array}\right]\left\{\begin{array}{c} \{\dddot{v}^N(\theta\Delta t)^3\}|_{t+\theta\Delta t} \\ \{p^w\}|_{t+\theta\Delta t} \\ \{p^a\}|_{t+\theta\Delta t} \\ \{s^w\}|_{t+\theta\Delta t} \end{array}\right\}$$

$$=\left\{\begin{array}{c} \{\dot{f}(\theta\Delta t)\}|_{t+\theta\Delta t}-K\left[\{v^N(\theta\Delta t)\}|_t+\{\dot{v}^N(\theta\Delta t)^2\}|_t+\dfrac{1}{3}\{\ddot{v}^N(\theta\Delta t)^3\}|_t\right] \\ -2L_W{}^T\left[\{p^w\}|_t+\dfrac{1}{2}\{\dot{p}^w(\theta\Delta t)\}|_t\right]-2L_A{}^T\left[\{p^a\}|_t+\dfrac{1}{2}\{\dot{p}^a(\theta\Delta t)\}|_t\right]-2L_S{}^T\left[\{s^w\}|_t+\dfrac{1}{2}\{\dot{s}^w(\theta\Delta t)\}|_t\right] \\[4pt] \{\dot{f}_w(\theta\Delta t)\}|_{t+\theta\Delta t}+L_{W2}\{v^N(\theta\Delta t)\}|_t+L_{\theta 2}{}^W\{\dot{v}^N(\theta\Delta t)^2\}|_t+L_{\theta 3}{}^W\{\ddot{v}^N(\theta\Delta t)^3\}|_t \\ -2S_{WC}\left[\{p^w\}|_t+\dfrac{1}{2}\{\dot{p}^w(\theta\Delta t)\}|_t\right]-2S_{SW}\left[\{s^w\}|_t+\dfrac{1}{2}\{\dot{s}^w(\theta\Delta t)\}|_t\right] \\ -\dfrac{1}{2(\theta\Delta t)}L_{BW}\left[2\{\dot{v}^N(\theta\Delta t)^2\}|_t+\{\ddot{v}^N(\theta\Delta t)^3\}|_t\right] \\[4pt] \{\dot{f}_a(\theta\Delta t)\}|_{t+\theta\Delta t}+L_{A2}\{v^N(\theta\Delta t)\}|_t+L_{\theta 2}{}^A\{\dot{v}^N(\theta\Delta t)^2\}|_t+L_{\theta 3}{}^A\{\ddot{v}^N(\theta\Delta t)^3\}|_t \\ -2S_{AC}\left[\{p^a\}|_t+\dfrac{1}{2}\{\dot{p}^a(\theta\Delta t)\}|_t\right]+2S_{SW}\left[\{s^w\}|_t+\dfrac{1}{2}\{\dot{s}^w(\theta\Delta t)\}|_t\right] \\ -\dfrac{1}{2(\theta\Delta t)}L_{BA}\left[2\{\dot{v}^N(\theta\Delta t)^2\}|_t+\{\ddot{v}^N(\theta\Delta t)^3\}|_t\right] \\[4pt] 2S_C\left[\{p^w\}|_t+\dfrac{1}{2}\{\dot{p}^w(\theta\Delta t)\}|_t\right]-2S_C\left[\{p^a\}|_t+\dfrac{1}{2}\{\dot{p}^a(\theta\Delta t)\}|_t\right]+2S_W\left[\{s^w\}|_t+\dfrac{1}{2}\{\dot{s}^w(\theta\Delta t)\}|_t\right] \end{array}\right\} \tag{293}$$

$\{\dddot{v}^N(\theta\Delta t)^3\}|_{t+\theta\Delta t}$ : total increment vector concerning jerk at time $t = t + \theta\Delta t$

[Math. 116]

$$\left[\begin{array}{cccc} M & 0 & 0 & 0 \\ 0 & 0 & 0 & 0 \\ 0 & 0 & 0 & 0 \\ 0 & 0 & 0 & 0 \end{array}\right]\dfrac{d^2}{dt^2}\left\{\begin{array}{c} \{v^N\} \\ \{p^w\} \\ \{p^a\} \\ \{s^w\} \end{array}\right\}+\left[\begin{array}{cccc} 0 & -L_W{}^T & -L_A{}^T & -L_S{}^T \\ L_{W1}+L_{BW} & -S_{WC} & 0 & -S_{SW} \\ L_{A1}+L_{BA} & 0 & -S_{AC} & S_{SW} \\ 0 & S_C & -S_C & S_W \end{array}\right]\dfrac{d}{dt}\left\{\begin{array}{c} \{v^N\} \\ \{p^w\} \\ \{p^a\} \\ \{s^w\} \end{array}\right\}$$

$$+\left[\begin{array}{cccc} K & 0 & 0 & 0 \\ -L_{W2} & H_W & 0 & 0 \\ -L_{A2} & 0 & H_A & 0 \\ 0 & 0 & 0 & 0 \end{array}\right]\left\{\begin{array}{c} \{v^N\} \\ \{p^w\} \\ \{p^a\} \\ \{s^w\} \end{array}\right\}=\left\{\begin{array}{c} \{\dot{f}\} \\ \{\dot{f}_w\} \\ \{\dot{f}_a\} \\ \{0\} \end{array}\right\} \tag{294}$$

[Math. 117]

$$\{\dot{v}^N\}|_{t+\theta\Delta t}=\{\dot{v}^N\}|_t+\dfrac{1}{2}\{\ddot{v}^N\}|_t(\theta\Delta t)+\dfrac{1}{2}\{\dddot{v}^N\}|_{t+\theta\Delta t}(\theta\Delta t) \tag{295}$$

$$\{v^N\}|_{t+\theta\Delta t}=\{v^N\}|_t+\{\dot{v}^N\}|_t(\theta\Delta t)+\dfrac{1}{3}\{\ddot{v}^N\}|_t(\theta\Delta t)^2+\dfrac{1}{6}\{\dddot{v}^N\}|_{t+\theta\Delta t}(\theta\Delta t)^2 \tag{296}$$

$$\left\{x^{N}\right\}\Big|_{t+\theta\Delta t} = \left\{x^{N}\right\}\Big|_{t} + \left\{v^{N}\right\}\Big|_{t}(\theta\Delta t) + \frac{1}{2}\left\{\dot{v}^{N}\right\}\Big|_{t}(\theta\Delta t)^2 + \frac{1}{8}\left\{\ddot{v}^{N}\right\}\Big|_{t}(\theta\Delta t)^3 + \frac{1}{24}\left\{\dddot{v}^{N}\right\}\Big|_{t+\theta\Delta t}(\theta\Delta t)^3 \tag{297}$$

[Math. 118]

$$\dddot{v}^{Nj}{}_r\Big|_{t+\theta\Delta t}(\theta\Delta t)^3 = \dddot{v}^{Nj}{}_r\Big|_{t}(\theta\Delta t)^3 + 24\theta^4\left(x^{Nj}{}_r\Big|_{t+\Delta t} - x^{Nj}{}_r\Big|_{t}\right) - 24\theta^3 v^{Nj}{}_r\Big|_{t}(\theta\Delta t) - 12\theta^2 \dot{v}^{Nj}{}_r\Big|_{t}(\theta\Delta t)^2 - 4\theta\ddot{v}^{Nj}{}_r\Big|_{t}(\theta\Delta t)^3 \tag{298}$$

$$\dddot{v}^{Nj}{}_r\Big|_{t+\theta\Delta t}(\theta\Delta t)^3 = \dddot{v}^{Nj}{}_r\Big|_{t}(\theta\Delta t)^3 + 6\theta^3\left\{v^{Nj}{}_r\Big|_{t+\Delta t}(\theta\Delta t) - v^{Nj}{}_r\Big|_{t}(\theta\Delta t)\right\} - 6\theta^2 \dot{v}^{Nj}{}_r\Big|_{t}(\theta\Delta t)^2 - 3\theta\ddot{v}^{Nj}{}_r\Big|_{t}(\theta\Delta t)^3 \tag{299}$$

$$\dddot{v}^{Nj}{}_r\Big|_{t+\theta\Delta t}(\theta\Delta t)^3 = \dddot{v}^{Nj}{}_r\Big|_{t}(\theta\Delta t)^3 + 2\theta^2\left\{\dot{v}^{Nj}{}_r\Big|_{t+\Delta t}(\theta\Delta t)^2 - \dot{v}^{Nj}{}_r\Big|_{t}(\theta\Delta t)^2\right\} - 2\theta\ddot{v}^{Nj}{}_r\Big|_{t}(\theta\Delta t)^3 \tag{300}$$

$x^{Nj}{}_r\Big|_{t+\Delta t}$ : $r$-direction component of position vector of $j$-th node at time $t = t + \Delta t$ set as boundary condition

$v^{Nj}{}_r\Big|_{t+\Delta t}$ : $r$-direction component of velocity vector of $j$-th node at time $t = t + \Delta t$ set as boundary condition

$\dot{v}^{Nj}{}_r\Big|_{t+\Delta t}$ : $r$-direction component of acceleration vector of $j$-th node at time $t = t + \Delta t$ set as boundary condition

$x^{Nj}{}_r\Big|_{t}$ : $r$-direction component of position vector of $j$-th node at time $t = t$

$v^{Nj}{}_r\Big|_{t}$ : $r$-direction component of velocity vector of $j$-th node at time $t = t$

$\dot{v}^{Nj}{}_r\Big|_{t}$ : $r$-direction component of acceleration vector of $j$-th node at time $t = t$

$\ddot{v}^{Nj}{}_r\Big|_{t}$ : $r$-direction component of jerk vector of $j$-th node at time $t = t$

$\dddot{v}^{Nj}{}_r\Big|_{t+\theta\Delta t}$ : $r$-direction component of converted jerk vector of $j$-th node at time $t = t + \theta\Delta t$

The program subsequently calculates an acceleration field, a velocity field, coordinates and various state quantities at the time t=t+θΔt and identifies a loading state of each Gauss point (step S1240). A total increment vector concerning acceleration, a total increment vector concerning velocity and a coordinate vector at the time t=t+θΔt are calculated according to Equations (301) to (303) derived from Equations (295) to (297) using the total increment vector concerning jerk at the time t=t+θΔt obtained by Equation (293) and various geometric quantities at the time t=t. Respective state quantities A, for example, effective stress, degree of structure R*, degree of overconsolidation R, and anisotropy, at the time t=t+θΔ are calculated by Equation (304). For example, an effective stress rate at each Gauss point in a certain element at the time t=t+θΔ is calculated according to Equation (305) from velocity vectors of nodes in the certain element out of the total increment vectors concerning velocity given by Equation (302). An effective stress is subsequently calculated according to Equation (304) . The rates, such as the degree of structure, the degree of overconsolidation and the anisotropy are determined according to Equations (265), (266) and (269) for the Gauss points in the elasto-plastic state and are set equal to 0 for the Gauss points in the elastic state. In the modified Cam-clay model with super-sub-loading yield surfaces and rotational hardening, the "plastic volume strain" at the time t=t+θΔt is calculated by Equation (309) for the Gauss points in the elasto-plastic state and is kept at a previous value calculated in the last elasto-plastic state for the Gauss points in the elastic state. In the elastic state, the degree of overconsolidation R at the time t=t+θΔ is accordingly calculable using an equation obtained by solving Equation (309) with respect to R. The pore water pressure, the pore air pressure and the degree of saturation at the time t=t+θΔ are directly determined by the global tangent stiffness equations of Equation (293). The rates of the pore water pressure, the pore air pressure and the saturation degree are determined by inversely applying Equation (304). With regard to whether the state of each Gauss point of the soil element is the elasto-plastic state or the elastic state, it is determined that the state of a Gauss point is the elasto-plastic state upon satisfaction of a loading criterion shown by Equation (310) obtained from the numerator of the plastic multiplier using, for example, effective stress and stretching of the Gauss point calculated from each node of the soil element. A column vector of the stretching is obtained by Equation (311). The components of the column vector at the time t=t+θΔt are expressed by Equation (312) for the two-dimensional plane strain condition, by Equation (313) for the axisymmetric condition and by Equation (314) for the three-dimensional condition.

[Math. 119]

$$\left\{\dot{v}^N(\theta\Delta t)^2\right\}\Big|_{t+\theta\Delta t} = \left\{\dot{v}^N(\theta\Delta t)^2\right\}\Big|_t + \frac{1}{2}\left\{\ddot{v}^N(\theta\Delta t)^3\right\}\Big|_t + \frac{1}{2}\left\{\ddot{v}^N(\theta\Delta t)^3\right\}\Big|_{t+\theta\Delta t} \tag{301}$$

$$\left\{v^N(\theta\Delta t)\right\}\Big|_{t+\theta\Delta t} = \left\{v^N(\theta\Delta t)\right\}\Big|_t + \left\{\dot{v}^N(\theta\Delta t)^2\right\}\Big|_t + \frac{1}{3}\left\{\ddot{v}^N(\theta\Delta t)^3\right\}\Big|_t + \frac{1}{6}\left\{\ddot{v}^N(\theta\Delta t)^3\right\}\Big|_{t+\theta\Delta t} \tag{302}$$

$$\left\{x^N\right\}\Big|_{t+\theta\Delta t} = \left\{x^N\right\}\Big|_t + \left\{v^N(\theta\Delta t)\right\}\Big|_t + \frac{1}{2}\left\{\dot{v}^N(\theta\Delta t)^2\right\}\Big|_t + \frac{1}{8}\left\{\ddot{v}^N(\theta\Delta t)^3\right\}\Big|_t + \frac{1}{24}\left\{\ddot{v}^N(\theta\Delta t)^3\right\}\Big|_{t+\theta\Delta t} \tag{303}$$

[Math. 120]

$$A\Big|_{t+\theta\Delta t} = A\Big|_t + \frac{1}{2}\left(\dot{A}\Big|_{t+\theta\Delta t} + \dot{A}\Big|_t\right)(\theta\Delta t) \tag{304}$$

$A|_t$, $A|_{t+\theta\Delta t}$ state quantities $A$ at time $t = t$ and at time $t = t + \theta\Delta t$
$\dot{A}|_t$, $\dot{A}|_{t+\theta\Delta t}$ : rates of state quantities $A$ at time $t = t$ and at time $t = t + \theta\Delta t$

[Math. 121]

$$\left\{\dot{T}'\right\}\Big|_{t+\theta\Delta t} \times (\theta\Delta t) = \left[D^{ep}\right]\left[B\right]\left\{v^{Ni}(\theta\Delta t)\right\}\Big|_{t+\theta\Delta t} - \left\{T'_\Omega\right\}\Big|_{t+\theta\Delta t} \times (\theta\Delta t) \tag{305}$$

$\{v^{Ni}(\theta\Delta t)\}|_{t+\theta\Delta t}$ : column vector expression of velocity vectors at respective nodes in element $i$ at time $t = t + \theta\Delta t$
$\{\dot{T}'\}|_{t+\theta\Delta t}$ : column vector expression of components of effective stress rate tensor $\dot{T}'$ at respective Gauss points in element $i$ at time $t = t + \theta\Delta t$

<2D plane strain condition>

$$\left\{\dot{T}'\right\}\Big|_{t+\theta\Delta t} = \left\{\begin{matrix}\dot{T}'_{11}\\\dot{T}'_{22}\\\dot{T}'_{12}\end{matrix}\right\}\Bigg|_{t+\theta\Delta t} \tag{306}$$

<axisymmetric condition>

$$\left\{\dot{T}'\right\}\Big|_{t+\theta\Delta t} = \left\{\begin{matrix}\dot{T}'_{11}\\\dot{T}'_{22}\\\dot{T}'_{12}\\\dot{T}'_{33}\end{matrix}\right\}\Bigg|_{t+\theta\Delta t} \tag{307}$$

<3D condition>

$$\{\dot{T}'\}\Big|_{t+\theta\Delta t} = \left\{\begin{array}{c} \dot{T}'_{11} \\ \dot{T}'_{22} \\ \dot{T}'_{33} \\ \dot{T}'_{12} \\ \dot{T}'_{23} \\ \dot{T}'_{31} \end{array}\right\}\Bigg|_{t+\theta\Delta t} \tag{308}$$

[Math. 122]

$$\varepsilon_v^{\,\mathrm{p}}\left(=-\int_0^t J\mathrm{tr}\boldsymbol{D}^\mathrm{p}\,\mathrm{d}\tau\right) = \mathrm{MD}\ln\frac{p'}{p'_0} + \mathrm{MD}\ln\frac{\mathrm{M}^2+\eta^{*2}}{\mathrm{M}^2} + \mathrm{MD}\ln R^* - \mathrm{MD}\ln R \tag{309}$$

：subloading yield surface in modified Cam-clay model

$\varepsilon_v^{\,\mathrm{p}}$ : "plastic volume strain" (compression: positive)

$p'_0$ ： $p'(> 0)$ value at which the normal yield surface (modified Cam-clay yield surface) at initial time $t = 0$ gives $\eta^* = 0$ in $p' \sim q$ stress space

[Math. 123]

$$\frac{\mathrm{MD}}{p'(\mathrm{M}^2+\eta^{*2})}\left\{6\widetilde{G}\hat{\boldsymbol{\eta}}\cdot\boldsymbol{D} - \widetilde{K}\alpha(\mathrm{tr}\boldsymbol{D})\right\} > 0 \tag{310}$$

$\{\boldsymbol{D}\} = [B]\{\boldsymbol{v}^{Ni}\}$ : column vector expression of components of stretching tensor D in element i (311)

<2D plane strain condition>

$$\{\boldsymbol{D}\}\Big|_{t+\theta\Delta t} = \left\{\begin{array}{c} D_{11} \\ D_{22} \\ 2D_{12} \end{array}\right\}\Bigg|_{t+\theta\Delta t} \tag{312}$$

<axisymmetric condition>

$$\{\boldsymbol{D}\}\Big|_{t+\theta\Delta t} = \left\{\begin{array}{c} D_{11} \\ D_{22} \\ 2D_{12} \\ D_{33} \end{array}\right\}\Bigg|_{t+\theta\Delta t} \tag{313}$$

<3D condition>

$$\{\boldsymbol{D}\}\big|_{t+\theta\Delta t} = \left\{\begin{array}{c} D_{11} \\ D_{22} \\ D_{33} \\ 2D_{12} \\ 2D_{23} \\ 2D_{31} \end{array}\right\}\Bigg|_{t+\theta\Delta t} \tag{314}$$

The program subsequently detects convergence using the effective stresses of the respective Gauss points in the respective elements (step S1250). The procedure of the first embodiment detects convergence, based on whether Equation (315) given below is satisfied for all the Gauss points in all the elements. In Equation (315), "ε" is a sufficiently small positive value. Upon no detection of the convergence, the program returns to the computation of the global $H_W$ matrix and the global $H_A$ matrix of step S1150 and repeats the processing of steps S1150 to S1250.

[Math. 124]

$$\left|\frac{T_e'^{(n)} - T_e'^{(n-1)}}{T_e'^{(n)}}\right| \le \varepsilon \tag{315}$$

$$T_e'^{(n)} = \left\|\boldsymbol{T}'^{(n)}\right\| = \sqrt{\boldsymbol{T}'^{(n)} \cdot \boldsymbol{T}'^{(n)}} \tag{316}$$

: norm of effective stress tensor $T(n)$ at Gauss point obtained by $n$-th iterative computation at time $t=t+\theta\Delta t$

Upon detection of the convergence at step S1250, on the other hand, the program subsequently calculates an acceleration field, a velocity field, coordinates and various state quantities at a time t=t+Δt and identifies a loading state of each Gauss point (step S1260). The acceleration field, the velocity field and the coordinates at the time t=t+Δt are calculated from the total increment vector concerning jerk at the time t=t+θΔt and the total increment vector concerning acceleration and the total increment vector concerning velocity and the coordinate vector at the time t=t according to Equations (317) to (320) given below. Respective state quantities and their rates at the time t=t+Δt are calculated from the state quantities at the time t=t and at the time t=t+θΔt according to Equations (321) and (322). The loading state of each Gauss point at the time t=t+Δt is identified, based on the respective state quantities at the time t=t+θΔt according to Equations (310) and (311).

[Math. 125]

$$\left\{\dddot{v}^N(\theta\Delta t)^3\right\}\Big|_{t+\Delta t} = \left\{\dddot{v}^N(\theta\Delta t)^3\right\}\Big|_t + \frac{1}{\theta}\left[\left\{\dddot{v}^N(\theta\Delta t)^3\right\}\Big|_{t+\theta\Delta t} - \left\{\dddot{v}^N(\theta\Delta t)^3\right\}\Big|_t\right] \tag{317}$$

$$\left\{\ddot{v}^N(\theta\Delta t)^2\right\}\Big|_{t+\Delta t} = \left\{\ddot{v}^N(\theta\Delta t)^2\right\}\Big|_t + \frac{1}{\theta}\left\{\dddot{v}^N(\theta\Delta t)^3\right\}\Big|_t + \frac{1}{2\theta^2}\left[\left\{\dddot{v}^N(\theta\Delta t)^3\right\}\Big|_{t+\theta\Delta t} - \left\{\dddot{v}^N(\theta\Delta t)^3\right\}\Big|_t\right] \tag{318}$$

$$\left\{\dot{v}^N(\theta\Delta t)\right\}\Big|_{t+\Delta t} = \left\{\dot{v}^N(\theta\Delta t)\right\}\Big|_t + \frac{1}{\theta}\left\{\ddot{v}^N(\theta\Delta t)^2\right\}\Big|_t + \frac{1}{2\theta^2}\left\{\dddot{v}^N(\theta\Delta t)^3\right\}\Big|_t + \frac{1}{6\theta^3}\left[\left\{\dddot{v}^N(\theta\Delta t)^3\right\}\Big|_{t+\theta\Delta t} - \left\{\dddot{v}^N(\theta\Delta t)^3\right\}\Big|_t\right] \tag{319}$$

$$\left\{x^N\right\}\Big|_{t+\Delta t} = \left\{x^N\right\}\Big|_t + \frac{1}{\theta}\left\{\dot{v}^N(\theta\Delta t)\right\}\Big|_t + \frac{1}{2\theta^2}\left\{\ddot{v}^N(\theta\Delta t)^2\right\}\Big|_t + \frac{1}{6\theta^3}\left\{\dddot{v}^N(\theta\Delta t)^3\right\}\Big|_t + \frac{1}{24\theta^4}\left[\left\{\dddot{v}^N(\theta\Delta t)^3\right\}\Big|_{t+\theta\Delta t} - \left\{\dddot{v}^N(\theta\Delta t)^3\right\}\Big|_t\right] \tag{320}$$

[Math. 126]

$$\dot{A}\Big|_{t+\Delta t}(\theta\Delta t) = \dot{A}\Big|_t(\theta\Delta t) + \frac{2}{\theta}\left\{A\Big|_{t+\theta\Delta t} - A\Big|_t - \dot{A}\Big|_t(\theta\Delta t)\right\} \tag{321}$$

$$A|_{t+\Delta t} = A|_t + \frac{1}{2\theta}\left\{\dot{A}|_{t+\Delta t}(\theta\Delta t) + \dot{A}|_t(\theta\Delta t)\right\} \tag{322}$$

$A|_{t+\Delta t}$ : state quantity A at time $t = t + \Delta t$

$\dot{A}|_{t+\Delta t}$ : rate of state quantity $A$ at time $t = t + \Delta t$

The program then determines whether the current computation flow reaches the number of computation steps input at step S100 (step S1270). When the current computation flow does not still reach the input number of computation steps, the program returns to the estimation of the various conditions (for example, effective stress, pore water pressure, pore air pressure, degree of saturation and structure/ overconsolidation/ anisotropy) at the time t=t+θΔt of step S1130 as a next computation step. When the current computation flow reaches the input number of computation steps, on the other hand, the soil-water-air coupled analyzing program 230 outputs the computation results in the form of a specified output file (step S1280) and is then terminated.

One example of the output file is shown in Fig. 12 like the first embodiment. The details of Fig. 12 have been described above.

The soil-water-air coupled analyzing program 230 according to the second embodiment described above inputs and sets a plurality of different soil types to elements and estimates the elasto-plastic deformation behaviors of the soil skeleton in the soil-water-air coupled field under various loading conditions including dynamic loading conditions and static loading conditions with regard to the naturally-deposited, highly structured and overconsolidated ground or specimen, as well as the artificially made remolded soil. The soil-water-air coupled analyzing program 30 of the embodiment accordingly addresses the following issues: deformation behaviors (including bifurcation behavior and post-peak behavior) and self weight consolidation analysis of the specimen; consolidation settlement and lateral movement behaviors (including "secondary consolidation"-like, long-term continuous settlement behavior) of soil under loading like road embankment; soil deformation behaviors in excavation; water pumping-induced soil subsidence; soil bearing capacity; liquefaction and liquefaction-induced settlement (shaking settlement) behaviors under repetitive loading like earthquake; stability/ instability by filtration flow; compacted soil improvement due to cylindrical cavity expansion by sand piles; interaction with an elastic structure (deformation behavior of piled-raft foundation); deformation behaviors of reinforced soil; soil improvement by improvement of mass permeability; and stability of an elasto-plastic embankment structure. Additionally, the broad scope from saturated soil to unsaturated soil expands the coverage of issues to be addressed to deformation and failure behaviors of embankment or slope due to rainfall seepage, deformation and failure behaviors on embankment or slope under the combined condition of torrential rain with earthquake, seismic assessment of river bank and slaking phenomenon of mudstone. In other words, this allows for deformation analysis of the soil skeleton, whether static analysis or dynamic analysis, with regard to unsaturated soil having the air in addition to water in voids of the soil skeleton as well as saturated soil having water saturated in voids of the soil skeleton. When these issues are to be addressed, the soil-water-air coupled analyzing program shown in Fig. 2 should be executed a plurality of times as needed with setting the results of the previous analysis as new analysis conditions or initial conditions. In this case, like the first embodiment, as shown in the flowchart of Fig. 13, after execution of the soil-water-air coupled analyzing program (step S300), it is determined whether further execution of the program is required (strep S310). Upon requirement for further execution, the soil-water-air coupled analyzing program is executed again at step S300 after change of the analysis conditions or the initial conditions (step S320). Upon no requirement for further execution, the soil-water-air coupled analyzing program is terminated.

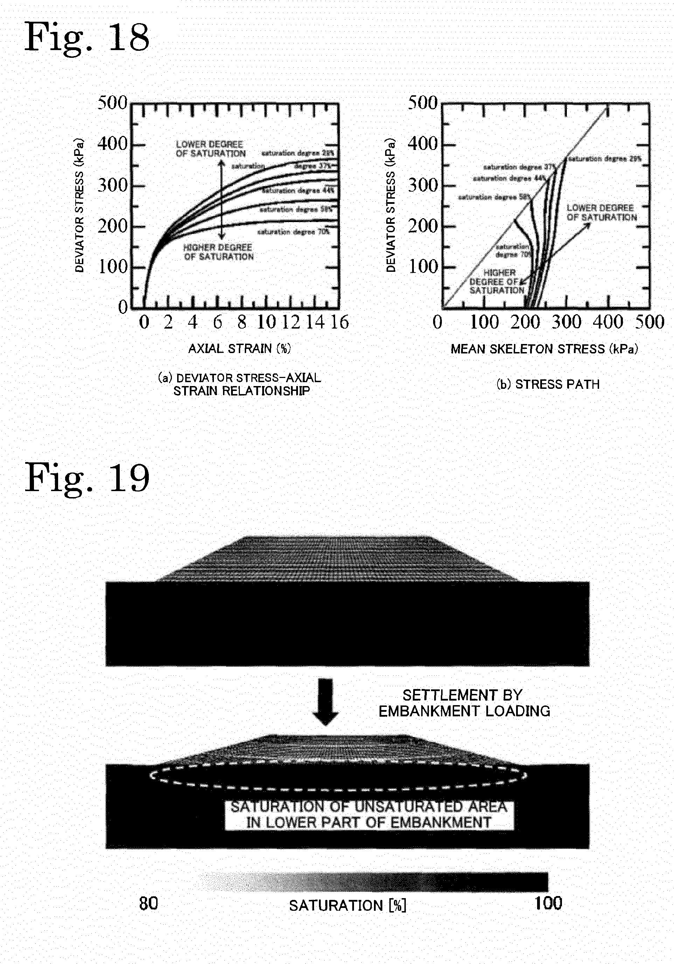

Fig. 18 illustrates the deviator stress to the axial strain relationship (Fig. 18A) and the stress path (Fig. 18B) as the simulation results of a undrained and unexhausted triaxial compression test performed with regard to five different initial degrees of saturation. These results indicate the mechanical behaviors of unsaturated soil according to the difference in degree of saturation.

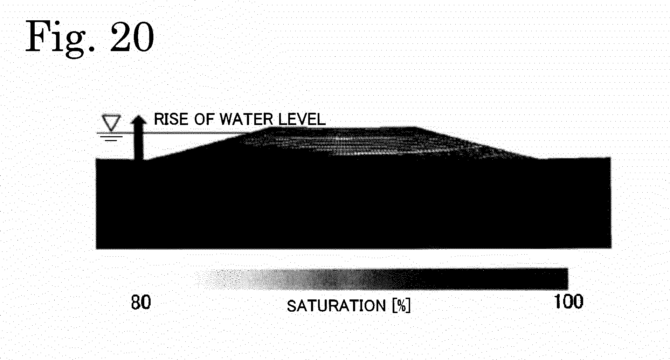

Fig. 19 illustrates a distribution of saturation degree by loading of embankment in the unsaturated state on the clay foundation in the substantially saturated state. This indicates saturation of the lower part of embankment, due to settlement by loading of embankment. Fig. 20 illustrates a distribution of saturation degree in the embankment with a rise of water level. This indicates a process of generating a phreatic surface in the embankment with a rise of water level.

The soil-water-air coupled analyzer 220 of the second embodiment described above computes the global tangent stiffness equations (simultaneous linear equations) using the global L matrix, the global $L_{W2}$ matrix, the global $L_{A2}$ matrix, the global $L_{\theta n}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $L_S{}^T$ matrix, the global $S_{WC}$ matrix, the global $S_{AC}$ matrix, the global $S_{SW}$ matrix, the global $S_C$ matrix and the global $S_W$ matrix to provide the geometric boundary condition with regard to the displacement, the displacement rate or the acceleration, the mechanical boundary condition with regard to the stress or the stress rate, the hydraulic boundary condition with regard to the pore water pressure or the total water head and the flow rate of pore water and the air boundary condition with regard to the pore air pressure or the flow rate of pore air to determine the solutions of the unknown "jerk field", pore water pressure field, pore air pressure field and degree of saturation. This allows for consideration of a geometric change of soil such as soil foundation and accordingly allows for large deformation analysis that needs accurate measurement of a volume change of the soil from deformation to failure or with regard to a behavior after the failure. In other words, this allows for deformation analysis of the soil skeleton, whether static analysis or dynamic analysis, with regard to unsaturated soil having the air in addition to water in voids of the soil skeleton as well as saturated soil having water saturated in voids of the soil skeleton. The first embodiment also allows for setting of a soil type and more specifically allows for setting of a soil type for each element and is thus applicable to compute the mechanical behaviors in a wide range of soil skeletons having various skeleton structures (structure, overconsolidation and anisotropy) including a continuously varying soil structure of sand, intermediate soil to clay, unusual or problematic soils like peat and volcanic ash soil and a layered soil structure of sand, clay and intermediate soil. Additionally, the broad scope from saturated soil to unsaturated soil expands the coverage of issues to be addressed to deformation and failure behaviors of embankment or slope due to rainfall seepage, deformation and failure behaviors on embankment or slope under the combined condition of torrential rain with earthquake, seismic assessment of river bank and slaking phenomenon of mudstone. When an obtained response has little effect of the acceleration, the global tangent stiffness equations are equal to global stiffness equations obtained by static analysis. This allows for computation of the mechanical behaviors of the soil skeleton, whether static of dynamic. More specifically, this allows for static deformation analysis subsequent to dynamic deformation analysis or dynamic deformation analysis subsequent to static deformation analysis, such as consolidation deformation behaviors after earthquake-induced liquefaction as well as seismic deformation behaviors during consolidation deformation.

The soil-water-air coupled analyzer 220 of the second embodiment uses the equation of average soil skeleton stress (equation of $X = s^w$ in the Bishop's effective stress equation), but $X = s^w$ is not restrictive and another effective stress equation may be employed.

The soil-water-air coupled analyzer 220 of the second embodiment uses the simultaneous linear equations with the pore water pressure, the pore air pressure and the degree of saturation as well as the jerk as unknowns. This is, however, not restrictive, but any simultaneous linear equations with part of these parameters as unknowns may be used alternatively; for example, an equation may be established with omission of the degree of saturation from the unknowns.

The soil-water-air coupled analyzer 220 of the second embodiment does not consider a temperature change but may consider a temperature change.

The soil-water-air coupled analyzer 220 of the second embodiment does not consider a phase change between three phases (solid phase, liquid phase and gas phase) but may consider a phase change between the respective phases.

The soil-water-air coupled analyzer 220 of the second embodiment allows for setting of a soil type and more specifically allows for setting of a soil type for each element. The available type of soils may be limited, or the soil type may be set for each element block, instead of each element.

The soil-water-air coupled analyzer 220 of the second embodiment performs rigorous calculation using the global L matrix, the global $L_{W2}$ matrix, the global $L_{A2}$ matrix, the global $L_{\theta n}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $L_S{}^T$ matrix, the global $S_{WC}$ matrix, the global $S_{AC}$ matrix, the global $S_{SW}$ matrix, the global $S_C$ matrix and the global $S_W$ matrixx as the respective element matrices, but the calculation may be approximate calculation.

The soil-water-air coupled analyzer 220 of the second embodiment calculates the global L matrix, the global $L_{W2}$ matrix, the global $L_{A2}$ matrix, the global $L_{en}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $L_S{}^T$ matrix, the global $S_{WC}$ matrix, the global $S_{AC}$ matrix, the global $S_{SW}$ matrix, the global $S_C$ matrix and the global $S_W$ matrix and establishes the global tangent stiffness equations (simultaneous linear equations) using all these plural different types of matrices. Alternatively the global tangent stiffness equations (simultaneous linear equations) may be established using part of the global L matrix, the global $L_{W2}$ matrix, the global $L_{A2}$ matrix, the global $L_{\theta n}{}^W$ matrix and the global $L_{\theta n}{}^A$ matrix, the global $H_W$ matrix, the global $H_A$ matrix, the global $L_{BW}$ matrix, the global $L_{BA}$ matrix, the global M matrix, the global K matrix, the global $L_W{}^T$ matrix, the global $L_A{}^T$ matrix, the global $L_S{}^T$ matrix, the global $S_{WC}$ matrix, the global $S_{AC}$ matrix, the global $S_{SW}$ matrix, the global $S_C$ matrix and the global $S_W$ matrix. In this case, any other suitable element but these plural different types of matrices may also be used.

The soil-water-air coupled analyzer 220 of the second embodiment employs the finite element method for deformation analysis of the soil skeleton but may perform deformation analysis of the soil skeleton without using the finite element method.

The soil-water-air coupled analyzer 220 of the second embodiment inputs the settings of the following analysis conditions as shown in the specific example of Figs. 5 and 6: the setting of "plane strain", "axisymmetric" or "three-dimensional" as the computation condition; selection of either "small deformation theory" or "finite deformation theory" as the deformation theory; the setting of either "Jaumann's rate of Cauchy stress" or "Green-Naghdi's rate" as the effective stress rate with objectivity used in the finite deformation theory; the setting of convergence or non-convergence; selection of either "original Cam-clay model with super-sub-loading yield surfaces and rotational hardening" or "modified Cam-clay model with super-sub-loading yield surfaces and rotational hardening" as the constitutive equation; the setting of either "element average of Gauss points" or "value of a specific Gauss point" as the state quantity output data; setting of either "Text" or "Binary" as the output data format; the setting of consideration or non-consideration of self weight; the current program number; the number of computation steps; the computation time DT per computation step (sec/step) ; the number of divisions of loading during embankment or excavation; the number of divisions of distributed load under application of distributed load; the input file for soil material parameters and initial values; the input file for mesh data; and specification of the output file for initial computation conditions used in execution of a next program. The analysis conditions to be set are, however, not limited to these parameters. Only part of these parameters may be input as the analysis conditions, or any other suitable parameter but the above parameters may be input. Additionally, any suitable format may be employed as the input format.

The soil-water-air coupled analyzer 220 of the second embodiment employs the van Genuchten's equation as the moisture characteristic equation of soil and the Mualem model as the water permeability coefficient/ air permeability coefficient model but may employ another moisture characteristic-related equation of soil which takes into account hysteresis, a void ratio change and temperature dependency and uses the volume water content and the moisture content or another water permeability coefficient/ air permeability coefficient model.

The soil-water-air coupled analyzer 220 of the second embodiment inputs the following settings of soils, such as clay, intermediate soil and sand, for the respective elements constituting the soil foundation as shown in the specific example of Fig. 18: the number of different types of soils as the elasto-plastic bodies, the number of different types of elastic elements, material numbers, specification of the progress scale of plastic deformation adopted in the evolution rule of overconsolidation, specification of the progress scale of plastic deformation adopted in the evolution rule of structure, the intercept N of the isotropic normal consolidation line of remolded soil, the critical state constant M, the compression index $\lambda$, the swelling index $\kappa$, the Poisson's ratio v, the coefficient of saturated water permeability $k_s^w$, the specific gravity $G_s$ of soil particles, the initial lateral pressure coefficient $K_0$, the initial degree of overconsolidation $1/R_0$, the initial degree of structure $1/R^*_0$, the normal consolidation index m, the U* shape, the structural degradation index a, the structural degradation index b, the structural degradation index c, the structural degradation index $c_s$, the rotational hardening index $b_r$, the rotational hardening limit surface $m_b$, the initial degree of anisotropy, the initial void ratio, the coefficient of dry air permeability $k_d^a$, the parameters $S^w_{max}$, $s^w_{min}$, $\alpha_v$, $m_v$ and $n_v$ of the moisture characteristic curve, the parameters $\xi$ and $\gamma$ of the water permeability coefficient/ air permeability coefficient model, the temperature of the air, the initial overburden pressure of the soil foundation, the cell pressure (tensile: positive), the air pressure, the suction, the gas constant of the air, the settings of the unit system (time, force and length), the density of the viscous boundary, the compression wave velocity and the shear wave velocity. The settings of soils are, however, not limited to these parameters. Only part of these parameters may be input as the settings, or any other suitable parameter but the above parameters may be input. Additionally, any suitable format may be employed as the input format.

The soil-water-air coupled analyzer 220 of the second embodiment inputs the following settings of the solid soil model as shown in the specific example of Fig. 8: the number of elements, the number of nodes and the number of elements with application of distributed load rate in the solid soil model, the number of nodes per element, the number of computation steps and the number of elastic elements as the data on the 1st line; the node number, the type of boundary condition in then x-direction, the value of the boundary condition in the x-direction, the type of boundary condition in the y-direction and the value of the boundary condition in the y-direction as the data on each of the 2nd to the 10th lines; the element number and the global node numbers mapped to the local node numbers with regard to the corresponding element as the data on each of the 11th to the 14th lines; the node number, the coordinate in the x-direction and the coordinate in the y-direction as the data on each of the 15th to the 23rd lines; the number of nodes requiring output of reactive force and their node numbers as the data on the 24th and the 25th lines; the element number with application of distributed load rate, the side of application (internode of application), the value in the x-direction of the distributed load rate of its left node, the value in the y-direction of the distributed load rate of the left node, the value in the x-direction of the distributed load rate of its right node and the value in the y-direction of the distributed load rate of the right node as the data on each of the 26th and the 27th lines; the element number, the element number adjacent to the internode 1 of the corresponding element, the element number adjacent to the internode 2 of the corresponding element, the element number adjacent to the internode 3 of the corresponding element and the element number adjacent to the internode 4 of the corresponding element (settings of hydraulic boundary) as the data on each of the 28th to the 31st lines; the element number, the element number adjacent to the internode 1 of the corresponding element, the element number adjacent to the internode 2 of the corresponding element, the element number adjacent to the internode 3 of the corresponding element and the element number adjacent to the internode 4 of the corresponding element (settings of air boundary) as the data on each of the 32nd to the 35th lines; the number of conditions satisfying the fixed length between two nodes (and the settings of the condition) as the data on the 36th line; the number of conditions satisfying the fixed angle between three nodes and the settings of the condition as the data on the 37th and the 38th

lines; the number of conditions satisfying the fixed direction of the relative velocity of two nodes specified by relative position vectors of former two nodes and latter two nodes and the settings of the condition as the data on the 39th and the 40th lines; the number of conditions satisfying the equal displacement (velocity) (and the settings of the condition) as the data on the 41st line; the soil type as the data on the 42nd line; the number of regular waves input into the model and the number of irregular waves input into the model as the data on the 43rd line; the type of regular wave vibration input into the model, the number of nodes under influence of the regular wave vibration input into the model and the amplitude and the period of the regular wave vibration as the data on the 44th line; the node number under influence of the regular wave vibration as the data on the 45th line; and the specification of the irregular waves as the data on a new line below the 45th line. The settings of the solid soil model are, however, not limited to these parameters. Only part of these parameters may be input as the settings of the solid soil model, or any other suitable parameter but the above parameters may be input. Additionally, any suitable format may be employed as the input format.

The soil-water-air coupled analyzer 220 of the second embodiment is allowed to execute the soil-water-air coupled analyzing program a plurality of times using the results of computation of the soil-water-air coupled analyzing program as the new computation conditions or initial conditions. The number of times of repeatedly executing the soil-water-air coupled analyzing program may be limited to a specific number or may be limited to only once.

The soil-water-air coupled analyzer 220 of the second embodiment calculates the element K matrix according to Equation (251) including: the term representing the tangent stiffness providing the mechanical behavior of the identified soil material (for example, sand or clay) (first term on the right side) ; the term representing the contribution of the time change of soil configuration to the tangent stiffness (second term on the right side) ; the term representing the contribution to the tangent stiffness under significant influence of the acceleration to the soil skeleton, for example, impact load (third term on the right side) ; and the term representing the contribution to the tangent stiffness under application of the body force (fourth term on the right side). The element K matrix may, however, be calculated without considering part of these terms under some analysis conditions.

The soil-water-air coupled analyzer 220 of the second embodiment is configured to perform the analysis for any of the two-dimensional plane strain condition, the axisymmetric condition and the three-dimensional condition. The soil-water-air coupled analyzer may, however, be configured to perform the analysis only for the two-dimensional plane strain condition or may be configured to perform the analysis for only the two-dimensional plane strain condition and the axisymmetric condition.

The soil-water-air coupled analyzer 220 of the second embodiment is configured to address the issues of: deformation behaviors (including bifurcation behavior and post-peak behavior) and self weight consolidation analysis of the specimen; consolidation settlement and lateral movement behaviors (including "secondary consolidation"-like, long-term continuous settlement behavior) of soil under loading like road embankment; soil deformation behaviors in excavation; water pumping-induced soil subsidence; soil bearing capacity; liquefaction and liquefaction-induced settlement (shaking settlement) behaviors under repetitive loading like earthquake; stability/ instability by filtration flow; compacted soil improvement due to cylindrical cavity expansion by sand piles; interaction with an elastic structure (deformation behavior of piled-raft foundation); deformation behaviors of reinforced soil; soil improvement by improvement of mass permeability; stability of an elasto-plastic embankment structure; deformation and failure behaviors of embankment or slope due to rainfall seepage; deformation and failure behaviors on embankment or slope under the combined condition of torrential rain with earthquake; seismic assessment of river bank; and slaking phenomenon of mudstone. The soil-water-air coupled analyzer may, however, be configured to address only part of these issues or may be configured to address only one of these issues.

The second embodiment describes the soil-water-air coupled analyzer 20 in which the soil-water-air coupled analyzing program 230 is installed. The invention may be implemented as the soil-water-air coupled analyzing program 230 or may be implemented as the computation module 234 of the soil-water-air coupled analyzing program 230.

The foregoing describes some aspects of the invention with reference to embodiments. The invention is, however, not limited to such embodiments but a multiplicity of variations and modifications may be made to the embodiments without departing from the scope of the invention.

The present application claims priority from Japanese application 2012-141888 filed on June 25, 2012, the content of which is hereby incorporated by reference into this application.

Industrial Applicability

The present invention is applicable to manufacturing industries using the soil-water-air coupled analyzer.

**Claims**

**1.**   A soil-water-air coupled analyzer, comprising:

a data input unit configured to input data regarding a condition of a soil skeleton and data regarding an external force;

an analyzer configured to establish rate-type simultaneous equations of motion including a jerk term of the soil skeleton with respect to a velocity of the soil skeleton, a pore water pressure and a pore air pressure, based on the input data regarding the condition of the soil skeleton and the input data regarding the external force, to integrate the rate-type simultaneous equations of motion as needed by providing a geometric boundary condition with regard to a displacement, a displacement rate or an acceleration, a mechanical boundary condition with regard to a stress or a stress rate, a hydraulic boundary condition with regard to a pore water pressure or a total water head and a flow rate of pore water and an air boundary condition with regard to a pore air pressure or a flow rate of pore air and to determine time history responses of a velocity field, a pore water pressure field and a pore air pressure field, so as to perform deformation analysis of the soil skeleton; and

a result output unit configured to output a result of the deformation analysis.

2. The soil-water-air coupled analyzer according to Claim 1,
wherein the analyzer calculates a first term regarding a time rate of volume change of the soil skeleton, a second term regarding water permeability in soil, a third term regarding air permeability in soil, a fourth term regarding a moisture characteristic of soil, a fifth term regarding time rates of volume change of the pore water and the pore air, a sixth term regarding a contribution of the pore water pressure to a load change rate, a seventh term regarding a contribution of the pore air pressure to the load change rate, an eighth term regarding conversion of the flow rate of the pore water, a ninth term regarding conversion of the flow rate of the pore air, a tenth term regarding a mass and an eleventh term regarding a tangent stiffness of the soil skeleton, based on the input data regarding the condition of the soil skeleton and the input data regarding the external force, and establishes the rate-type simultaneous equations of motion using the calculated terms.

3. The soil-water-air coupled analyzer according to Claim 1,
wherein the rate-type simultaneous equations of motion are equations established from an equation of motion of a three-phase mixture consisting of the air, the water and the soil skeleton, an equation for conservation of mass between the water and the soil skeleton and an equation for conservation of mass between the air and the soil skeleton.

4. The soil-water-air coupled analyzer according to Claim 3,
wherein the equation of motion of the three-phase mixture consisting of the air, the water and the soil skeleton is expressed by Equation (A1),
the equation for conservation of mass between the water and the soil skeleton is expressed by Equation (B1), and
the equation for conservation of mass between the air and the soil skeleton is expressed by Equation (C1).
[Math. 1]

$$\rho \overset{\backslash\backslash}{\boldsymbol{v}}_s + \left[ \left(\rho^w s^w + \rho^a s^a\right)(\mathrm{tr}\,\boldsymbol{D}_s) + n\left\{ \frac{s^w \rho^w}{\mathrm{K}_w} - C\left(\rho^w - \rho^a\right) \right\} \overset{\backslash}{p}{}^w + n\left\{ \frac{s^a}{\overline{\overline{\mathrm{R}\Theta}}} + C\left(\rho^w - \rho^a\right) \right\} \overset{\backslash}{p}{}^a \right]\left( \overset{\backslash}{\boldsymbol{v}}_s - \boldsymbol{b} \right) = \mathrm{div}\,\overset{\backslash}{\boldsymbol{S}}_t \qquad (A1)$$

$$s^w \,\mathrm{div}\,\boldsymbol{v}_s + \frac{1}{\rho^w}\mathrm{div}\left\{ \rho^w \frac{k^w}{\gamma_w}\left( -\mathrm{grad}\,p^w + \rho^w \boldsymbol{b} - \rho^w \overset{\backslash}{\boldsymbol{v}}_s \right) \right\} + \frac{ns^w}{\mathrm{K}_w}\overset{\backslash}{p}{}^w + nC\left( \overset{\backslash}{p}{}^a - \overset{\backslash}{p}{}^w \right) = 0 \qquad (B1)$$

$$s^a \,\mathrm{div}\,\boldsymbol{v}_s + \frac{1}{\rho^a}\mathrm{div}\left\{ \rho^a \frac{k^a}{\gamma_w}\left( -\mathrm{grad}\,p^a + \rho^a \boldsymbol{b} - \rho^a \overset{\backslash}{\boldsymbol{v}}_s \right) \right\} + \frac{ns^a}{\rho^a \overline{\overline{\mathrm{R}\Theta}}}\overset{\backslash}{p}{}^a - nC\left( \overset{\backslash}{p}{}^a - \overset{\backslash}{p}{}^w \right) = 0 \qquad (C1)$$

$\boldsymbol{v}_s$ : velocity vector of soil skeleton
$\boldsymbol{D}_s$ : stretching tensor of soil skeleton
$\rho$ : density of entire mixture

$\rho^w$ : density of water

$\rho^a$ : density of air

$s^w$ : degree of saturation

$s^a$ : air void ratio

n : porosity

$p^w$ : pore water pressure

$p^a$ : pore air pressure

$C$ : specific moisture capacity

$K_w$ : volume elasticity modulus of water

R : gas constant of air

$\Theta$ : absolute temperature of air

$b$ : body force vector per unit mass

$k^w$ : coefficient of water permeability

$k^a$ : coefficient of air permeability

$\gamma_w$ : unit volume weight of water at 4°C (standard density)

$$\overset{\backslash}{\boldsymbol{S}}_t = \overset{\backslash}{\boldsymbol{T}}_{\text{-}} + (\text{tr}\boldsymbol{D_s})\boldsymbol{T}\text{-}\boldsymbol{TL}_s{}^T$$

$T$ : total stress tensor

$\boldsymbol{L_s}$ : velocity gradient tensor of soil skeleton

5. The soil-water-air coupled analyzer according to Claim 1,
wherein the rate-type simultaneous equations of motion including the jerk term of the soil skeleton are formulated with respect to the velocity of the soil skeleton, the pore water pressure, the pore air pressure and a degree of saturation, and
the deformation analysis of the soil skeleton integrates the rate-type simultaneous equations of motion as needed by providing the geometric boundary condition, the mechanical boundary condition, the hydraulic boundary condition and the air boundary condition and determines time history responses of the velocity field, the pore water pressure field, the pore air pressure field and a saturation degree field.

6. The soil-water-air coupled analyzer according to Claim 5,
wherein the analyzer calculates a first term regarding a time rate of volume change of the soil skeleton, a second term regarding water permeability in soil, a third term regarding air permeability in soil, a fourth term regarding a moisture characteristic of soil, a fifth term regarding a time rate of volume change of the pore water, a sixth term regarding a time rate of volume change of the pore air, a seventh term regarding a time rate of change of saturation degree, an eighth term regarding a contribution of the pore water pressure to a load change rate, a ninth term regarding a contribution of the pore air pressure to the load change rate, a tenth term regarding a contribution of the degree of saturation to the load change rate; an eleventh term regarding conversion of the flow rate of the pore water, a twelfth term regarding conversion of the flow rate of the pore air, a thirteenth term regarding a mass and a fourteenth term regarding a tangent stiffness of the soil skeleton, based on the input data regarding the condition of the soil skeleton and the input data regarding the external force, and establishes the rate-type simultaneous equations of motion using the calculated terms.

7. The soil-water-air coupled analyzer according to Claim 5,
wherein the rate-type simultaneous equations of motion are equations established from an equation of motion of a three-phase mixture consisting of the air, the water and the soil skeleton, an equation for conservation of mass between the water and the soil skeleton, an equation for conservation of mass between the air and the soil skeleton and an equation regarding moisture characteristic of soil.

8. The soil-water-air coupled analyzer according to Claim 7,
wherein the equation of motion of the three-phase mixture consisting of the air, the water and the soil skeleton is expressed by Equation (A2),
the equation for conservation of mass between the water and the soil skeleton is expressed by Equation (B2),
the equation for conservation of mass between the air and the soil skeleton is expressed by Equation (C2), and
the equation regarding the moisture characteristic of soil is expressed by Equation (D2).
[Math. 2]

$$\rho \overset{\backslash\backslash}{\boldsymbol{v}}_{\mathrm{s}} + \left[ \left( \rho^{\mathrm{w}} s^{\mathrm{w}} + \rho^{\mathrm{a}} s^{\mathrm{a}} \right)(\mathrm{tr}\,\boldsymbol{D}_{\mathrm{s}}) + \frac{ns^{\mathrm{w}}\rho^{\mathrm{w}}}{\mathrm{K}_{\mathrm{w}}} \overset{\backslash}{p}^{\mathrm{w}} + \frac{ns^{\mathrm{a}}}{\overline{\mathrm{R}\Theta}} \overset{\backslash}{p}^{\mathrm{a}} + n\left( \rho^{\mathrm{w}} - \rho^{\mathrm{a}} \right)s^{\mathrm{w}} \right]\left( \overset{\backslash}{\boldsymbol{v}}_{\mathrm{s}} - \boldsymbol{b} \right) = \mathrm{div}\,\overset{\backslash}{\boldsymbol{S}}_{\mathrm{t}} \qquad (\mathrm{A2})$$

$$s^{\mathrm{w}}\,\mathrm{div}\,\boldsymbol{v}_{\mathrm{s}} + \frac{1}{\rho^{\mathrm{w}}}\,\mathrm{div}\left\{ \rho^{\mathrm{w}}\,\frac{k^{\mathrm{w}}}{\gamma_{\mathrm{w}}}\left( -\mathrm{grad}\,p^{\mathrm{w}} + \rho^{\mathrm{w}}\boldsymbol{b} - \rho^{\mathrm{w}}\overset{\backslash}{\boldsymbol{v}}_{\mathrm{s}} \right) \right\} + n\,\overset{\backslash}{s}^{\mathrm{w}} + \frac{ns^{\mathrm{w}}}{\mathrm{K}_{\mathrm{w}}}\,\overset{\backslash}{p}^{\mathrm{w}} = 0 \qquad (\mathrm{B2})$$

$$s^{\mathrm{a}}\,\mathrm{div}\,\boldsymbol{v}_{\mathrm{s}} + \frac{1}{\rho^{\mathrm{a}}}\,\mathrm{div}\left\{ \rho^{\mathrm{a}}\,\frac{k^{\mathrm{a}}}{\gamma_{\mathrm{w}}}\left( -\mathrm{grad}\,p^{\mathrm{a}} + \rho^{\mathrm{a}}\boldsymbol{b} - \rho^{\mathrm{a}}\overset{\backslash}{\boldsymbol{v}}_{\mathrm{s}} \right) \right\} + n\,\overset{\backslash}{s}^{\mathrm{a}} + \frac{ns^{\mathrm{a}}}{\rho^{\mathrm{a}}\overline{\mathrm{R}\Theta}}\,\overset{\backslash}{p}^{\mathrm{a}} = 0 \qquad (\mathrm{C2})$$

$$SD(p^{\mathrm{w}}, p^{\mathrm{a}}, s^{\mathrm{w}}, \mathrm{e}, \overset{\backslash}{p}^{\mathrm{w}}, \overset{\backslash}{p}^{\mathrm{a}}, \overset{\backslash}{s}^{\mathrm{w}}, \overset{\backslash}{\mathrm{e}}, \cdots) = 0 \qquad \text{(The argument of the function depends on the model used.)}$$

$\boldsymbol{v}_{\mathrm{s}}$ : velocity vector of soil skeleton

$\boldsymbol{D}_{\mathrm{s}}$ : stretching tensor of soil skeleton

$\rho$ : density of entire mixture

$\rho^{\mathrm{w}}$ : density of water

$\rho^{\mathrm{a}}$ : density of air

$s^{\mathrm{w}}$ : degree of saturation

$s^{\mathrm{a}}$ : air void ratio

$\boldsymbol{n}$ : porosity

e : void ratio

$p^{\mathrm{w}}$ : pore water pressure

$p^{\mathrm{a}}$ : pore air pressure

$C$ : specific moisture capacity

$\mathrm{K}_{\mathrm{w}}$ : volume elasticity modulus of water

$\overline{\mathrm{R}}$ : gas constant of air

$\Theta$ : absolute temperature of air

$\boldsymbol{b}$ : body force vector per unit mass

$k^{\mathrm{w}}$ : coefficient of water permeability

$k^{\mathrm{a}}$ : coefficient of air permeability

$\gamma_{\mathrm{w}}$ : unit volume weight of water at 4°C (standard unit volume weight)

$SD$ : one example of function expressing equation regarding moisture characteristic of soil $\overset{\backslash}{\boldsymbol{S}}_{t}$

$= \overset{\backslash}{\boldsymbol{T}}_{\cdot} + (\mathrm{tr}\boldsymbol{D}_{S})\boldsymbol{T} - \boldsymbol{T}\boldsymbol{L}_{\mathrm{s}}^{\mathrm{T}}$

$\boldsymbol{T}$ : total stress tensor

$\boldsymbol{L}_{S}$ : velocity gradient tensor of soil skeleton

9. The soil-water-air coupled analyzer according to any one of Claims 1 to 8,
wherein the analyzer establishes the rate-type simultaneous equations of motion using a numerical analysis method such as a finite element method or a finite difference method.

10. The soil-water-air coupled analyzer according to any one of Claims 1 to 9,
wherein the data regarding the condition of the soil skeleton includes data regarding a profile of each element of a plurality of elements constituting the soil skeleton and data regarding a relationship to an adjacent element and/or adjacent elements.

**11.** The soil-water-air coupled analyzer according to Claim 10,
wherein the data regarding the profile of each element includes data regarding a soil profile.

**12.** The soil-water-air coupled analyzer according to any one of Claims 1 to 11,
wherein the data regarding the external force includes data regarding a load and a displacement applied to a soil-water-air coupled system.

**13.** The soil-water-air coupled analyzer according to any one of Claims 1 to 12,
wherein the data regarding the external force includes data regarding a vibration applied to a soil-water-air coupled system.

**14.** The soil-water-air coupled analyzer according to any one of Claims 1 to 13,
wherein the analyzer performs the analysis with changing the data regarding the external force a plurality of times.

**15.** The soil-water-air coupled analyzer according to Claim 14,
wherein when the data regarding the external force is changed, the analyzer performs the analysis using a result of the analysis prior to the change as data regarding an initial state of the soil skeleton.

**16.** A soil-water-air coupled analyzing method, comprising:

establishing rate-type simultaneous equations of motion including a jerk term of the soil skeleton with respect to a velocity of a soil skeleton, a pore water pressure and a pore air pressure, based on data regarding a condition of the soil skeleton and data regarding an external force; integrating the rate-type simultaneous equations of motion as needed by providing a geometric boundary condition with regard to a displacement, a displacement rate or an acceleration, a mechanical boundary condition with regard to a stress or a stress rate, a hydraulic boundary condition with regard to a pore water pressure or a total water head and a flow rate of pore water and an air boundary condition with regard to a pore air pressure or a flow rate of pore air; and determining time history responses of a velocity field, a pore water pressure field and a pore air pressure field, so as to perform deformation analysis of the soil skeleton.

**17.** The soil-water-air coupled analyzing method according to Claim 16, further comprising:

calculating a first term regarding a time rate of volume change of the soil skeleton, a second term regarding water permeability in soil, a third term regarding air permeability in soil, a fourth term regarding a moisture characteristic of soil, a fifth term regarding time rates of volume change of the pore water and the pore air, a sixth term regarding a contribution of the pore water pressure to a load change rate; a seventh term regarding a contribution of the pore air pressure to the load change rate, an eighth term regarding conversion of the flow rate of the pore water, a ninth term regarding conversion of the flow rate of the pore air, a tenth term regarding a mass and an eleventh term regarding a tangent stiffness of the soil skeleton, based on the data regarding the condition of the soil skeleton and the data regarding the external force, and establishing the rate-type simultaneous equations of motion using the calculated terms.

**18.** The soil-water-air coupled analyzing method according to Claim 16,
wherein the rate-type simultaneous equations of motion including the jerk term of the soil skeleton are formulated with respect to the velocity of the soil skeleton, the pore water pressure, the pore air pressure and a degree of saturation, and
the deformation analysis of the soil skeleton integrates the rate-type simultaneous equations of motion as needed by providing the geometric boundary condition, the mechanical boundary condition, the hydraulic boundary condition and the air boundary condition and determines time history responses of the velocity field, the pore water pressure field, the pore air pressure field and a saturation degree field.

**19.** The soil-water-air coupled analyzing method according to Claim 18, further comprising:

calculating a first term regarding a time rate of volume change of the soil skeleton, a second term regarding water permeability in soil, a third term regarding air permeability in soil, a fourth term regarding a moisture characteristic of soil, a fifth term regarding a time rate of volume change of the pore water, a sixth term regarding a time rate of volume change of the pore air, a seventh term regarding a time rate of change of saturation degree, an eighth term regarding a contribution of the pore water pressure to a load change rate, a ninth term regarding a contribution of the pore air pressure to the load change rate; a tenth term regarding a contribution of the degree of saturation to the load change rate; an eleventh term regarding conversion of the flow rate of the pore water, a twelfth term regarding conversion of the flow rate of the pore air, a thirteenth term regarding a mass and a fourteenth term regarding a tangent stiffness of the soil skeleton, based on the data regarding the condition of the soil skeleton and the data regarding the external force, and establishing the rate-type simultaneous equations of motion using the calculated terms.

20. A soil-water-air coupled analyzing program configured to perform deformation analysis of a soil skeleton, comprising:

    a data input module configured to input data regarding a condition of the soil skeleton and data regarding an external force; and
    an analyzing module configured to establish rate-type simultaneous equations of motion including a jerk term of the soil skeleton with respect to a velocity of the soil skeleton, a pore water pressure and a pore air pressure, based on the input data regarding the condition of the soil skeleton and the input data regarding the external force, to integrate the rate-type simultaneous equations of motion as needed by providing a geometric boundary condition with regard to a displacement, a displacement rate or an acceleration, a mechanical boundary condition with regard to a stress or a stress rate, a hydraulic boundary condition with regard to a pore water pressure or a total water head and a flow rate of pore water and an air boundary condition with regard to a pore air pressure or a flow rate of pore air and to determine time history responses of a velocity field, a pore water pressure field and a pore air pressure field, so as to perform deformation analysis of the soil skeleton.

21. The soil-water-air coupled analyzing program according to Claim 20,
wherein the analyzing module calculates a first term regarding a time rate of volume change of the soil skeleton, a second term regarding water permeability in soil, a third term regarding air permeability in soil, a fourth term regarding a moisture characteristic of soil, a fifth term regarding time rates of volume change of the pore water and the pore air, a sixth term regarding a contribution of the pore water pressure to a load change rate; a seventh term regarding a contribution of the pore air pressure to the load change rate, an eighth term regarding conversion of the flow rate of the pore water, a ninth term regarding conversion of the flow rate of the pore air, a tenth term regarding a mass and an eleventh term regarding a tangent stiffness of the soil skeleton, based on the data regarding the condition of the soil skeleton and the data regarding the external force, and establishes the rate-type simultaneous equations of motion using the calculated terms.

22. The soil-water-air coupled analyzing program according to Claim 20,
wherein the rate-type simultaneous equations of motion including the jerk term of the soil skeleton are formulated with respect to the velocity of the soil skeleton, the pore water pressure, the pore air pressure and a degree of saturation, and
the deformation analysis of the soil skeleton integrates the rate-type simultaneous equations of motion as needed by providing the geometric boundary condition, the mechanical boundary condition, the hydraulic boundary condition and the air boundary condition and determines time history responses of the velocity field, the pore water pressure field, the pore air pressure field and a saturation degree field.

23. The soil-water-air coupled analyzing program according to Claim 22,
wherein the analyzing module calculates a first term regarding a time rate of volume change of the soil skeleton, a second term regarding water permeability in soil, a third term regarding air permeability in soil, a fourth term regarding a moisture characteristic of soil, a fifth term regarding a time rate of volume change of the pore water, a sixth term regarding a time rate of volume change of the pore air, a seventh term regarding a time rate of change of saturation degree, an eighth term regarding a contribution of the pore water pressure to a load change rate, a ninth term regarding a contribution of the pore air pressure to the load change rate; a tenth term regarding a contribution of the degree of saturation to the load change rate; an eleventh term regarding conversion of the flow rate of the pore water, a twelfth term regarding conversion of the flow rate of the pore air, a thirteenth term regarding a mass and a fourteenth term regarding a tangent stiffness of the soil skeleton, based on the data regarding the condition of the soil skeleton and the data regarding the external force, and establishes the rate-type simultaneous equations of motion using the calculated terms.

# Fig. 1

SOIL-WATER-AIR COUPLED
ANALYZING PROGRAM — 30

DATA INPUT MODULE — 32

COMPUTATION MODULE — 34

RESULT OUTPUT MODULE — 36

20

22

# Fig. 2

SOIL-WATER-AIR COUPLED ANALYZING PROGRAM

INPUT SETTINGS OF ANALYSIS CONDITIONS
COMPUTATION TECHNIQUE (NUMERICAL TIME INTEGRATION, STRESS RATE), NUMBER OF COMPUTATION STEPS, COMPUTATION CONDITION (2D PLANE STRAIN, AXISYMMETRIC, 3D) — S100

INPUT SETTINGS OF SOILS SUCH AS CLAY, INTERMEDIATE SOIL AND SAND
MATERIAL PARAMETERS AND INITIAL CONDITIONS — S110

INPUT SETTINGS OF SOLID SOIL MODEL
STRATAL ARCHITECTURE, LOADING CONDITION AND BOUNDARY CONDITIONS OF PORE WATER AND PORE AIR — S120

ESTIMATE VARIOUS CONDITIONS (EFFECTIVE STRESS, PORE WATER PRESSURE, PORE AIR PRESSURE, STRUCTURE/ OVERCONSOLIDATION/ ANISOTROPY) AT TIME $t=t+\theta \Delta t$ — S130

COMPUTE GLOBAL L MATRIX — S140

COMPUTE GLOBAL $H_W$ AND GLOBAL $H_A$ MATRICES — S150

COMPUTER GLOBAL $L_{BW}$ AND GLOBAL $L_{BA}$ MATRICES — S160

CALCULATE TOTAL LOAD INCREMENT VECTOR AND TOTAL FLOW INCREMENT VECTORS — S170

COMPUTE GLOBAL M MATRIX — S180

COMPUTER GLOBAL K MATRIX — S190

COMPUTE GLOBAL $L_W{}^T$ AND GLOBAL $L_A{}^T$ MATRICES — S200

COMPUTE GLOBAL $S_{WA}$ AND GLOBAL $S_{AC}$ MATRICES — S210

COMPUTE GLOBAL $L_{W2}$, GLOBAL $L_{A2}$, GLOBAL $L_{\theta n}{}^W$ AND GLOBAL $L_{\theta n}{}^A$ MATRICES — S220

SOLVE GLOBAL TANGENT STIFFNESS EQUATIONS (SIMULTANEOUS LINEAR EQUATIONS) TO DETERMINE "JERK FIELD", PORE WATER PRESSURE FIELD AND PORE AIR PRESSURE FIELD — S230

CALCULATE ACCELERATION FIELD, VELOCITY FIELD, COORDINATES AND VARIOUS STATE QUANTITIES AT TIME $t=t+\theta \Delta t$
IDENTIFY LOADING STATE OF EACH GAUSS POINT — S240

NO ◄ CONVERGENCE IS DETECTED? — S250

YES

CALCULATE (DETERMINE) ACCELERATION FIELD, VELOCITY FIELD, COORDINATES AND VARIOUS STATE QUANTITIES AT TIME $t=t+\Delta t$
IDENTIFY LOADING STATE OF EACH GAUSS POINT — S260

NO ◄ REACH INPUT NUMBER OF COMPUTATION STEPS? — S270

YES

OUTPUT COMPUTATION RESULTS — S280

END

# Fig. 3

# Fig. 4

# Fig. 5

```
1  /  JCOND   /!  computation condition:plane strain (1),axisymmetric (3),three-dimensional (5)
1  /  JDEF    /!  deformation theory:small deformation (0),finite deformation (1)
2  /  IRATE   /!  effective stress rate in finite deformation theory:Cauchy-Jaumann (1),Green-Naghdi (2)
1  /  ICON    /!  convergence:Yes (1),No (0)
23 /  ISLMDL  /!  constitutive equation:modified Cam-clay model with super-sub-loading yield surfaces and rotational hardening (23)
                  original Cam-clay model with super-sub-loading yield surfaces and rotational hardening (3)
0  /  ielorge /!  state quantity output data:element average of Gauss points (0),Gauss points (1-4)
0  /  JBINRY  /!  output data format:Text (0),Binary (1)
0  /  jgrav   /!  consideration or self weight:No (specimen) (0),Yes (soil foundation) (1: specific volume distribution at the initial
                  stage,2: overconsolidation ratio distribution at the initial stage)
```

# Fig. 6

| | |
|---|---|
| 1 | !current program number (NPRO) |
| 100 | !number of computation steps |
| 0.1000000D+01 | !computation time DT |
| 0.1000000D+01 | !number of divisions of loading (REMO) during embankment or excavation |
| 0.0000000D+00 | !number of divisions of distributed load (remt) under application of distributed load |
| 10 | !output skip interval (jump) |
| .¥OUTDAT¥test¥soil¥SILT.dat | !input file for soil material parameters and initial values |
| .¥OUTDAT¥test¥mesh¥test.dat | !input file for mesh data |
| .¥OUTDAT¥test¥file20¥npro1 | !specification of output file for initial computation conditions used in next NPRO |

# Fig. 7

| | | | |
|---|---|---|---|
| 1 | 0 | | !number of different types of soils, number of different types of elastic elements |
| 1 | | | !material numbers |
| 0 | : | irevo | !specification of progress scale of plastic deformation adopted in evolution rule of overconsolidation |
| 4 | : | irevs [DP]:0 [Dvp]:1 [Dsp]:2 [TDp]:3 [Dvp+Ds]:4 | !specification of progress scale of plastic deformation adopted in evolution rule of structure |
| 1.9812 | : | CNYU | !intercept N of isotropic normal consolidation line of remolded soil |
| 1.230 | : | CMYU | !critical state constant M |
| 0.050 | : | RAMDA | !compression index $\lambda$ |
| 0.010 | : | KAPPA | !swelling index $\kappa$ |
| 0.150 | : | POI | !Poisson's ratio $\nu$ |
| 6.94d-5 | : | PK | !coefficient of saturated water permeability $k_s^w$ |
| 2.650 | : | RS | !specific gravity $G_s$ of soil particles |
| 1.000 | : | KI | !initial lateral pressure coefficient $K_0$ |
| 2.500 | : | OCR | !initial degree of overconsolidation $1/R_0$ |
| 4.700 | : | STR | !initial degree of structure $1/R*_0$ |
| 1.300 | : | COSUB | !normal consolidation index m |
| 8 | : | NKS | !U* shape |
| 0.900 | : | COSUP:a,m* | !structural degradation index a |
| 1.000 | : | COSUPb:b | !structural degradation index b |
| 1.000 | : | COSUP2c:c | !structural degradation index c |
| 0.800 | : | COSUPr:cs $(1-cs)*(-Dvp)+cs\sqrt{(3/2)}\|Dsp\|$ | !structural degradation index $c_s$ |
| 0.000 | : | BR | !rotational hardening index br |
| 0.650 | : | CMB | !rotational hardening limit surface mb |
| 1.000 | : | bk0 | !initial degree of anisotropy |
| 0.566 | : | EEM | !initial void ratio |
| 3.82d-3 | : | PAK ka | !coefficient of dry air permeability $k_d^a$ |
| 0.70000 | : | SWS SWmax | !maximum degree of saturation $Sr_{max}$ |
| 0.052 | : | SWR SWmin | !residual degree of saturation $Sr_{min}$ |
| 0.009 | : | VGA $\alpha$ [cm-1] head | !parameter $\alpha$ of moisture characteristic curve |
| 1.45 | : | VGN n (m=1-1/n) | !parameter n of moisture characteristic curve |
| 0.50 | : | MWG $\xi$ | !parameter $\xi$ of water permeability coefficient model |
| 0.50 | : | MAG $\gamma$ | !parameter $\gamma$ of water permeability coefficient model |
| 20.0 | : | AAT Absolute Temperature | !temperature (°C) of air |
| #———————————————— | | | |
| #———————————————— | | | |
| 0.00 | | PDEL | !initial overburden pressure of soil foundation |
| -450.0 | | CONSTP | !initial cell pressure of specimen |
| 250.0 | | PA0 | !initial pore air pressure of specimen |
| 100.0 | | PSC0 | !initial suction of specimen |
| 287.042 | | AGC | !gas constant of air |
| #——— unit ——— | | | |
| 1 | 2 | 2 | !specification of unit system |
| #density(g/cm3), | Vp(m/sec), | Vs(m/sec) | |
| 2.0 | 700.0 | 300.0 | !density of viscous boundary, compression wave velocity, shear wave velocity. |

EP 2 866 019 A1

# Fig. 8

```
4  9  2  4  1  0 ─────────── number of elements, number of nodes, number of elements with application of distributed load rate,
                             number of nodes per element, number of computation steps, number of elastic elements

1  -1  0.00000  -1  0.00000 ┐
2   1  0.00000  -1  0.00000 │
3   1  0.00000  -1  0.00000 │  node number, type of (boundary) condition in x-direction, its value, type of (boundary) condition in
4  -1  0.00000   1  0.00000 │  y-direction, its value
5   1  0.00000   1  0.00000 ├    type of (boundary) condition
6   1  0.00000   1  0.00000 │      0: coordinate control, 1: load rate control,-1: displacement rate control
7  -1  0.00000   1  0.00000 │      -2: displacement acceleration control, 2: acceleration boundary by taking into account viscosity
8  -1  0.00000   1  0.00000 │
9  -1  0.00000   1  0.00000 ┘

1  1  2  5  4  ┐
2  2  3  6  5  │  element number, mapping of local node number to global node number
3  4  5  8  7  │
4  5  6  9  8  ┘

1     0.00000     0.00000 ┐
2     1.25000     0.00000 │
3     2.50000     0.00000 │
4     0.00000     2.50000 │
5     1.25000     2.50000 ├  node number, coordinate in x-direction, coordinate in y-direction
6     2.50000     2.50000 │
7     0.00000     5.00000 │
8     1.25000     5.00000 │
9     2.50000     5.00000 ┘
3         ─────────────── number of nodes requiring output of reactive force
14  16  18 ─────────────── corresponding node numbers including directions (example: y-direction of node number 7: 7 × 2
                            =14)
2  2  0.00000  0.00000  0.00000  0.00000 ┐
4  2  0.00000  0.00000  0.00000  0.00000 ┘  elements with application of distributed load rate, side of application
                                            values of distributed load rate of its left node in x-direction and y-direction
                                            values of distributed load rate of its right node in x-direction and y-direction
1  1  2  3  1  ┐
2  2  2  4  1  │  element number, specification of hydraulic conditions of (four) relevant elements
3  1  4  3  3  │  relevant element number identical with element number: undrained condition
4  2  4  4  3  ┘

1  1  2  3  1  ┐
2  2  2  4  1  │  element number, specification of air conditions of (four) relevant elements
3  1  4  3  3  │  relevant element number identical with element number: unexhausted condition
4  2  4  4  3  ┘

0         ─────────────── number of conditions satisfying fixed length between two nodes
1         ─────────────── number of conditions satisfying fixed angle between three nodes
1  7  8  9  1 ──────────── condition number satisfying fixed angle, specification of nodes involved in the condition
1         ─────────────── number of conditions satisfying fixed direction of relative velocity of two nodes specified by relative position vectors of former
                           two nodes and latter two nodes
1  8  7  4  ─────────────── condition number satisfying fixed direction, specification of nodes involved in the condition
0         ─────────────── number of conditions satisfying equal displacement (velocity)

1  1  1  1  ─────────────── soil type

1  0        ─────────────── number of input regular waves, number of input irregular waves
1  1  -2.549291  6.283184 ───── type of regular wave vibration, number of nodes under influence of regular wave vibration, amplitude, period
14        ─────────────── corresponding node numbers including directions (example: y direction of node number 7: 7 × 2 =14)
```

89

# Fig. 9

$l_m$ : relative position vector from center of gravity of element *i* to center of gravity of element *m*

$l_{im}$ : distance from center of gravity of element *i* to center of gravity on boundary surface of element *m*

$l_{mm}$ : distance from center of gravity of element *m* to center of gravity on boundary surface of element *m*

$l_m$ : distance from center of gravity of element *i* to center of gravity of element *m*

$v_i'^m$ : flow vector of pore water or pore air in element *i* toward element *m*

$v_m'^m$ : flow vector of pore water or pore air in element *m* from element *i*

$h$ : total water head at center of gravity of element *i*

$h_m$ : total water head at center of gravity of element *m*

$h_c$ : total water head at intersection of $l_m$ and boundary surface between element *i* and element *m*

$n^m$ : outward unit normal vector (viewed from element *i*) perpendicular to boundary side between element *i* and *m*

$S^m$ : distance between two nodes on boundary surface between element *i* and element *m*

# Fig. 10

$l_{m}$ : relative position vector from center of gravity of element $i$ to center of gravity of element $m$

$l_{im}$ : distance from center of gravity of element $i$ to center of gravity on boundary surface of element $m$

$l_{mm}$ : distance from center of gravity of element $m$ to center of gravity on boundary surface of element $m$

$l_{m}$ : distance from center or gravity of element $i$ to center of gravity of element $m$

$v_{i}^{\prime m}$ : flow vector of pore water or pore air in element $i$ toward element $m$

$v_{m}^{\prime m}$ : flow vector of pore water or pore air in element $m$ from element $i$

$h$ : total water head at center of gravity of element $i$

$h_{m}$ : total water head at center of gravity of element $m$

$h_{c}$ : total water head at intersection of $l_{m}$ and boundary surface between element $i$ and element $m$

$n^{m}$ : outward unit normal vector (viewed from element $i$) perpendicular to boundary side between element $i$ and $m$

$S^{m}$ : (distance between two nodes) on boundary surface between element $i$ and element $m$ (distance $r'$ from axis of rotational symmetry to center of gravity on side)

$r_{a}$ , $r_{b}$ : distances of two nodes on boundary surface between element $i$ and element $m$ from axis of rotational symmetry

# Fig. 11

$l_m$ : relative position vector from center of gravity of element $i$ to center of gravity of element $m$

$l_{im}$ : distance from center of gravity of element $i$ to center of gravity on boundary surface of element $m$

$l_{mm}$ : distance from center of gravity of element $m$ to center of gravity on boundary surface of element $m$

$l_m$ : distance from center or gravity of element $i$ to center of gravity of element $m$

$v_i'^m$ : flow vector of pore water or pore air in element $i$ toward element $m$

$v_m'^m$ : flow vector of pore water or pore air in element $m$ from element $i$

$h$ : total water head at center of gravity of element $i$

$h_m$ : total water head at center of gravity of element $m$

$h_c$ : total water head at intersection of $l_m$ and boundary surface between element $i$ and element $m$

$n^m$ : outward unit normal vector (viewed from element $i$) perpendicular to two sets of diagonal lines on boundary surface between element $i$ and element $m$

$S^m$ : area of boundary surface between element $i$ and element $m$

# Fig. 12

```
4  9  2  4  18  8  200  200  2  4  0
              number of elements, number of nodes, number of elements with application of distributed load rate, number of nodes per
              element, number of nodes × number of dimensions, number of nodes per element × number of dimensions, specified number
              of computation steps, total number of implemented computation steps, number of dimensions, number of Gauss points per
              element, number of elastic elements
1  1  2  1  23  0  0  0
              selection of analysis conditions, deformation theory, effective stress rate used for finite deformation theory, convergence
              or non-convergence, constitutive equation, method of outputting state quantity data, format of output data and
              consideration or non-consideration of self weight (see input file)

1  -1  0.000000000000000E+00  -1  0.000000000000000E+00 ⌐
2   1  0.000000000000000E+00  -1  0.000000000000000E+00
3   1  0.000000000000000E+00  -1  0.000000000000000E+00
4  -1  0.000000000000000E+00   1  0.000000000000000E+00
5   1  0.000000000000000E+00   1  0.000000000000000E+00      node number, type of (boundary) condition in x-direction, its value
6   1  0.000000000000000E+00   1  0.000000000000000E+00            type of (boundary) condition in y-direction, its value
7  -1  0.000000000000000E+00   1  0.000000000000000E+00
8  -1  0.000000000000000E+00   1  0.000000000000000E+00
9  -1  0.000000000000000E+00   1  0.000000000000000E+00 ⌐

1  1  2  5  4 ⌐
2  2  3  6  5
3  4  5  8  7        element number, mapping of local node number to global node number
4  5  6  9  8 ⌐

3                   ─────────────────── number of nodes requiring output of reactive force
14  16  18          ─────────────────── corresponding node numbers including directions (example: y-direction of node number 7: 7×2
                                         =14)

2  2  0.00000  0.00000  0.00000  0.00000 ⌐
4  2  0.00000  0.00000  0.00000  0.00000 ⌐   elements with application of distributed load rate, side of application
                                             values of distributed load rate of its left node in x-direction and y-direction
                                             values of distributed load rate of its right node in x-direction and y-direction

1  1  2  3  1 ⌐
2  2  2  4  1
3  1  4  3  3        element number, specification of hydraulic conditions of (four) relevant elements
4  2  4  4  3 ⌐      relevant element number identical with element number: undrained condition

1  1  2  3  1 ⌐
2  2  2  4  1        element number, specification of air conditions of (four) relevant elements
3  1  4  3  3        relevant element number identical with element number: unexhausted condition
4  2  4  4  3 ⌐
```

(omitted) values of components at respective nodes, values of effective stress component at respective Gauss points in respective
elements, values of water pressure in respective elements, values of air pressure in respective elements, values of various
parameters at respective Gauss points in respective elements (for example, degree of structure)

```
0                   ─────────────────── number of conditions satisfying fixed length between two nodes
1                   ─────────────────── number of conditions satisfying fixed angle between three nodes
1  7  8  9  1       ─────────────────── condition number satisfying fixed angle, specification of nodes involved in the condition
1                   ─────────────────── number of conditions satisfying fixed direction of relative velocity of two nodes specified by relative position vectors of former
                                         two nodes and latter two nodes
1  8  7  4          ─────────────────── condition number satisfying fixed direction, specification of nodes involved in the condition
0                   ─────────────────── number of conditions satisfying equal displacement (velocity)

1  1  1  1          ─────────────────── soil type

1  0                ─────────────────── number of input regular waves, number of input irregular waves
1  1  -2.549291  6.283184 ─────────── type of regular wave vibration, number of nodes under influence of regular wave vibration, amplitude, period
14                  ─────────────────── corresponding node numbers including directions (example: y direction of node number 7: 7×2 =14)

0.200000000000000E+03            total time from start of computation
```

# Fig. 13

```
                    ┌─────────┐
                    │  START  │
                    └─────────┘
                         │
      ┌──────────────────▼────────────────────────────┐
      │  EXECUTE SOIL-WATER-AIR COUPLED ANALYZING PROGRAM │ ～ S300
      └──────────────────┬────────────────────────────┘
                         │
  S310              ╱────▼────╲              NO
      ───────    ╱  FURTHER EXECUTION OF  ╲───────┐
                ╲   PROGRAM IS REQUIRED?  ╱       │
                  ╲─────────┬──────╱              │
  S320                YES   │                      │
      ───────  ┌───────────▼──────────────────┐   │
      │        │ CHANGE ANALYSIS CONDITIONS OR INITIAL CONDITIONS │   │
      │        │ (FOR EXAMPLE, BOUNDARY CONDITIONS INCLUDING LOADING │   │
      │        │   CONDITION AND TIME PER STEP)  │   │
      │        └──────────────────────────────┘   │
      └──────────────────────────────────────     │
                                                   │
                         ┌─────────┐               │
                         │   END   │◀──────────────┘
                         └─────────┘
```

# Fig. 14

(a)

DEVIATOR STRESS $q$ (kPa)

500, 400, 300, 200, 100, 0

SHEAR STRAIN $\varepsilon_s$ (%)

0 10 20

(b)

DEVIATOR STRESS $q$ (kPa)

500, 400, 300, 200, 100, 0

$q = Mp'$

MEAN EFFECTIVE STRESS $p'$ (kPa)

0 100 200 300 400 500

| | |
|---|---|
| o | ANALYSIS OF SATURATED SOIL CONSISTING OF WATER AND SOIL SKELETON |
| —— | ANALYSIS OF UNSATURATED SOIL CONSISTING OF AIR, WATER AND SOIL SKELETON |

## Fig. 15

(a)

(b)

| | ANALYSIS OF SATURATED SOIL CONSISTING OF WATER AND SOIL SKELETON |
|---|---|
| ○ | |
| ——— | ANALYSIS OF UNSATURATED SOIL CONSISTING OF AIR, WATER AND SOIL SKELETON |

## Fig. 16

(a)

(b)

# Fig. 17

```
        ( SOIL-WATER-AIR COUPLED ANALYZING PROGRAM )
                            │
        ┌───────────────────────────────────────────────┐
        │           INPUT SETTINGS OF ANALYSIS CONDITIONS        │
        │ COMPUTATION TECHNIQUE (NUMERICAL TIME INTEGRATION, STRESS │ ── S1100
        │ RATE), NUMBER OF COMPUTATION STEPS, COMPUTATION CONDITION │
        │            (2D PLANE STRAIN, AXISYMMETRIC, 3D)          │
        └───────────────────────────────────────────────┘
                            │
        ┌───────────────────────────────────────────────┐
        │ INPUT SETTINGS OF SOILS SUCH AS CLAY, INTERMEDIATE SOIL AND SAND │ ── S1110
        │        MATERIAL PARAMETERS AND INITIAL CONDITIONS       │
        └───────────────────────────────────────────────┘
                            │
        ┌───────────────────────────────────────────────┐
        │           INPUT SETTINGS OF SOLID SOIL MODEL          │
        │ STRATAL ARCHITECTURE, LOADING CONDITION AND BOUNDARY │ ── S1120
        │       CONDITIONS OF PORE WATER AND PORE AIR           │
        └───────────────────────────────────────────────┘
                            │
        ┌───────────────────────────────────────────────┐
        │ ESTIMATE VARIOUS CONDITIONS (EFFECTIVE STRESS, PORE WATER │
        │ PRESSURE, PORE AIR PRESSURE, DEGREE OF SATURATION STRUCTURE/ │ ── S1130
        │     OVERCONSOLIDATION/ ANISOTROPY) AT TIME t=t+θΔt     │
        └───────────────────────────────────────────────┘
                            │
        ┌───────────────────────────────────────────────┐
        │            COMPUTE GLOBAL L MATRIX                   │ ── S1140
        └───────────────────────────────────────────────┘
                            │
        ┌───────────────────────────────────────────────┐
        │     COMPUTE GLOBAL H_W AND GLOBAL H_A MATRICES      │ ── S1150
        └───────────────────────────────────────────────┘
                            │
        ┌───────────────────────────────────────────────┐
        │    COMPUTE GLOBAL L_BW AND GLOBAL L_BA MATRICES     │ ── S1160
        └───────────────────────────────────────────────┘
                            │
        ┌───────────────────────────────────────────────┐
        │        CALCULATE TOTAL LOAD INCREMENT VECTOR        │ ── S1170
        │        AND TOTAL FLOW INCREMENT VECTORS            │
        └───────────────────────────────────────────────┘
                            │
        ┌───────────────────────────────────────────────┐
        │            COMPUTE GLOBAL M MATRIX                  │ ── S1180
        └───────────────────────────────────────────────┘
                            │
        ┌───────────────────────────────────────────────┐
        │            COMPUTE GLOBAL K MATRIX                  │ ── S1190
        └───────────────────────────────────────────────┘
                            │
        ┌───────────────────────────────────────────────┐
        │ COMPUTE GLOBAL L_W^T AND GLOBAL L_A^T AND GLOBAL L_S^T MATRICES │ ── S1200
        └───────────────────────────────────────────────┘
                            │
        ┌───────────────────────────────────────────────┐
        │ COMPUTE GLOBAL S_WC, GLOBAL S_AC, GLOBAL S_SW, GLOBAL S_C AND GLOBAL S_W │ ── S1210
        │                    MATRICES                        │
        └───────────────────────────────────────────────┘
                            │
        ┌───────────────────────────────────────────────┐
        │ COMPUTE GLOBAL L_WI, GLOBAL L_AI, GLOBAL L_W2, GLOBAL L_A2, GLOBAL L_n^W │ ── S1220
        │           AND GLOBAL L_n^A MATRICES                │
        └───────────────────────────────────────────────┘
                            │
        ┌───────────────────────────────────────────────┐
        │ SOLVE GLOBAL TANGENT STIFFNESS EQUATIONS (SIMULTANEOUS LINEAR │
        │ EQUATIONS) TO DETERMINE "JERK FIELD", PORE WATER PRESSURE FIELD, │ ── S1230
        │ PORE AIR PRESSURE FIELD AND SATURATION DEGREE FIELD │
        └───────────────────────────────────────────────┘
                            │
        ┌───────────────────────────────────────────────┐
        │ CALCULATE ACCELERATION FIELD, VELOCITY FIELD, COORDINATES AND │
        │ VARIOUS STATE QUANTITIES AT TIME t=t+θΔt IDENTIFY LOADING STATE │ ── S1240
        │             OF EACH GAUSS POINT                    │
        └───────────────────────────────────────────────┘
                            │
             NO    ◇ CONVERGENCE ◇
            ◄──────  IS DETECTED?  ──────────── S1250
                            │ YES
        ┌───────────────────────────────────────────────┐
        │ CALCULATE (DETERMINE) ACCELERATION FIELD, VELOCITY FIELD, │
        │ COORDINATES AND VARIOUS STATE QUANTITIES AT TIME t=t+θΔt │ ── S1260
        │       IDENTIFY LOADING STATE OF EACH GAUSS POINT   │
        └───────────────────────────────────────────────┘
                            │
             NO    ◇ REACH INPUT NUMBER OF ◇
            ◄──────  COMPUTATION STEPS?  ──────────── S1270
                            │ YES
        ┌───────────────────────────────────────────────┐
        │            OUTPUT COMPUTATION RESULTS              │ ── S1280
        └───────────────────────────────────────────────┘
                            │
                        ( END )
```

# Fig. 18

(a) DEVIATOR STRESS-AXIAL
STRAIN RELATIONSHIP

(b) STRESS PATH

# Fig. 19

SETTLEMENT BY
EMBANKMENT LOADING

SATURATION OF UNSATURATED AREA
IN LOWER PART OF EMBANKMENT

80          SATURATION [%]          100

Fig. 20

## INTERNATIONAL SEARCH REPORT

| | International application No. |
|---|---|
| | PCT/JP2013/067315 |

| A. CLASSIFICATION OF SUBJECT MATTER |
|---|
| *G01N3/00*(2006.01)i, *E02D3/02*(2006.01)i |

According to International Patent Classification (IPC) or to both national classification and IPC

| B. FIELDS SEARCHED |
|---|
| Minimum documentation searched (classification system followed by classification symbols) |
| G01N3/00, E02D3/02 |

| Documentation searched other than minimum documentation to the extent that such documents are included in the fields searched |
|---|
| Jitsuyo Shinan Koho 1922-1996 Jitsuyo Shinan Toroku Koho 1996-2013 |
| Kokai Jitsuyo Shinan Koho 1971-2013 Toroku Jitsuyo Shinan Koho 1994-2013 |

| Electronic data base consulted during the international search (name of data base and, where practicable, search terms used) |
|---|
| JSTPlus/JMEDPlus/JST7580(JDreamIII) |

| C. DOCUMENTS CONSIDERED TO BE RELEVANT |
|---|

| Category* | Citation of document, with indication, where appropriate, of the relevant passages | Relevant to claim No. |
|---|---|---|
| Y | WO 2007/046178 A1 (Nagoya University), 26 April 2007 (26.04.2007), entire text; all drawings & JP 4441693 B & US 2009/0164179 A1 & EP 1939605 A1 | 1-23 |
| Y | Ryosuke UZUOKA et al., "Soil-water-air coupled dynamic analysis of unsaturated fill", Proceedings of the Conference on computational Engineerings and Science, vol.14, no.1, 12 May 2009 (12.05.2009), pages 461 to 464 | 1-23 |
| Y | Nadarajah Ravichandran, "Fully coupled finite element model for dynamics of partially saturated soils", Soil Dynamics and Earthquake Engineering Vol.29, No.9, 2009.09, pp.1294-1304 | 1-23 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 July, 2013 (30.07.13) | 06 August, 2013 (06.08.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007046178 A1
- JP 2012141888 A